(19) Europäisches Patentamt
European Patent Office
Office européen des brevets

(11) EP 2 773 625 B1

(12) EUROPÄISCHE PATENTSCHRIFT

(45) Veröffentlichungstag und Bekanntmachung des Hinweises auf die Patenterteilung:
**24.08.2016 Patentblatt 2016/34**

(21) Anmeldenummer: **12778353.8**

(22) Anmeldetag: **29.10.2012**

(51) Int Cl.:
***C07D 249/14*** *(2006.01)* ***C07D 257/06*** *(2006.01)*
***A01N 43/653*** *(2006.01)*

(86) Internationale Anmeldenummer:
**PCT/EP2012/071378**

(87) Internationale Veröffentlichungsnummer:
**WO 2013/064457 (10.05.2013 Gazette 2013/19)**

(54) **5-PHENYLSUBSTITUIERTE N-(TETRAZOL-5-YL)- UND N-(TRIAZOL-5-YL)ARYLCARBONSÄUREAMIDE UND IHRE VERWENDUNG ALS HERBIZIDE**

5-PHENYL SUBSTITUTED N-(TETRAZOL-5-YL)- AND N-(TRIAZOL-5-YL)ARYL CARBOXYLIC ACID AMIDES AND USE OF SAME AS HERBICIDES

AMIDES D'ACIDE CARBONIQUE DE N-(TÉTRAZOL-5-YL)- ET N-(TRIAZOL-5-YL)ARYLE 5-PHÉNYLE-SUBSTITUÉ ET SON UTILISATION COMME HERBICIDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.11.2011 EP 11187669**

(43) Veröffentlichungstag der Anmeldung:
**10.09.2014 Patentblatt 2014/37**

(73) Patentinhaber: **Bayer Intellectual Property GmbH**
**40789 Monheim (DE)**

(72) Erfinder:
- **BRAUN, Ralf**
  **76857 Ramberg (DE)**
- **AHRENS, Hartmut**
  **63329 Egelsbach (DE)**
- **VAN ALMSICK, Andreas**
  **61184 Karben (DE)**
- **LEHR, Stefan**
  **69006 Lyon (FR)**
- **HÄUSER-HAHN, Isolde**
  **51375 Leverkusen (DE)**
- **DIETRICH, Hansjörg**
  **65835 Liederbach (DE)**
- **GATZWEILER, Elmar**
  **61231 Bad Nauheim (DE)**
- **HEINEMANN, Ines**
  **65719 Hofheim (DE)**
- **ROSINGER, Christopher, Hugh**
  **65719 Hofheim am Taunus (DE)**

(74) Vertreter: **BIP Patents**
**c/o Bayer Intellectual Property GmbH**
**Alfred-Nobel-Straße 10**
**40789 Monheim am Rhein (DE)**

(56) Entgegenhaltungen:
**EP-A1- 0 049 071 WO-A1-2012/028579**

Bemerkungen:
Die Akte enthält technische Angaben, die nach dem Eingang der Anmeldung eingereicht wurden und die nicht in dieser Patentschrift enthalten sind.

EP 2 773 625 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus WO2003/010143 und WO2003/010153 sind bestimmte N-(Tetrazol-5-yl)- und N-(Triazol-5-yl)benzamide und ihre pharmakologische Wirkung bekannt. Aus EP 0 049 071 A1 sind herbizid wirksame N-Arylbenzamld bekannt. Speziell sind dort die Verbindungen N-[5-(1-ethyl-1-methylpropyl)-4H-1,2,4- triazol-3-yl]-2,6-dimethoxy-benzamid (Beispiel Nr. 118) und N-(1H-1,2,4-triazol-3-yl)-2,6-dimethoxy-benzamid (Beispiel Nr. 119) genannt. Diese Verbindungen weisen jedoch keine ausreichende herbizide Wirkung auf. Insbesondere bei niedrigen Aufwandmengen ist die herbizide Wirkung der aus EP 0 049 071 A1 bekannten Verbindungen nicht zufriedenstellend. WO 2012/028579 A1 offenbart bestimmte N-(Tetrazol-5-yl)- und N-(Triazol-5-yl)benzamide und Nicotinamide als Herbizide.

Aufgabe vorliegender Erfindung ist die Bereitstellung von Verbidungen, die auch bei niedrigen Aufwandmengen eine gute Wirkung gegen Schadpflanzen aufweisen. Es wurde nun gefunden, dass N-(Tetrazol-5-yl)- und N-(Triazol-5-yl)arylcarbonsäureamide, die in 5-Stellung des Phenylrings bestimmte Substituenten tragen, als Herbizide besonders gut geeignet sind.

Ein Gegenstand der vorliegenden Erfindung sind somit 5-phenylsubstituierte N-(Tetrazol-5-yl)- und N-(Triazol-5-yl)arylcarbonsäureamide der Formel (I) oder deren Salze

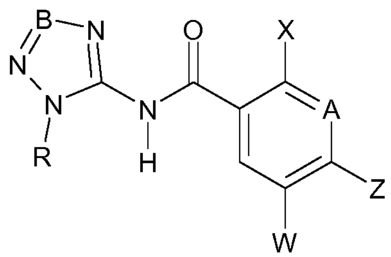

(I),

worin

A bedeutet N oder CY,

B bedeutet N oder CH,

X bedeutet Nitro, Halogen, Cyano, Formyl, Rhodano, $(C_1\text{-}C_6)$-Alkyl, Halogen-$(C_1\text{-}C_6)$-alkyl, $(C_2\text{-}C_6)$-Alkenyl, Halogen-$(C_2\text{-}C_6)$-alkenyl, $(C_2\text{-}C_6)$-Alkinyl, Halogen-$(C_3\text{-}C_6)$-alkinyl, $(C_3\text{-}C_6)$-Cycloalkyl, Halogen-$(C_3\text{-}C_6)$-cycloalkyl, $(C_3\text{-}C_6)$-Cycloalkyl-$(C_1\text{-}C_6)$-alkyl, Halogen-$(C_3\text{-}C_6)$-cycloalkyl-$(C_1\text{-}C_6)$-alkyl, $COR^1$, $COOR^1$, $OCOOR^1$, $NR^1COOR^1$, $C(O)N(R^1)_2$, $NR^1C(O)N(R^1)_2$, $OC(O)N(R^1)_2$, $C(O)NR^1OR^1$, $OR^1$, $OCOR^1$, $OSO_2R^2$, $S(O)_nR^2$, $SO_2OR^1$, $SO_2N(R^1)_2$, $NR^1SO_2R^2$, $NR^1COR^1$, $(C_1\text{-}C_6)$-Alkyl-$S(O)_nR^2$, $(C_1\text{-}C_6)$-Alkyl-$OR^1$, $(C_1\text{-}C_6)$-Alkyl-$OCOR^1$, $(C_1\text{-}C_6)$-Alkyl-$OSO_2R^2$, $(C_1\text{-}C_6)$-Alkyl-$CO_2R^1$, $(C_1\text{-}C_6)$-Alkyl-$SO_2OR^1$, $(C_1\text{-}C_6)$-Alkyl-$CON(R^1)_2$, $(C_1\text{-}C_6)$-Alkyl-$SO_2N(R^1)_2$, $(C_1\text{-}C_6)$-Alkyl-$NR^1COR^1$, $(C_1\text{-}C_6)$-Alkyl-$NR^1SO_2R^2$, $NR_1R_2$, $P(O)(OR^5)_2$, $CH_2P(O)(OR^5)_2$, $(C_1\text{-}C_6)$-Alkyl-Heteroaryl, $(C_1\text{-}C_6)$-Alkyl-Heterocyclyl, wobei die beiden letztgenannten Reste jeweils durch s Reste aus der Gruppe bestehend aus Halogen, $(C_1\text{-}C_6)$-Alkyl, Halogen-$(C_1\text{-}C_6)$-alkyl, $S(O)_n$-$(C_1\text{-}C_6)$-Alkyl, $(C_1\text{-}C_6)$-Alkoxy und Halogen-$(C_1\text{-}C_6)$-alkoxy substituiert sind, und wobei Heterocyclyl n Oxogruppen trägt,

Y bedeutet Wasserstoff, Nitro, Halogen, Cyano, Rhodano, $(C_1\text{-}C_6)$-Alkyl, Halogen-$(C_1\text{-}C_6)$-alkyl, $(C_2\text{-}C_6)$-Alkenyl, Halogen-$(C_2\text{-}C_6)$-alkenyl, $(C_2\text{-}C_6)$-Alkinyl, Halogen-$(C_2\text{-}C_6)$-alkinyl, $(C_3\text{-}C_6)$-Cycloalkyl, $(C_3\text{-}C_6)$-Cycloalkenyl, Halogen-$(C_3\text{-}C_6)$-cycloalkyl, $(C_3\text{-}C_6)$-Cycloalkyl-$(C_1\text{-}C_6)$-alkyl, Halogen-$(C_3\text{-}C_6)$-cycloalkyl-$(C_1\text{-}C_6)$-alkyl, $COR^1$, $COOR^1$, $OCOOR^1$, $NR^1COOR^1$, $C(O)N(R^1)_2$, $NR^1C(O)N(R^1)_2$, $OC(O)N(R^1)_2$, $CO(NOR^1)R^1$, $NR^1SO_2R^2$, $NR^1COR^1$, $OR^1$, $OSO_2R^2$, $S(O)_nR^2$, $SO_2OR^1$, $SO_2N(R^1)_2$ $(C_1\text{-}C_6)$-Alkyl-$S(O)_nR^2$, $(C_1\text{-}C_6)$-Alkyl-$OR^1$, $(C_1\text{-}C_6)$-Alkyl-$OCOR^1$, $(C_1\text{-}C_6)$-Alkyl-OSO2$R^2$, $(C_1\text{-}C_6)$-Alkyl-$CO_2R^1$, $(C_1\text{-}C_6)$-Alkyl-CN, $(C_1\text{-}C_6)$-Alkyl-$SO_2OR^1$, $(C_1\text{-}C_6)$-Alkyl-$CON(R^1)_2$, $(C_1\text{-}C_6)$-Alkyl-$SO_2N(R^1)_2$, $(C_1\text{-}C_6)$-Alkyl-$NR^1COR^1$, $(C_1\text{-}C_6)$-Alkyl-$NR^1SO_2R^2$, $N(R^1)_2$, $P(O)(OR^5)_2$, $CH_2P(O)(OR^5)_2$, $(C_1\text{-}C_6)$-Alkyl-Phenyl, $(C_1\text{-}C_6)$-Alkyl-Heteroaryl, $(C_1\text{-}C_6)$-Alkyl-Heterocyclyl, Phenyl, Heteroaryl oder Heterocyclyl, wobei die sechs letztgenannten Reste jeweils durch s Reste aus der Gruppe bestehend aus Halogen, Nitro, Cyano, $(C_1\text{-}C_6)$-Alkyl, Halogen-$(C_1\text{-}C_6)$-alkyl, $(C_3\text{-}C_6)$-Cycloalkyl, $S(O)_n$-$(C_1\text{-}C_6)$-Alkyl, $(C_1\text{-}C_6)$-Alkoxy, Halogen-$(C_1\text{-}C_6)$-alkoxy, $(C_1\text{-}C_6)$-Alkoxy-$(C_1\text{-}C_4)$-alkyl und Cyanomethyl substituiert sind, und wobei Heterocyclyl n Oxogruppen trägt,

Z bedeutet Halogen, Cyano, Rhodano, Halogen-($C_1$-$C_6$)-alkyl, ($C_2$-$C_6$)-Alkenyl, Halogen-($C_2$-$C_6$)-alkenyl, ($C_2$-$C_6$)-Alkinyl, Halogen-($C_2$-$C_6$)-alkinyl, ($C_3$-$C_6$)-Cycloalkyl, Halogen-($C_3$-$C_6$)-cycloalkyl, ($C_3$-$C_6$)-Cycloalkyl-($C_1$-$C_6$)-alkyl, Halogen-($C_3$-$C_6$)-cycloalkyl-($C_1$-$C_6$)-alkyl, $COR^1$, $COOR^1$, $OCOOR^1$, $NR^1COOR^1$, $C(O)N(R^1)_2$, $NR^1C(O)N(R^1)_2$, $OC(O)N(R^1)_2$, $C(O)NR^1OR^1$, $OSO_2R^2$, $S(O)_nR^2$, $SO_2OR^1$, $SO_2N(R^1)_2$, $NR^1SO_2R^2$, $NR^1COR^1$, ($C_1$-$C_6$)-Alkyl-$S(O)_nR^2$, ($C_1$-$C_6$)-Alkyl-$OR^1$, ($C_1$-$C_6$)-Alkyl-$OCOR^1$, ($C_1$-$C_6$)-Alkyl-$OSO_2R^2$, ($C_1$-$C_6$)-Alkyl-$CO_2R^1$, ($C_1$-$C_6$)-Alkyl-$SO_2OR^1$, ($C_1$-$C_6$)-Alkyl-$CON(R^1)_2$, ($C_1$-$C_6$)-Alkyl-$SO_2N(R^1)_2$, ($C_1$-$C_6$)-Alkyl-$NR^1COR^1$, ($C_1$-$C_6$)-Alkyl-$NR^1SO_2R^2$, $N(R^1)_2$, $P(O)(OR^5)_2$, Heteroaryl, Heterocyclyl oder Phenyl, wobei die drei letztgenannten Reste jeweils durch s Reste aus der Gruppe bestehend aus Halogen, Nitro, Cyano, ($C_1$-$C_6$)-Alkyl, Halogen-($C_1$-$C_6$)-alkyl, ($C_3$-$C_6$)-Cycloalkyl, $S(O)_n$-($C_1$-$C_6$)-Alkyl, ($C_1$-$C_6$)-Alkoxy oder Halogen-($C_1$-$C_6$)-alkoxy substituiert sind, und wobei Heterocyclyl n Oxogruppen trägt, oder

Z kann auch Wasserstoff, ($C_1$-$C_6$)-Alkyl oder ($C_1$-$C_6$)-Alkoxy bedeuten, falls Y für den Rest $S(O)_nR^2$ steht,

W bedeutet ($C_1$-$C_6$)-Alkyl, Halogen-($C_1$-$C_6$)-alkyl, ($C_2$-$C_6$)-Alkenyl, Halogen-($C_2$-$C_6$)-alkenyl, ($C_2$-$C_6$)-Alkinyl, Halogen-($C_2$-$C_6$)-alkinyl, ($C_3$-$C_7$)-Cycloalkyl, ($C_3$-$C_7$)-Halogencycloalkyl, ($C_1$-$C_6$)-Alkoxy, ($C_1$-$C_6$)-Halogenalkoxy, $S(O)_n$-($C_1$-$C_6$)-Alkyl, $S(O)_n$-($C_1$-$C_6$)-Halogenalkyl, ($C_1$-$C_6$)-Alkoxy-($C_1$-$C_4$)-alkyl, ($C_1$-$C_6$)-Alkoxy-($C_1$-$C_4$)-halogenalkyl, Halogen, Nitro, $NR^3COR^3$ oder Cyano,

R bedeutet ($C_1$-$C_8$)-Alkyl, Halogen-($C_1$-$C_8$)-alkyl, ($C_2$-$C_8$)-Alkenyl, Halogen-($C_2$-$C_8$)-alkenyl, ($C_2$-$C_8$)-Alkinyl, Halogen-($C_2$-$C_8$)-alkinyl, wobei diese sechs vorstehend genannten Reste jeweils durch s Reste aus der Gruppe bestehend aus Hydroxy, Nitro, Cyano, $SiR^5_3$, $PO(OR^5)_2$, $S(O)_n$-($C_1$-$C_6$)-Alkyl, $S(O)_n$-($C_1$-$C_6$)-Halogenalkyl, ($C_1$-$C_6$)-Alkoxy, Halogen-($C_1$-$C_6$)-alkoxy, $N(R^3)_2$, $COR^3$, $COOR^3$, $OCOR^3$, $NR^3COR^3$, $NR^3SO_2R^4$, O($C_1$-$C_2$)-Alkyl-($C_3$-$C_6$)-Cycloalkyl, ($C_3$-$C_6$)-Cycloalkyl, Heteroaryl, Heterocyclyl, Phenyl, Q-Heteroaryl, Q-Heterocyclyl, Q-Phenyl und Q-Benzyl substituiert sind, wobei die sieben letztgenannten Reste jeweils durch s Reste aus der Gruppe bestehend aus Methyl, Ethyl, Methoxy, Trifluormethyl, Cyano und Halogen substituiert sind, und wobei Heterocyclyl n Oxogruppen trägt, oder

R bedeutet jeweils durch s Reste aus der Gruppe bestehend aus Halogen, Nitro, Cyano, ($C_1$-$C_6$)-Alkyl, Halogen-($C_1$-$C_6$)-alkyl, ($C_3$-$C_6$)-Cycloalkyl, $S(O)_n$-($C_1$-$C_6$)-Alkyl, ($C_1$-$C_6$)-Alkoxy, Halogen-($C_1$-$C_6$)-alkoxy und ($C_1$-$C_6$)-Alkoxy-($C_1$-$C_4$)-alkyl substituiertes ($C_3$-$C_7$)-Cycloalkyl, Heteroaryl, Heterocyclyl oder Phenyl, wobei Heterocyclyl n Oxogruppen trägt,

Q bedeutet O, S, oder $NR^3$,

$R^1$ bedeutet Wasserstoff, ($C_1$-$C_6$)-Alkyl, ($C_1$-$C_6$)-Halogenalkyl, ($C_2$-$C_6$)-Alkenyl, ($C_2$-$C_6$)-Halogenalkenyl, ($C_2$-$C_6$)-Alkinyl, ($C_2$-$C_6$)-Halogenalkinyl, ($C_3$-$C_6$)-Cycloalkyl, ($C_3$-$C_6$)-Cycloalkenyl, ($C_3$-$C_6$)-Halogencycloalkyl, ($C_1$-$C_6$)-Alkyl-O-($C_1$-$C_6$)-alkyl, ($C_3$-$C_6$)-Cycloalkyl-($C_1$-$C_6$)-alkyl, Phenyl, Phenyl-($C_1$-$C_6$)-alkyl, Heteroaryl, ($C_1$-$C_6$)-Alkyl-Heteroaryl, Heterocycl, ($C_1$-$C_6$)-Alkyl-Heterocyclyl, ($C_1$-$C_6$)-Alkyl-O-Heteroaryl, ($C_1$-$C_6$)-Alkyl-O-Heterocyclyl, ($C_1$-$C_6$)-Alkyl-$NR^3$-Heteroaryl oder ($C_1$-$C_6$)-Alkyl-$NR^3$-Heterocyclyl wobei die 21 letztgenannten Reste jeweils durch s Reste aus der Gruppe bestehend aus Cyano, Halogen, Nitro, Rhodano, $OR^3$, $S(O)_nR^4$, $N(R^3)_2$, $NR^3OR^3$, $COR^3$, $OCOR^3$, $SCOR^4$, $NR^3COR^3$, $NR^3SO_2R^4$ $CO_2R^3$, $COSR^4$, $CON(R^3)_2$ und ($C_1$-$C_4$)-Alkoxy-($C_2$-$C_6$)-alkoxycarbonyl substituiert sind, und wobei Heterocyclyl n Oxogruppen trägt,

$R^2$ bedeutet ($C_1$-$C_6$)-Alkyl, ($C_1$-$C_6$)-Halogenalkyl, ($C_2$-$C_6$)-Alkenyl, ($C_2$-$C_6$)-Halogenalkenyl, ($C_2$-$C_6$)-Alkinyl, ($C_2$-$C_6$)-Halogenalkinyl, ($C_3$-$C_6$)-Cycloalkyl, ($C_3$-$C_6$)-Cycloalkenyl, ($C_3$-$C_6$)-Halogencycloalkyl, ($C_1$-$C_6$)-Alkyl-O-($C_1$-$C_6$)-alkyl, ($C_3$-$C_6$)-Cycloalkyl-($C_1$-$C_6$)-alkyl, Phenyl, Phenyl-($C_1$-$C_6$)-alkyl, Heteroaryl, ($C_1$-$C_6$)-Alkyl-Heteroaryl, Heterocyclyl, ($C_1$-$C_6$)-Alkyl-Heterocyclyl, ($C_1$-$C_6$)-Alkyl-O-Heteroaryl, ($C_1$-$C_6$)-Alkyl-O-Heterocyclyl, ($C_1$-$C_6$)-Alkyl-$NR^3$-Heteroaryl oder ($C_1$-$C_6$)-Alkyl-$NR^3$-Heterocyclyl, wobei die 21 letztgenannten Reste jeweils durch s Reste aus der Gruppe bestehend aus Cyano, Halogen, Nitro, Rhodano, $OR^3$, $S(O)_nR^4$, $N(R^3)_2$, $NR^3OR^3$, $COR^3$, $OCOR^3$, $SCOR^4$, $NR^3COR^3$, $NR^3SO_2R^4$, $CO_2R^3$, $COSR^4$, $CON(R^3)_2$ und ($C_1$-$C_4$)-Alkoxy-($C_2$-$C_6$)-alkoxycarbonyl substituiert sind, und wobei Heterocyclyl n Oxogruppen trägt,

$R^3$ bedeutet Wasserstoff, ($C_1$-$C_6$)-Alkyl, ($C_2$-$C_6$)-Alkenyl, ($C_2$-$C_6$)-Alkinyl, ($C_3$-$C_6$)-Cycloalkyl, ($C_3$-$C_6$)-Cycloalkyl-($C_1$-$C_6$)-alkyl oder Phenyl,

$R^4$ bedeutet ($C_1$-$C_6$)-Alkyl, ($C_2$-$C_6$)-Alkenyl, ($C_2$-$C_6$)-Alkinyl, ($C_3$-$C_6$)-Cycloalkyl, ($C_3$-$C_6$)-Cycloalkyl-($C_1$-$C_6$)-alkyl oder Phenyl,

$R^5$ bedeutet ($C_1$-$C_4$)-Alkyl,

n bedeutet 0, 1 oder 2,

s bedeutet 0, 1, 2 oder 3.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Analog bedeutet Alkenyl z.B. Allyl, 1-Methylprop-2-en-1-yl, 2-Methylprop-2-en-1-yl, But-2-en-1-yl, But-3-en-1-yl, 1-Methyl-but-3-en-1-yl und 1-Methyl-but-2-en-1-yl. Alkinyl bedeutet z.B. Propargyl, But-2-in-1-yl, But-3-in-1-yl, 1-Methyl-but-3-in-1-yl. Die Mehrfachbindung kann sich jeweils in beliebiger Position des ungesättigten Rests befinden. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl. Analog bedeutet Cycloalkenyl eine monocyclische Alkenylgruppe mit drei bis sechs Kohlenstoffringgliedern, z.B. Cyclopropenyl, Cyclobutenyl, Cyclopentenyl und Cyclohexenyl, wobei sich die Doppelbindung an beliebiger Position befinden kann.

Halogen steht für Fluor, Chlor, Brom oder lod.

Heterocyclyl bedeutet einen gesättigten, teilgesättigten oder vollständig ungesättigten cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heterocyclyl für Piperidinyl, Pyrrolidinyl, Tetrahydrofuranyl, Dihydrofuranyl und Oxetanyl,

Heteroaryl bedeutet einen aromatischen cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heteroaryl für Benzimidazol-2-yl, Furanyl, Imidazolyl, Isoxazolyl, Isothiazolyl, Oxazolyl, Pyrazinyl, Pyrimidinyl, Pyridazinyl, Pyridinyl, Benzisoxazolyl, Thiazolyl, Pyrrolyl, Pyrazolyl, Thiophenyl, 1,2,3-Oxadiazolyl, 1,2,4-Oxadiazolyl, 1,2,5-Oxadiazolyl, 1,3,4-Oxadiazolyl, 1,2,4-Triazolyl, 1,2,3-Triazolyl, 1,2,5-Triazolyl, 1,3,4-Triazolyl, 1,2,4-Triazolyl, 1,2,4-Thiadiazolyl, 1,3,4-Thiadiazolyl, 1,2,3-Thiadiazolyl, 1,2,5-Thiadiazolyl, 2H-1,2,3,4-Tetrazolyl, 1H-1,2,3,4-Tetrazolyl, 1,2,3,4-Oxatriazolyl, 1,2,3,5-Oxatriazolyl, 1,2,3,4-Thiatriazolyl und 1,2,3,5-Thiatriazolyl.

Ist eine Gruppe mehrfach durch Reste substituiert, so ist darunter zu verstehen, daß diese Gruppe durch ein oder mehrere gleiche oder verschiedene der genannten Reste substituiert ist. Analoges gilt für den Aufbau von Ringsystemen durch verschiedene Atome und Elemente. Dabei sollen solche Verbindungen vom Anspruchsbegehren ausgenommen sein, von denen der Fachmann weiß, dass sie unter Normalbedingungen chemisch instabil sind.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind. Die erfindungsgemäßen Verbindungen können auf Grund der Oximether-Struktur auch als geometrische Isomere (E-/Z-Isomere) auftreten. Die Erfindung betrifft auch alle E-/Z-Isomere und deren Gemische, die von der allgemeinen Formel (I) umfasst, jedoch nicht spezifisch definiert sind.

Die Verbindungen der Formel (I) können Salze bilden. Salzbildung kann durch Einwirkung einer Base auf solche Verbindungen der Formel (I) erfolgen, die ein acides Wasserstoffatom tragen, z.B. im Falle dass $R^1$ eine COOH-Gruppe oder eine Sulfonamid-Gruppe $-NHSO_2-$ enthält. Geeignete Basen sind beispielsweise organische Amine , wie Trialkylamine, Morpholin, Piperidin oder Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel $[NRR'R''R''']^+$, worin R bis R''' jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie ($C_1$-$C_4$)-Trialkylsulfonium- und ($C_1$-$C_4$)-Trialkylsulfoxoniumsalze.

Die Verbindungen der Formel (I) können durch Anlagerung einer geeigneten anorganischen oder organischen Säure, wie beispielsweise Mineralsäuren, wie beispielsweise HCl, HBr, $H_2SO_4$, $H_3PO_4$ oder $HNO_3$, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion.

Bevorzugt sind Verbindungen der allgemeinen Formel (I), worin

A bedeutet N oder CY,

B bedeutet N oder CH,

X bedeutet Nitro, Halogen, Cyano, Rhodano, $(C_1-C_6)$-Alkyl, Halogen-$(C_1-C_6)$-alkyl, $(C_2-C_6)$-Alkenyl, Halogen-$(C_2-C_6)$-alkenyl, $(C_2-C_6)$-Alkinyl, Halogen-$(C_3-C_6)$-alkinyl, $(C_3-C_6)$-Cycloalkyl, Halogen-$(C_3-C_6)$-cycloalkyl, $(C_1-C_6)$-Alkyl-O-$(C_1-C_6)$-alkyl, $(C_3-C_6)$-Cycloalkyl-$(C_1-C_6)$-alkyl, Halogen-$(C_3-C_6)$-cycloalkyl-$(C_1-C_6)$-alkyl, $COR^1$, $OR^1$, $OCOR^1$, $OSO_2R^2$, $S(O)_nR^2$, $SO_2OR^1$, $SO_2N(R^1)_2$, $NR^1SO_2R^2$, $NR^1COR^1$, $(C_1-C_6)$-Alkyl-$S(O)_nR^2$, $(C_1-C_6)$-Alkyl-$OR^1$, $(C_1-C_6)$-Alkyl-$OCOR^1$, $(C_1-C_6)$-Alkyl-$OSO_2R^2$, $(C_1-C_6)$-Alkyl-$CO_2R^1$, $(C_1-C_6)$-Alkyl-$SO_2OR^1$, $(C_1-C_6)$-Alkyl-$CON(R^1)_2$, $(C_1-C_6)$-Alkyl-$SO_2N(R^1)_2$, $(C_1-C_6)$-Alkyl-$NR^1COR^1$ oder $(C_1-C_6)$-Alkyl-$NR^1SO_2R^2$, $(C_1-C_6)$-Alkyl-Heteroaryl, $(C_1-C_6)$-Alkyl-Heterocyclyl, wobei die beiden letztgenannten Reste jeweils durch s Reste aus der Gruppe bestehend aus Halogen, $(C_1-C_6)$-Alkyl, Halogen-$(C_1-C_6)$-alkyl, $S(O)_n$-$(C_1-C_6)$-Alkyl, $(C_1-C_6)$-Alkoxy und Halogen-$(C_1-C_6)$-alkoxy substituiert sind, und wobei Heterocyclyl n Oxogruppen trägt,

Y bedeutet Wasserstoff, Nitro, Halogen, Cyano, Rhodano, $(C_1-C_6)$-Alkyl, Halogen-$(C_1-C_6)$-alkyl, $(C_2-C_6)$-Alkenyl, Halogen-$(C_2-C_6)$-alkenyl, $(C_2-C_6)$-Alkinyl, Halogen-$(C_3-C_6)$-alkinyl, $(C_3-C_6)$-Cycloalkyl, $(C_3-C_6)$-Cycloalkenyl, Halogen-$(C_3-C_6)$-cycloalkyl, $(C_3-C_6)$-Cycloalkyl-$(C_1-C_6)$-alkyl, Halogen-$(C_3-C_6)$-cycloalkyl-$(C_1-C_6)$-alkyl, $COR^1$, $OR^1$, $COOR^1$, $OSO_2R^2$, $S(O)_nR^2$, $SO_2OR^1$, $SO_2$ $N(R^1)_2$, $N(R^1)_2$, $NR^1SO_2R^2$, $NR^1COR^1$, $(C_1-C_6)$-Alkyl-$S(O)_nR^2$ $(C_1-C_6)$-Alkyl-$OR^1$, $(C_1-C_6)$-Alkyl-$OCOR^1$, $(C_1-C_6)$-Alkyl-$OSO_2R^2$, $(C_1-C_6)$-Alkyl-$CO_2R^1$, $(C_1-C_6)$-Alkyl-$SO_2OR^1$, $(C_1-C_6)$-Alkyl-$CON(R^1)_2$, $(C_1-C_6)$-Alkyl-$SO_2N(R^1)_2$, $(C_1-C_6)$-Alkyl-$NR^1COR^1$, $(C_1-C_6)$-Alkyl-$NR^1SO_2R^2$, $(C_1-C_6)$-Alkyl-Phenyl, $(C_1-C_6)$-Alkyl-Heteroaryl, $(C_1-C_6)$-Alkyl-Heterocyclyl, Phenyl, Heteroaryl oder Heterocyclyl, wobei die sechs letztgenannten Reste jeweils durch s Reste aus der Gruppe bestehend Halogen, Nitro, Cyano, $(C_1-C_6)$-Alkyl, Halogen-$(C_1-C_6)$-alkyl, $(C_3-C_6)$-Cycloalkyl, $S(O)_n$-$(C_1-C_6)$-Alkyl, $(C_1-C_6)$-Alkoxy, Halogen-$(C_1-C_6)$-alkoxy, $(C_1-C_6)$-Alkoxy-$(C_1-C_4)$-alkyl und Cyanomethyl substituiert sind, und wobei Heterocyclyl n Oxogruppen trägt,

Z bedeutet Halogen, Cyano, Rhodano, Halogen-$(C_1-C_6)$-alkyl, $(C_2-C_6)$-Alkenyl, Halogen-$(C_2-C_6)$-alkenyl, $(C_2-C_6)$-Alkinyl, Halogen-$(C_3-C_6)$-alkinyl, $(C_3-C_6)$-Cycloalkyl, Halogen-$(C_3-C_6)$-cycloalkyl, $(C_3-C_6)$-Cycloalkyl-$(C_1-C_6)$-alkyl, Halogen-$(C_3-C_6)$-cycloalkyl-$(C_1-C_6)$-alkyl, $COR^1$, $COOR^1$, $C(O)N(R^1)_2$, $C(O)NR^1OR^1$, $OSO_2R^2$, $S(O)_nR^2$, $SO_2OR^1$, $SO_2N(R^1)_2$, $NR^1SO_2R^2$, $NR^1COR^1$, $(C_1-C_6)$-Alkyl-$S(O)_nR^2$, $(C_1-C_6)$-Alkyl-$OR^1$, $(C_1-C_6)$-Alkyl-$OCOR^1$, $(C_1-C_6)$-Alkyl-$OSO_2R^2$, $(C_1-C_6)$-Alkyl-$CO_2R^1$, $(C_1-C_6)$-Alkyl-$SO_2OR^1$, $(C_1-C_6)$-Alkyl-$CON(R^1)_2$, $(C_1-C_6)$-Alkyl-$SO_2N(R^1)_2$, $(C_1-C_6)$-Alkyl-$NR^1COR^1$, $(C_1-C_6)$-Alkyl-$NR^1SO_2R^2$, 1,2,4-Triazol-1-yl, oder
Z kann auch Wasserstoff, $(C_1-C_6)$-Alkyl oder $(C_1-C_6)$-Alkoxy bedeuten, falls Y für den Rest $S(O)_nR^2$ steht,

W bedeutet $(C_1-C_6)$-Alkyl, Halogen-$(C_1-C_6)$-alkyl, $(C_1-C_6)$-Alkoxy, $(C_1-C_6)$-Halogenalkoxy, $S(O)_n$-$(C_1-C_6)$-alkyl, $S(O)_n$-$(C_1-C_6)$-halogenalkyl, $(C_1-C_6)$-Alkoxy-$(C_1-C_4)$-alkyl, Halogen, Nitro oder Cyano,

R bedeutet $(C_1-C_8)$-Alkyl, Halogen-$(C_1-C_8)$-alkyl, $(C_2-C_8)$-Alkenyl, Halogen-$(C_2-C_8)$-alkenyl, $(C_2-C_8)$-Alkinyl, Halogen-$(C_2-C_8)$-alkinyl, wobei diese sechs vorstehend genannten Reste jeweils durch s Reste aus der Gruppe bestehend aus Nitro, Cyano, $SiR^5_3$, $P(OR^5)_3$, $S(O)_n$-$(C_1-C_6)$-Alkyl, $(C_1-C_6)$-Alkoxy, Halogen-$(C_1-C_6)$-alkoxy, $N(R^3)_2$, $COR^3$, $COOR^3$, $OCOR^3$, $NR^3COR^3$, $NR^3SO_2R^4$, $(C_3-C_6)$-Cycloalkyl, Heteroaryl, Heterocyclyl, Phenyl, Q-Heteroaryl, Q-Heterocyclyl, Q-Phenyl und Q-Benzyl substituiert sind, wobei die sieben letztgenannten Reste jeweils durch s Reste aus der Gruppe bestehend aus Methyl, Ethyl, Methoxy, Trifluormethyl, Cyano und Halogen substituiert sind, und wobei Heterocyclyl n Oxogruppen trägt, oder

R bedeutet jeweils durch s Reste aus der Gruppe bestehend aus Halogen, Nitro, Cyano, $(C_1-C_6)$-Alkyl, Halogen-$(C_1-C_6)$-alkyl, $(C_3-C_6)$-Cycloalkyl, $S(O)_n$-$(C_1-C_6)$-Alkyl, $(C_1-C_6)$-Alkoxy, Halogen-$(C_1-C_6)$-alkoxy und $(C_1-C_6)$-Alkoxy-$(C_1-C_4)$-alkyl substituiertes $(C_3-C_7)$-Cycloalkyl, Heteroaryl, Heterocyclyl oder Phenyl, Q bedeutet O, S, oder $NR^3$,

$R^1$ bedeutet Wasserstoff, $(C_1-C_6)$-Alkyl, $(C_2-C_6)$-Alkenyl, $(C_2-C_6)$-Alkinyl, $(C_3-C_6)$-Cycloalkyl, $(C_3-C_6)$-Cycloalkyl-$(C_1-C_6)$-alkyl, $(C_1-C_6)$-Alkyl-O-$(C_1-C_6)$-alkyl, Phenyl, Phenyl-$(C_1-C_6)$-alkyl, Heteroaryl, $(C_1-C_6)$-Alkyl-Heteroaryl, Heterocyclyl, $(C_1-C_6)$-Alkyl-Heterocyclyl, $(C_1-C_6)$-Alkyl-O-Heteroaryl, $(C_1-C_6)$-Alkyl-O-Heterocyclyl, $(C_1-C_6)$-Alkyl-$NR^3$-Heteroaryl oder $(C_1-C_6)$-Alkyl-$NR^3$-Heterocyclyl, wobei die sechzehn letztgenannten Reste jeweils durch s Reste aus der Gruppe bestehend aus Cyano, Halogen, Nitro, $OR^3$, $S(O)_nR^4$, $N(R^3)_2$, $NR^3OR^3$, $COR^3$, $OCOR^3$, $NR^3COR^3$, $NR^3SO_2R^4$, $CO_2R^3$, $CON(R^3)_2$ und $(C_1-C_4)$-Alkoxy-$(C_2-C_6)$-alkoxycarbonyl substituiert sind, und wobei Heterocyclyl n Oxogruppen trägt,

$R^2$ bedeutet $(C_1-C_6)$-Alkyl, $(C_2-C_6)$-Alkenyl, $(C_2-C_6)$-Alkinyl, $(C_3-C_6)$-Cycloalkyl, $(C_3-C_6)$-Cycloalkyl-$(C_1-C_6)$-alkyl,

$(C_1-C_6)$-Alkyl-O-$(C_1-C_6)$-alkyl, Phenyl, Phenyl-$(C_1-C_6)$-alkyl, Heteroaryl, $(C_1-C_6)$-Alkyl-Heteroaryl, Heterocyclyl, $(C_1-C_6)$-Alkyl-Heterocyclyl, $(C_1-C_6)$-Alkyl-O-Heteroaryl, $(C_1-C_6)$-Alkyl-O-Heterocyclyl, $(C_1-C_6)$-Alkyl-$NR^3$-Heteroaryl oder $(C_1-C_6)$-Alkyl-$NR^3$-Heterocyclyl, wobei diese sechzehn letztgenannten Reste jeweils durch s Reste aus der Gruppe bestehend aus Cyano, Halogen, Nitro, $OR^3$, $S(O)_nR^4$, $N(R^3)_2$, $NR^3OR^3$, $NR^3SO_2R^4$, $COR^3$, $OCOR^3$, $NR^3COR^3$, $CO_2R^3$, $CON(R^3)_2$ und $(C_1-C_4)$-Alkoxy-$(C_2-C_6)$-alkoxycarbonyl substituiert sind, und wobei Heterocyclyl n Oxogruppen trägt,

$R^3$ bedeutet Wasserstoff, $(C_1-C_6)$-Alkyl, $(C_2-C_6)$-Alkenyl, $(C_2-C_6)$-Alkinyl, $(C_3-C_6)$-Cycloalkyl oder $(C_3-C_6)$-Cycloalkyl-$(C_1-C_6)$-alkyl,

$R^4$ bedeutet $(C_1-C_6)$-Alkyl, $(C_2-C_6)$-Alkenyl oder $(C_2-C_6)$-Alkinyl,

$R^5$ bedeutet Methyl oder Ethyl,

n bedeutet 0, 1 oder 2,

s bedeutet 0, 1, 2 oder 3.

Besonders bevorzugt sind Verbindungen der allgemeinen Formel (I), worin

A bedeutet N oder CY,

B bedeutet N oder CH,

X bedeutet Nitro, Halogen, Cyano, $(C_1-C_6)$-Alkyl, Halogen-$(C_1-C_6)$-alkyl, $(C_3-C_6)$-Cycloalkyl, $OR^1$, $S(O)_nR^2$, $(C_1-C_6)$-Alkyl-$S(O)_nR^2$, $(C_1-C_6)$-Alkyl-$OR^1$, $(C_1-C_6)$-Alkyl-$CON(R^1)_2$, $(C_1-C_6)$-Alkyl-$SO_2N(R^1)_2$, $(C_1-C_6)$-Alkyl-$NR^1COR^1$, $(C_1-C_6)$-Alkyl-$NR^1SO_2R^2$, $(C_1-C_6)$-Alkyl-Heteroaryl, $(C_1-C_6)$-Alkyl-Heterocyclyl, wobei die beiden letzt genannten Reste jeweils durch s Reste Halogen, $(C_1-C_6)$-Alkyl, Halogen-$(C_1-C_6)$-alkyl, $S(O)_n$-$(C_1-C_6)$-Alkyl, $(C_1-C_6)$-Alkoxy und Halogen-$(C_1-C_6)$-alkoxy substituiert sind, und wobei Heterocyclyl n Oxogruppen trägt,

Y Wasserstoff, Nitro, Halogen, Cyano, $(C_1-C_6)$-Alkyl, $(C_1-C_6)$-Halogenalkyl, $OR^1$, $S(O)_nR^2$, $SO_2N(R^1)_2$, $N(R^1)_2$, $NR^1SO_2R^2$, $NR^1COR^1$, $(C_1-C_6)$-Alkyl-$S(O)_nR^2$, $(C_1-C_6)$-Alkyl-$OR^1$, $(C_1-C_6)$-Alkyl-$CON(R^1)_2$, $(C_1-C_6)$-Alkyl-$SO_2N(R^1)_2$, $(C_1-C_6)$-Alkyl-$NR^1COR^1$, $(C_2-C_6)$-Alkyl-$NR^1SO_2R^2$, $(C_1-C_6)$-Alkyl-Phenyl, $(C_1-C_6)$-Alkyl-Heteroaryl, $(C_1-C_6)$-Alkyl-Heterocyclyl, Phenyl, Heteroaryl oder Heterocyclyl, wobei die sechs letztgenannten Reste jeweils durch s Reste aus der Gruppe bestehend Halogen, Nitro, Cyano, $(C_1-C_6)$-Alkyl, Halogen-$(C_1-C_6)$-alkyl, $(C_3-C_6)$-Cycloalkyl, $S(O)_n$-$(C_1-C_6)$-Alkyl, $(C_1-C_6)$-Alkoxy, Halogen-$(C_1-C_6)$-alkoxy, $(C_1-C_6)$-Alkoxy-$(C_1-C_4)$-alkyl und Cyanomethyl substituiert sind, und wobei Heterocyclyl n Oxogruppen trägt,

Z bedeutet Halogen, Cyano, Halogen-$(C_1-C_6)$-alkyl, $(C_3-C_6)$-Cycloalkyl, $S(O)_nR^2$, 1,2,4-Triazol-1-yl, oder Z kann auch Wasserstoff, Methyl, Methoxy oder Ethoxy bedeuten, falls Y für den Rest $S(O)_nR^2$ steht,

W bedeutet Methyl, Ethyl, Methoxymethyl, Methoxy, Fluor, Chlor oder $S(O)_nCH_3$,

R bedeutet $(C_1-C_8)$-Alkyl, Halogen-$(C_1-C_8)$-alkyl, $(C_2-C_8)$-Alkenyl, Halogen-$(C_2-C_8)$-alkenyl, $(C_2-C_8)$-Alkinyl, Halogen-$(C_2-C_8)$-alkinyl, wobei diese sechs vorstehend genannten Reste jeweils durch s Reste aus der Gruppe bestehend aus Cyano, $S(O)_n$-$(C_1-C_6)$-Alkyl, $(C_1-C_6)$-Alkoxy, Halogen-$(C_1-C_6)$-alkoxy, $COR^3$, $COOR^3$, $OCOR^3$, $NR^3COR^3$, $NR^3SO_2R^4$, $(C_3-C_6)$-Cycloalkyl, Heteroaryl, Heterocyclyl und Phenyl substituiert sind, wobei die drei letztgenannten Reste jeweils durch s Reste aus der Gruppe bestehend aus Methyl, Ethyl, Methoxy, Trifluormethyl, Cyano und Halogen substituiert sind, und wobei Heterocyclyl 0 bis 2 Oxogruppen trägt, oder R bedeutet durch s Reste aus der Gruppe bestehend aus Halogen, Nitro, Cyano, $(C_1-C_6)$-Alkyl, Halogen-$(C_1-C_6)$-alkyl, $(C_3-C_6)$-Cycloalkyl, $S(O)_n$-$(C_1-C_6)$-Alkyl, $(C_1-C_6)$-Alkoxy, Halogen-$(C_1-C_6)$-alkoxy, $(C_1-C_6)$-Alkoxy-$(C_1-C_4)$-alkyl substituiertes Phenyl,

$R^1$ bedeutet Wasserstoff, $(C_1-C_6)$-Alkyl, $(C_2-C_6)$-Alkenyl, $(C_2-C_6)$-Alkinyl, $(C_3-C_6)$-Cycloalkyl, $(C_3-C_6)$-Cycloalkyl-$(C_1-C_6)$-alkyl, $(C_1-C_6)$-Alkyl-O-$(C_1-C_6)$-alkyl, Phenyl, Phenyl-$(C_1-C_6)$-alkyl, Heteroaryl, $(C_1-C_6)$-Alkyl-Heteroaryl, Heterocyclyl, $(C_1-C_6)$-Alkyl-Heterocyclyl, $(C_1-C_6)$-Alkyl-O-Heteroaryl, $(C_1-C_6)$-Alkyl-O-Heterocyclyl, $(C_1-C_6)$-Alkyl-$NR^3$-Heteroaryl oder $(C_1-C_6)$-Alkyl-$NR^3$-Heterocyclyl, wobei die sechzehn letztgenannten Reste jeweils durch s Reste aus der Gruppe bestehend aus Cyano, Halogen, Nitro, $OR^3$, $S(O)_nR^4$, $N(R^3)_2$, $NR^3OR^3$, $COR^3$, $OCOR^3$, $NR^3COR^3$, $NR^3SO_2R^4$, $CO_2R^3$, $CON(R^3)_2$ und $(C_1-C_4)$-Alkoxy-$(C_2-C_6)$-alkoxycarbonyl substituiert sind, und wobei

Heterocyclyl n Oxogruppen trägt,

$R^2$ bedeutet jeweils durch s Reste aus der Gruppe bestehend aus Halogen und $OR^3$ substituiertes ($C_1$-$C_6$)-Alkyl, ($C_3$-$C_6$)-Cycloalkyl oder ($C_3$-$C_6$)-Cycloalkyl-($C_1$-$C_6$)-alkyl,

$R^3$ bedeutet Wasserstoff oder ($C_1$-$C_6$)-Alkyl,

$R^4$ bedeutet ($C_1$-$C_6$)-Alkyl,

$R^5$ bedeutet Methyl oder Ethyl,

n bedeutet 0, 1 oder 2,

s bedeutet 0, 1, 2 oder 3.

In allen nachfolgend genannten Formeln haben die Substituenten und Symbole, sofern nicht anders definiert, dieselbe Bedeutung wie unter Formel (I) beschrieben.

Erfindungsgemäße Verbindungen können beispielsweise nach der in Schema 1 angegebenen Methode durch basenkatalysierte Umsetzung eines Pyridincarbonsäurechlorids (II) mit einem 5-Amino-1-H-1,2,4-triazol, bzw. 5-Amino-1H-tetrazol (III) hergestellt werden:

Schema 1

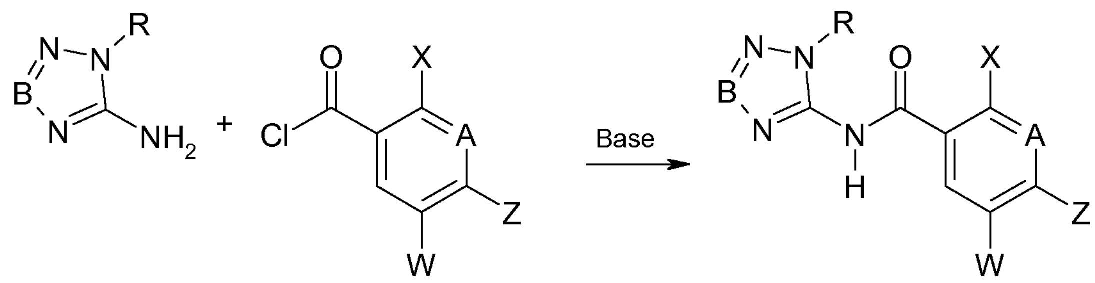


(III) (II) (I)

Die Benzoesäurechloride der Formel (II) beziehungsweise die ihnen zugrunde liegenden Benzoesäuren sind grundsätzlich bekannt und können beispielsweise gemäß den in JP 63122673, WO 99/54328 A1 und WO 97/46530 A1 beschriebenen Methoden hergestellt werden.

Erfindungsgemäße Verbindungen können auch nach der in Schema 2 angegebenen Methode durch Umsetzung einer Benzoesäure der Formel (IV) mit einem 5-Amino-1-H-1,2,4-triazol, bzw. 5-Amino-1 H-tetrazol (III) hergestellt werden:

Schema 2

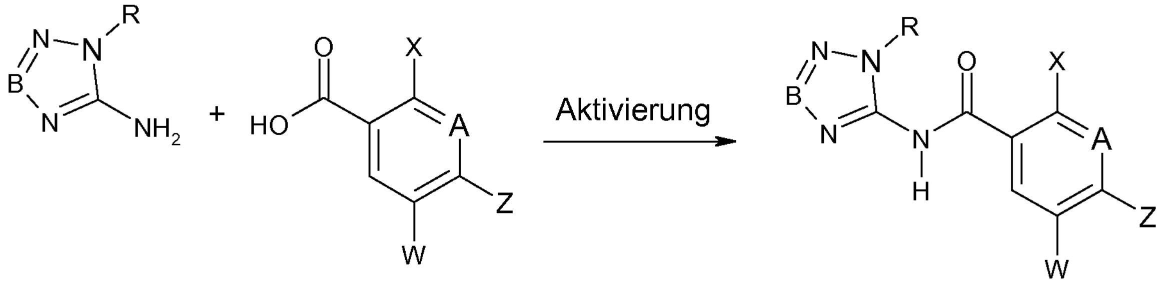


(III) (IV) (I)

Für die Aktivierung können wasserentziehende Reagenzien, die üblicherweise für Amidierungsreaktionen, wie z. B. 1,1'-Carbonyldiimidazol (CDI), Dicyclohexylcarbodiimid (DCC), 2,4,6-Tripropyl-1,3,5,2,4,6-trioxatriphosphinane 2,4,6-trioxide (T3P) etc. eingesetzt werden.

Erfindungsgemäße Verbindungen können auch nach der in Schema 3 angegebenen Methode durch Umsetzung eines N-(1 H-1,2,4-triazol-5-yl)benzamides, N-(1 H-tetrazol-5-yl)benzamides, N-(1 H-1,2,4-triazol-5-yl)nicotinamide oder N-(1 H-tetrazol-5-yl)nicotinamide hergestellt werden: Schema 3

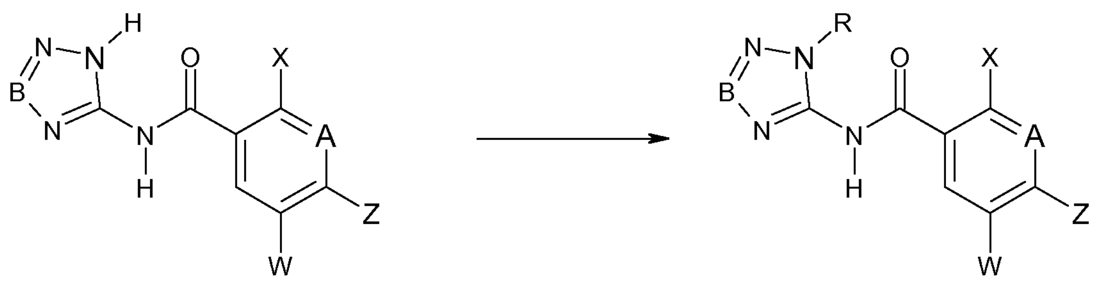

(V) (I)

Für diese in Schema 3 genannte Reaktion können z. B. Alkylierungsmittel wie z. B. Alkylhalogenide, -sulfonate oder Dialkylsulfate in Gegenwart einer Base eingesetzt werden.

Es kann zweckmäßig sein, Reaktionsschritte in ihrer Reihenfolgezu ändern.So sind Benzoesäuren, die ein Sulfoxid tragen, nicht ohne weiteres in ihre Säurechloride zu überführen. Hier bietet sich an, zunächst auf Thioether-Stufe das Amid zu herzustellen und danach den Thioether zum Sulfoxid zu oxidieren.

Die 5-Amino-1H-tetrazole der Formel (III) sind entweder käuflich erhältlich oder können analog zu literaturbekannten Methoden hergestellt werden. Beispielsweise können 5-Amino-1-R-tetrazole nach der in Journal of the American Chemical Society (1954), 76, 923-924 beschriebenen Methode aus Amino-tetrazol hergestellt werden:

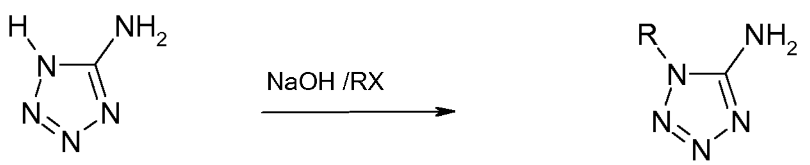


In der oben genannten Formel steht R beispielsweise für einen Alkylrest. 5-Amino-1-R-tetrazole können zum Beispiel wie in Journal of the American Chemical Society (1954) 76, 88-89 beschrieben, synthetisiert werden:

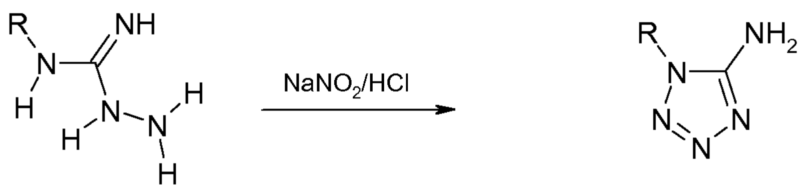


Die 5-Amino-1 H-triazole der Formel (III) sind entweder käuflich erhältlich oder können analog zu literaturbekannten Methoden hergestellt werden. Beispielsweise können 5-Amino-1-R-triazole nach der Zeitschrift füuer Chemie (1990), 30(12), 436 - 437 beschriebenen Methode aus Aminotriazol hergestellt werden:

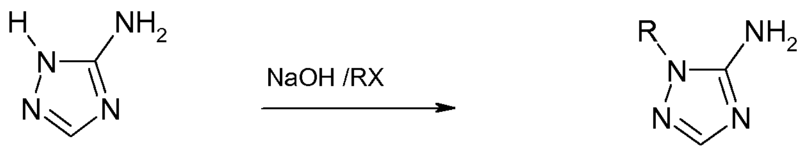


5-Amino-1-R-triazole können auch zum Beispiel wie in Chemische Berichte (1964), 97(2), 396-404 beschrieben, synthetisiert werden:

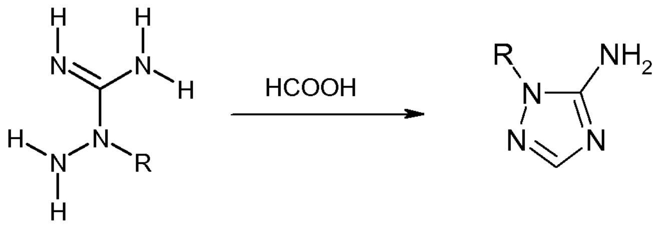


5-Amino-1-R-triazole können auch zum Beispiel wie in Angewandte Chemie (1963), 75, 918 beschrieben, synthetisiert werden:

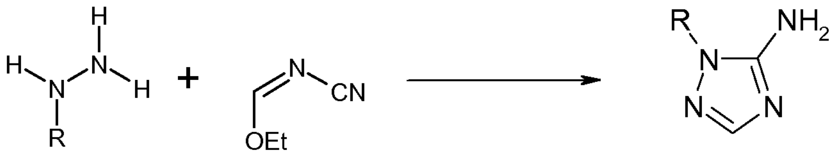

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, Iowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasenunterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Bevorzugt ist die Anwendung der erfindungsgemäßen Verbindungen oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen

- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualität auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt, siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY, oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise

Braun et al., EMBO J. 11 (1992), 3219-3227, Wolter et al., Proc. Natl. Acad. Sci. USA 85 (1988), 846-850, Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie",Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispielsweise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder

Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 15th edition, The British Crop Protection Council and the Royal Soc. of Chemistry, 2009 und dort zitierter Literatur beschrieben sind.

Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:

Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, Beflubutamid, Benazolin, Benazolin-ethyl, Bencarbazone, Benfluralin, Benfuresate, Bensulide, Bensulfuron, Bensulfuron-methyl,

Bentazone, Benzfendizone, Benzobicyclon, Benzofenap, Benzofluor, Benzoylprop, Bicyclopyrone, Bifenox, Bilanafos, Bilanafos-natrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenacnatrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthaldimethyl, Chlorotoluron, Chlorsulfuron, Cinidon, Cinidon-ethyl, Cinmethylin, Cinosulfuron, Clethodim, Clodinafop, Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dimefuron, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3-fluorpropyl)-4,5-dihydro-5-oxo-1H-tetrazol-1-yl]-phenyl]-ethansulfonamid, F-7967, d. h. 3-[7-Chlor-5-fluor-2-(trifluormethyl)-1H-benzimidazol-4-yl]-1-methyl-6-(trifluormethyl)pyrimidin-2,4(1H,3H)-dion, Fenoprop, Fenoxaprop, Fenoxaprop-P, Fenoxaprop-ethyl, Fenoxaprop-P-ethyl, Fenoxasulfone, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, Glufosinate-ammonium, Glufosinate-P, Glufosinate-P-ammonium, Glufosinate-P-natrium, Glyphosate, Glyphosate-isopropylammonium, H-9201, d. h. O-(2,4-Dimethyl-6-nitrophenyl)-O-ethyl-isopropylphosphoramidothioat, Halosafen, Halosulfuron, Halosulfuron-methyl, Haloxyfop, Haloxyfop-P, Haloxyfop-ethoxyethyl, Haloxyfop-P-ethoxyethyl, Haloxyfopmethyl, Haloxyfop-P-methyl, Hexazinone, HW-02, d. h. 1-(Dimethoxyphosphoryl)-ethyl(2,4-dichlorphenoxy)acetat, Imazamethabenz, Imazamethabenz-methyl, Imazamox, Imazamox-ammonium, Imazapic, Imazapyr, Imazapyrisopropylammonium, Imazaquin, Imazaquin-ammonium, Imazethapyr, Imazethapyrammonium, Imazosulfuron, Inabenfide, Indanofan, Indaziflam, Indolessigsäure (IAA), 4-Indol-3-ylbuttersäure (IBA), Iodosulfuron, Iodosulfuron-methyl-natrium, Ioxynil, Ipfencarbazone, Isocarbamid, Isopropalin, Isoproturon, Isouron, Isoxaben, Isoxachlortole, Isoxaflutole, Isoxapyrifop, KUH-043, d. h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Metazasulfuron, Methazole, Methiopyrsulfuron, Methiozolin, Methoxyphenone, Methyldymron, 1-Methylcyclopropen, Methylisothiocyanat, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monosulfuron-ester, Monuron, MT-128, d. h. 6-Chlor-N-[(2E)-3-chlorprop-2-en-1-yl]-5-methyl-N-phenylpyridazin-3-amin, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-Dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron, Pyrazosulfuron-ethyl, Pyrazoxyfen, Pyribambenz, Pyribambenz-isopropyl, Pyribambenz-propyl, Pyribenzoxim, Pyributicarb, Pyridafol, Pyridate, Pyriftalid, Pyriminobac, Pyriminobac-methyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, d. h. Methyl-(2R)-2-({7-[2-chlor-4-(trifluormethyl)phenoxy]-2-naphthyl}oxy)propanoat, Sulcotrione, Sulfallate (CDEC), Sulfentrazone, Sulfometuron, Sulfometuron-methyl, Sulfosate (Glyphosate-trimesium), Sulfosulfuron, SYN-523, SYP-249, d. h. 1-Ethoxy-3-methyl-1-oxobut-3-en-2-yl-5-[2-chlor-4-(trifluormethyl)phenoxy]-2-nitrobenzoat, SYP-300, d. h. 1-[7-Fluor-3-oxo-4-(prop-2-in-1-yl)-3,4-dihydro-2H-1,4-benzoxazin-6-yl]-3-propyl-2-thioxoimidazolidin-4,5-dion, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapac-ethyl, Tritosulfuron, Tsitodef, Uniconazole, Uniconazole-P, Vernolate, ZJ-0862, d. h. 3,4-Dichlor-N-{2-[(4,6-dimethoxypyrimidin-2-yl)oxy]benzyl}anilin, sowie die folgenden Verbindungen:

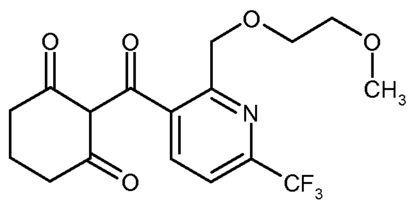
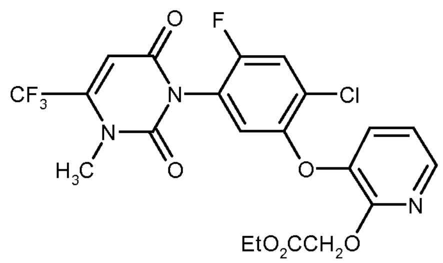

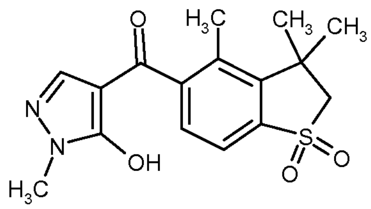
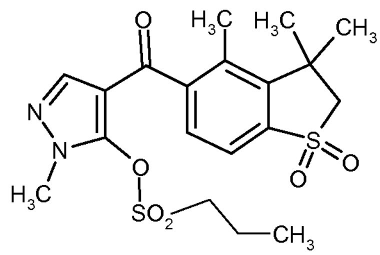

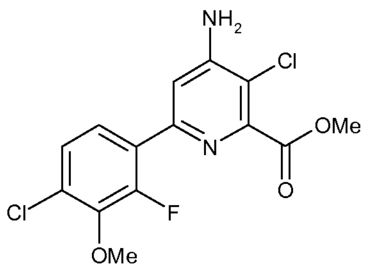
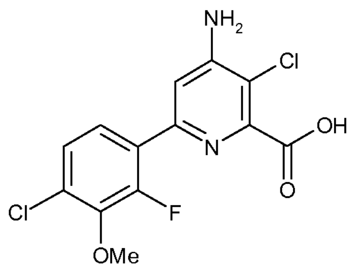

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

A. Chemische Beispiele

Synthese von 2-Chlor-5-methyl-3-methylthio-N-(1-methyltetrazol-5-yl)-benzamid (Nr. 2-27)

Schritt 1: Synthese von N-(Trifluoracetyl)-2,6-dibrom-4-methylanilin

50.0 g (189 mmol) 2,6-Dibrom-4-methylanilin wurden in 500 ml trockenem Dichlormethan unter Argon-Atmosphäre auf eine Temperatur von 0 °C - 5 °C abgekühlt. Bei dieser Temperatur wurden 15.7 g (198 mmol) Pyridin zugegeben. Anschließend wurde bei einer Temperatur von 5°C - 7°C 41.6 g (198 mmol) Trifluoressigsäureanhydrid tropfenweise zugegeben. Das Reaktionsgemisch wurde 1 h bei dieser Temperatur und dann 16 h bei Raumtemperatur (RT) gerührt. Zur Aufarbeitung wurde der Inhalt einmal mit Wasser und anschließend zweimal mit 1 M Salzsäure gewaschen. Die organische Phase wurde getrocknet und das Filtrat wurde vom Lösungsmittel befreit. Als Rückstand wurden 69.3 g N-(Trifluoracetyl)-2,6-dibrom-4-methylanilin gewonnen.

Schritt 2: Synthese von N-(Trifluoracetyl)-2-brom-4-methyl-6-(methylthio)anilin

69.3 g (95 Gew.-% Reinheit, 182 mmol) N-(Trifluoracetyl)-2,6-dibrom-4-methylanilin wurden in 900 ml trockenem Diethylether und unter Argon-Atmosphäre auf eine Temperatur von -70°C abgekühlt. Bei dieser Temperatur wurden 153 ml einer 2.5M Lösung (382.5 mmol) von n-Butyllithium in Hexan tropfenweise zugegeben. Das Gemisch wurde anschließend noch 30 Minuten bei dieser Temperatur gerührt. Danach wurde bei dieser Temperatur eine Lösung von 51.5 g (547 mmol) Dimethyldisulfid in 100 ml trockenem Diethylether tropfenweise zugegeben. Der Inhalt wurde 1 h bei dieser Temperatur, danach 3 h bei RT gerührt und dann auf 1000 ml 1M Salzsäure gegossen. Nach der Phasentrennung wurde die wässrige Phase mit Diethylether extrahiert, die vereinigten organischen Phasen wurden getrocknet und das Filtrat wurde von Lösungsmitteln befreit. Der Rückstand wurde 30 Minuten mit Heptan gerührt und danach filtriert. Als Rückstand wurden 46.6 g N-(Trifluoracetyl)-2-brom-4-methyl-6-(methylthio)anilin isoliert.

Schritt 3: Synthese von 5-Methyl-3-(methylthio)-2-(trifluoracetylamino)benzoesäure

46.6 g (142 mmol) N-(Trifluoracetyl)-2-brom-4-methyl-6-(methylthio)anilin wurden in 750 ml trockenem Diethylether und unter Argon-Atmosphäre auf eine Temperatur von -65 °C abgekühlt. Bei dieser Temperatur wurden 119 ml einer 2.5M Lösung (297.5 mmol) von n-Butyllithium in Hexan tropfenweise zugegeben. Das Gemisch wurde anschließend 2 h bei dieser Temperatur gerührt. Danach wurde der Inhalt vorsichtig auf eine Mischung aus 62.5 g (1.42 mol) Kohlendioxid als gemörsertem Trockeneis und 150 ml trockenem Ether gegossen. Das Gemisch wurde auf RT aufgetaut und dreimal mit Wasser extrahiert. Die vereinigten wässrigen Phasen wurden mit verdünnter Salzsäure auf einen pH-Wert von pH < 2 angesäuert und auf eine Temperatur von 0°C - 5°C abgekühlt. Das Gemisch wurde filtriert, wobei als Rückstand 31.0 g 5-Methyl-3-(methylthio)-2-(trifluoracetylamino)benzoesäure gewonnen wurden.

Schritt 4: Synthese von 2-Amino-5-methyl-3-(methylthio)benzoesäure

22.0 g (75.0 mmol) 5-Methyl-3-(methylthio)-2-(trifluoracetylamino)benzoesäure und 9.44 g (225 mmol) Lithiumhydroxidmonohydrat wurden in einem Gemisch aus 200 ml Methanol und 30 ml Wasser 4 h unter Rückfluss erhitzt. Zur Aufarbeitung wurde der Inhalt abgekühlt und von den Lösungsmitteln befreit. Der Rückstand wurde in Wasser aufgenommen und das Gemisch wurde mit verdünnter Salzsäure auf einen pH-Wert von pH < 3 angesäuert. Das Gemisch wurde 30 Minuten bei einer Temperatur von 0°C - 5°C gerührt und anschließend filtriert. Als Rückstand wurden 15.0 g 2-Amino-5-methyl-3-(methylthio)benzoesäure mit einer Reinheit von > 95 Gew.-% gewonnen.

Schritt 5: Synthese von 2-Chlor-5-methyl-3-(methylthio)benzoesäure

6.00 g (30.4 mmol) 2-Amino-5-methyl-3-(methylthio)benzoesäure wurden in 35 ml Wasser mit 13 ml konzentrierter Salzsäure versetzt und einige Minuten auf 60°C erhitzt. Die Lösung wurde danach auf eine Temperatur von 0°C - 5°C abgekühlt und bei dieser Temperatur wurde eine Lösung von 2.31 g (33.5 mmol) Natriumnitrit in 10 ml Wasser langsam zugetropft. Das Gemisch wurde danach noch 1 h bei dieser Temperatur gerührt. Diese Lösung wurde tropfenweise zu einer auf RT befindliche Mischung aus 4.52 g (45.6 mmol) Kupfer(I)chlorid, 35 ml Wasser und 27 ml konzentrierter Salzsäure gegeben. Das Gemisch wurde 15 Minuten bei Raumtemperatur gerührt und anschließend langsam auf Rückflusstemperatur erhitzt. Der Inhalt wurde danach 15 Minuten unter Rückfluss erhitzt. Zur Aufarbeitung wurde das Reaktionsgemisch abgekühlt, filtriert und der Rückstand wurde mit Wasser gewaschen. Als Rückstand wurden 5.62 g 2-Chlor-5-methyl-3-(methylthio)benzoesäure isoliert.

Schritt 6: Synthese von 2-Chlor-5-methyl-3-methylthio-N-(1-methyltetrazol-5-yl)-benzamid (Nr. 2-16)

500 mg (2.31 mmol) 2-Chlor-5-methyl-3-(methylthio)benzoesäure und 320 mg (3.23 mmol) 5-Amino-1-methyltetrazol sowie eine katalytische Menge 4-(Dimethylamino)-pyridin wurden in 10 ml Pyridin gelöst. Anschließend wurden 410 mg (3.23 mmol) Oxalylchlorid vorsichtig zugetropft. Nach zehn Minuten wurde das Gemisch 4 h lang bei 80°C gerührt. Zur Vervollständigung der Reaktion wurden dann bei RT 137 mg (1.08 mmol) Oxalylchlorid nachgegeben und das Gemisch nochmals 4 h bei 80°C gerührt. Zur Aufarbeitung wurde der Inhalt auf RT abgekühlt, am Rotationsverdanpfer

vom Lösungsmittel befreit und der Rückstand wurde in Dichlormethan gelöst. Die Lösung wurde mit gesättigter, wässriger Natriumhydrogencarbonat-Lösung gewaschen und die organische Phase wurde anschließend vom Lösungsmittel befreit. Der Rückstand wurde chromatographisch gereinigt, wobei 495 mg 2-Chlor-5-methyl-3-methylthio-N-(1-methyltetrazol-5-yl)-benzamid gewonnen wurden.

Die in den nachfolgenden Tabellen aufgeführten Beispiele wurden analog oben genannten Methoden hergestellt beziehungsweise sind analog oben genannten Methoden erhältlich. Die in den nachfolgenden Tabellen aufgeführten Verbindungen sind ganz besonders bevorzugt.

Die verwendeten Abkürzungen bedeuten:

Et = Ethyl Me = Methyl

Tabelle 1: Erfindungsgemäße Verbindungen der allgemeinen Formel (I), worin A für CY und B für CH steht

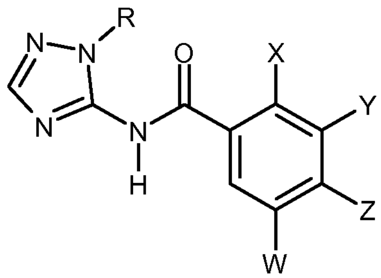

| Nr. | R | X | Y | Z | W | Physikalische Daten ($^1$H-NMR, DMSO-$d_6$, 400 MHz) |
|---|---|---|---|---|---|---|
| 1-1 | Me | I | SMe | H | Me | 7.37 (s,1H), 7.05 (s,1H), 7.00 (s,1H), 3.97 (s,3H), 2.50 (s,3H), 2.36 (s,3H) in $CDCl_3$ |
| 1-2 | Me | I | $SO_2Me$ | H | Me | 8.15 (s,1H), 7.63 (s,1H), 7.48 (s,1H), 3.95 (s,3H), 3.29 (s,3H), 2.44 (s,3H) in $CDCl_3$ |
| 1-3 | Me | I | SOMe | H | Me | 7.71 - 7.69 (m,2H), 7.48 (s,1H), 3.96 (s,3H), 2.76 (s,3H), 2.49 (s,3H) in $CDCl_3$ |
| 1-4 | Me | Br | SMe | H | Me | 7.34 (s,1 H), 7.12 (s,1 H), 7.05 (s,1H), 3.92 (s,3H), 2.51 (s,3H), 2.36 (s,3H) in $CDCl_3$ |
| 1-5 | Me | Br | SOMe | H | Me | 7.80 (s,1 H), 7.75 (s,1H), 7.57 (s,1H), 3.92 (s,3H), 2.82 (s,3H), 2.48 (s,3H) in $CDCl_3$ |
| 1-6 | Me | Br | $SO_2Me$ | H | Me | 8.14 (s,1 H), 7.70 (s,1 H), 7.60 (s,1H), 3.91 (s,3H), 3.31 (s,3H), 2.45 (s,3H) in $CDCl_3$ |
| 1-7 | Me | Cl | SMe | H | Me | |
| 1-8 | Me | Cl | SOMe | H | Me | |
| 1-9 | Me | Cl | $SO_2Me$ | H | Me | |
| 1-10 | Me | Me | SMe | H | Me | |
| 1-11 | Me | Me | SOMe | H | Me | |
| 1-12 | Me | Me | $SO_2Me$ | H | Me | |
| 1-13 | Me | Cl | H | F | Me | 11.14 (brs,1H), 7.88 (s,1H), 7.68 (d,1H), 7.53 (d,1H), 3.75 (s,3H), 2.56 (s,3H), 2.28 (s,3H) |
| 1-14 | Me | Cl | H | SMe | Me | 11.04 (brs,1H), 7.88 (s,1H), 7.51 (s,1 H), 7.28 (s,1 H), 3.74 (s,3H), 2.56 (s,3H), 2.26 (s,3H) |

(fortgesetzt)

| Nr. | R | X | Y | Z | W | Physikalische Daten ($^1$H-NMR, DMSO-$d_6$, 400 MHz) |
|---|---|---|---|---|---|---|
| 1-15 | Me | Cl | H | $SO_2Me$ | Me | 11.42 (brs,1H), 7.99 (s,1H), 7.93 (s,1 H), 7.88 (s,1H), 3.78 (s,3H), 3.34 (s,3H), 2.68 (s,3H) |

Tabelle 2: Erfindungsgemäße Verbindungen der allgemeinen Formel (I), worin A für CY und B für N steht

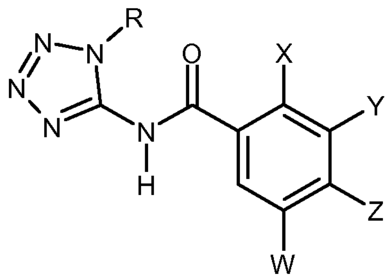

| Nr. | R | X | Y | Z | W | Physikalische Daten ($^1$H-NMR, DMSO-$d_6$, 400 MHz) |
|---|---|---|---|---|---|---|
| 2-1 | Me | Me | SMe | H | Me | 7.16 (s,1H), 4.11 (s,3H), 2.49 (s,3H), 2.45 (s,3H), 2.40 (s,3H) |
| 2-2 | Me | Me | SOMe | H | Me | 7.92 (s,1H), 7.59 (s,1H), 4.11 (s,3H), 2.70 (s,3H), 2.50 (s,3H), 2.42 (s,3H) |
| 2-3 | Me | Me | $SO_2Me$ | H | Me | 8.11 (s,1H), 7.77 (s,1H), 4.14 (s,3H), 3.14 (s,3H), 2.82 (s,3H), 2.49 (s,3H) |
| 2-4 | Et | Me | SMe | H | Me | 7.16 (s,1H), 4.47 (q,2H), 2.49 (s,3H), 2.44 (s,3H), 2.40 (s,3H), 1.63 (t,3H) |
| 2-5 | Et | Me | SOMe | H | Me | 7.86 (s,1H), 7.59 (s,1H), 4.46 (q,2H), 2.69 (s,3H), 2.50 (s,3H), 2.41 (s,3H), 1.64 (t,3H) |
| 2-6 | Et | Me | $SO_2Me$ | H | Me | 8.11 (s,1H), 7.74 (s,1H), 4.51 (q,2H), 3.15 (s,3H), 2.83 (s,3H), 2.49 (s,3H), 1.63 (t,3H) |
| 2-7 | n-Pr | Me | SMe | H | Me | |
| 2-8 | n-Pr | Me | SOMe | H | Me | |
| 2-9 | n-Pr | Me | $SO_2Me$ | H | Me | |
| 2-10 | Me | $NH_2$ | SMe | H | Me | |
| 2-11 | Me | $NH_2$ | SOMe | H | Me | 8.06 (s,1H), 7.29 (s,1H), 4.05 (s,3H), 2.93 (s,3H), 2.37 (s,3H) |
| 2-12 | Me | $NH_2$ | $SO_2Me$ | H | Me | |
| 2-13 | Me | $NH(C{=}O)CF_3$ | SMe | H | Me | 7.55 (s,1H), 7.35 (s,1H), 4.08 (s,3H), 2.52 (s,3H), 2.41 (s,3H) |
| 2-14 | Me | $NH(C{=}O)CF_3$ | SOMe | H | Me | |

(fortgesetzt)

| Nr. | R | X | Y | Z | W | Physikalische Daten ($^1H$-NMR, DMSO-$d_6$, 400 MHz) |
|---|---|---|---|---|---|---|
| 2-15 | Me | $NH(C=O)CF_3$ | $SO_2Me$ | H | Me | 8.01 (brs,2H), 4.03 (brs,3H), 3.13 (s,3H), 2.47 (brs,3H) |
| 2-16 | Me | Cl | SMe | H | Me | 7.32 (s,1H), 7.14 (s,1H), 4.12 (s,3H), 2.52 (s,3H), 2.41 (s,3H) |
| 2-17 | Me | Cl | SOMe | H | Me | 7.82 (s,1H), 7.65 (s,1H), 4.12 (s,3H), 2.83 (s,3H), 2.52 (s,3H) |
| 2-18 | Me | Cl | $SO_2Me$ | H | Me | 8.20 (s,1H), 7.75 (s,1H), 4.14 (s,3H), 3.32 (s,3H), 2.51 (s,3H) |
| 2-19 | Et | Cl | SMe | H | Me | 7.31 (s,1H), 7.14 (s,1H), 4.48 (q,2H), 2.52 (s,3H), 2.41 (s,3H), 1.63 (t,3H) |
| 2-20 | Et | Cl | SOMe | H | Me | 7.77 (s,1H), 7.63 (s,1H), 4.48 (q,2H), 2.82 (s,3H), 2.51 (s,3H), 1.64 (t,3H) |
| 2-21 | Et | Cl | $SO_2Me$ | H | Me | 8.18 (s,1H), 7.75 (s,1H), 4.51 (q,2H), 3.34 (s,3H), 2.51 (s,3H), 1.62 (t,3H) |
| 2-22 | n-Pr | Cl | SMe | H | Me | 7.31 (s,1H), 7.13 (s,1H), 4.41 (t,2H), 2.51 (s,3H), 2.41 (s,3H), 2.03 (sex,2H), 0.99 (t,3H) |
| 2-23 | n-Pr | Cl | SOMe | H | Me | 7.77 (s,1H), 7.63 (s,1H), 4.41 (t,2H), 2.82 (s,3H), 2.52 (s,3H), 2.05 (sex,2H), 1.01 (t,3H) |
| 2-24 | n-Pr | Cl | $SO_2Me$ | H | Me | 8.18 (s,1H), 7.73 (s,1H), 4.44 (t,2H), 3.34 (s,3H), 2.50 (s,3H), 2.03 (sex,2H), 0.99 (t,3H) |
| 2-25 | Me | Br | SMe | H | Me | 7.20 (s,1H), 7.08 (s,1H), 4.15 (s,3H), 2.51 (s,3H), 2.39 (s,3H) |
| 2-26 | Me | Br | SOMe | H | Me | 7.82 (s,1H), 7.57 (s,1H), 4.15 (s,3H), 2.82 (s,3H), 2.51 (s,3H) |
| 2-27 | Me | Br | $SO_2Me$ | H | Me | 8.21 (s,1H), 7.64 (s,1H), 4.17 (s,3H), 3.33 (s,3H), 2.49 (s,3H) |
| 2-28 | Et | Br | SMe | H | Me | 7.19 (s,1H), 7.08 (s,1H), 4.51 (q,2H), 2.51 (s,3H), 2.39 (s,3H), 1.65 (t,3H) |
| 2-29 | Et | Br | SOMe | H | Me | 7.72 (s,1H), 7.55 (s,1H), 4.50 (q,2H), 2.79 (s,3H), 2.51 (s,3H), 1.66 (t,3H) |
| 2-30 | Et | Br | $SO_2Me$ | H | Me | 8.19 (s,1H), 7.63 (s,1H), 4.53 (q,2H), 3.33 (s,3H), 2.49 (s,3H), 1.65 (t,3H) |
| 2-31 | n-Pr | Br | SMe | H | Me | 7.19 (s,1H), 7.08 (s,1H), 4.44 (t,2H), 2.51 (s,3H), 2.39 (s,3H), 2.06 (sex,2H), 1.00 (t,3H) |

(fortgesetzt)

| Nr. | R | X | Y | Z | W | Physikalische Daten ($^1$H-NMR, DMSO-$d_6$, 400 MHz) |
|---|---|---|---|---|---|---|
| 2-32 | n-Pr | Br | SOMe | H | Me | 7.70 (s,1H), 7.54 (s,1H), 4.43 (t,2H), 2.78 (s,3H), 2.51 (s,3H), 2.07 (sex,2H), 1.03 (t,3H) |
| 2-33 | n-Pr | Br | $SO_2Me$ | H | Me | 8.20 (s,1H), 7.62 (s,1 H), 4.45 (t,2H), 3.33 (s,3H), 2.49 (s,3H), 2.06 (sex,2H), 1.01 (t,3H) |
| 2-34 | Me | I | SMe | H | Me | 7.11 (s,1 H), 7.03 (s,1 H), 4.20 (s,3H), 2.50 (s,3H), 2.39 (s,3H) in $CDCl_3$ |
| 2-35 | Me | I | SOMe | H | Me | 7.70 (s,1H), 7.64 (s,1H), 4.04 (s,3H), 2.79 (s,3H), 2.45 (s,3H) |
| 2-36 | Me | I | $SO_2Me$ | H | Me | |
| 2-37 | Me | OMe | SMe | H | F | 11.45 (brs,1H), 7.29 (2d,2H), 3.98 (s,3H), 3.80 (s,3H), 2.5 (s,3H) |
| 2-38 | Me | OMe | $SO_2Me$ | H | F | 11.94 (brs,1H), 8.01 (dd,1H), 7.80 (dd,1H), 4.02 (s,3H), 3.94 (s,3H), 3.37 (s,3H) |
| 2-39 | Me | Cl | H | $SO_2Me$ | Me | 11.96 (brs,1H), 8.00 (s,1H), 7.91 (s,1H), 4.01 (s,3H), 3.35 (s,3H), 2.68 (s,3H) |
| 2-40 | Me | Cl | H | SOMe | Cl | 8.18 (s,1H), 7.88 (s,1H), 4.02 (s,3H), 2.90 (s,3H) |
| 2-41 | Me | Cl | H | $SO_2Me$ | Cl | 8.32 (s,1 H), 8.11 (s,1 H), 4.02 (s,3H), 3.48 (s,3H) |
| 2-42 | Me | Cl | Me | F | Me | 7.56 (d,1H), 3.98 (s,3H), 2.31 (d,3H), 2.28 (d,3H) |
| 2-43 | Me | Cl | Me | SMe | Me | |
| 2-44 | Me | Cl | Me | SOMe | Me | 7.53 (s,1H), 4.00 (s,3H), 2.96 (s,3H), 2.66 (s,3H), 2.57 (s,3H) |
| 2-45 | Me | Cl | Me | $SP_2Me$ | Me | 11.88 (brs,1H), 7.69 (s,1H), 4.01 (s,3H), 3.34 (s,3H), 2.76 (s,3H), 2.69 (s,3H) |
| 2-46 | Et | Cl | Me | SMe | Me | |
| 2-47 | Et | Cl | Me | SOMe | Me | 7.53 (s,1H), 4.36 (q,2H), 2.96 (s,3H), 2.66 (s,3H), 2.57 (s,3H), 1.47 (t,3H) |
| 2-48 | Et | Cl | Me | $SO_2Me$ | Me | 11,78 (brs,1H), 7.68 (s,1H), 4.37 (q,2H), 3.34 (s,3H), 2.76 (s,3H), 2.69 (s,3H), 1.48 (t,3H) |
| 2-49 | Pr | Cl | Me | SMe | Me | |
| 2-50 | Pr | Cl | Me | SOMe | Me | 7.53 (s,1H), 4.32 (t,2H), 2.96 (s,3H), 2.66 (s,3H), 2.57 (s,3H), 1.88 (m,2H), 0.88 (t,3H) |

(fortgesetzt)

| Nr. | R | X | Y | Z | W | Physikalische Daten ($^1$H-NMR, DMSO-$d_6$, 400 MHz) |
|---|---|---|---|---|---|---|
| 2-51 | Pr | Cl | Me | $SO_2Me$ | Me | 7.66 (s,1H), 4.32 (t,2H), 3.31 (s,3H), 2.76 (s,3H), 2.70 (s,3H), 1.88 (m,2H), 0.88 (t,3H) |
| 2-52 | Me | Cl | OMe | SMe | OMe | 9.95 (brs,1H), 7.14 (s,1H), 4.11 (s,3H), 3.97 (s,3H), 3.94 (s,3H), 2.50 (s,3H) |
| 2-53 | Me | Cl | OMe | $SO_2Me$ | OMe | 11.92 (brs,1H), 7.49 (s,1H), 4.03 (s,3H), 3.98 (s,3H), 3.89 (s,3H), 3.37 (s,3H) |
| 2-54 | Me | Cl | $CO_2Me$ | Cl | Me | |
| 2-55 | Me | Cl | $CO_2Me$ | SMe | Me | |
| 2-56 | Me | Cl | $CO_2Me$ | $SO_2Me$ | Me | |
| 2-57 | Me | Cl | $CO_2Me$ | Cl | Cl | |
| 2-58 | Me | Cl | $CO_2Me$ | SMe | Cl | |
| 2-59 | Me | Cl | $CO_2Me$ | $SO_2Me$ | Cl | |
| 2-60 | Me | Me | $NH_2$ | $SO_2Me$ | Me | 8.67 (s,1H), 6.81 (s,1H), 5.72 (s,2H), 4.12 (s,3H), 3.11 (s,3H), 2.66 (s,3H), 2.26 (s, 3H) |
| 2-61 | Me | Me | NMe2 | $SO_2Me$ | Me | 11.65 (brs,1H), 7.48 (s,1H), 4.00 (s,3H), 3.38 (s,3H), 2.80 (s,6H), 2.62 (s,3H), 2.35 (s,3H) |
| 2-62 | Me | Me | NHEt | $SO_2Me$ | Me | 10.66 (brs,1H), 7.13 (s,1H), 6.28 (brs,1H), 4.11 (s,3H), 3.15 (q,2H), 3.14 (s,3H), 2.68 (s,3H), 2.41 (s,3H), 1.26 (t,3H) |
| 2-63 | Me | Cl | H | SMe | Cl | 11.82 (brs,1H), 7.90 (s,1H), 7.41 (s,1H), 3.95 (s,3H), 2.60 (s,3H) |
| 2-64 | Me | Cl | H | SMe | Me | 11.61 (brs,1H), 7.57 (s,1H), 7.30 (s,1H), 3.97 (s,3H), 2.57 (s,3H), 2.27 (s,3H) |
| 2-65 | Me | Cl | H | SOMe | Me | 11.85 (brs,1H), 7.85 (s,1H), 7.73 (s,1H), 4.00 (s,3H), 2.75 (s,3H), 2.38 (s,3H) |
| 2-66 | Me | F | H | CN | F | 11.95 (brs,1H), 8.25 (dd,1H), 8.05 (dd,1H), 3.98 (s,3H) |
| 2-67 | Et | F | H | CN | F | 11.89 (brs,1H), 8.25 (dd,1H), 8.06 (dd,1H), 4.33 (q,2H), 1.45 (t,3H) |
| 2-68 | Et | Cl | H | $SO_2Me$ | Cl | |
| 2-69 | Et | Me | NMe2 | $SO_2Me$ | Me | 11.57 (brs,1H), 7.47 (s,1H), 4.34 (q,2H), 3.38 (s,3H), 2.80 (s,6H), 2.62 (s,3H), 2.35 (s,3H), 1.48 (t,3H) |
| 2-70 | Me | Cl | H | F | Me | |
| 2-71 | Et | Cl | H | Cl | Cl | 11.85 (brs,1H), 8.17 (s,1H), 8.07 (s,1H), 4.36 (q,2H), 1.47 (t,3H) |

(fortgesetzt)

| Nr. | R | X | Y | Z | W | Physikalische Daten ($^1$H-NMR, DMSO-$d_6$, 400 MHz) |
|---|---|---|---|---|---|---|
| 2-72 | $C_2H_4OMe$ | Me | SMe | H | Me | |
| 2-73 | $C_2H_4OMe$ | Me | SOMe | H | Me | |
| 2-74 | $C_2H_4OMe$ | Me | $SO_2Me$ | H | Me | |
| 2-75 | $C_2H_4OMe$ | Cl | SMe | H | Me | |
| 2-76 | $C_2H_4OMe$ | Cl | SOMe | H | Me | |
| 2-77 | $C_2H_4OMe$ | Cl | $SO_2Me$ | H | Me | |
| 2-78 | $C_2H_4OMe$ | Br | SMe | H | Me | |
| 2-79 | $C_2H_4OMe$ | Br | SOMe | H | Me | |
| 2-80 | $C_2H_4OMe$ | Br | $SO_2Me$ | H | Me | |
| 2-81 | $C_2H_4OMe$ | Cl | Me | SMe | Me | |
| 2-82 | $C_2H_4OMe$ | Cl | Me | $SO_2Me$ | Me | |
| 2-83 | $C_2H_4OMe$ | Me | NMe2 | $SO_2Me$ | Me | 10.02 (brs,1H), 7.31 (s,1H), 4.64 (t,4H), 3.84 (t,2H), 3.37 (s,3H), 3.33 (s,3H), 2.88 (s,6H), 2.70 (s,3H), 2.45 (s,3H) |

Tabelle 3: Erfindungsgemäße Verbindungen der allgemeinen Formel (I), worin A und B für N steht

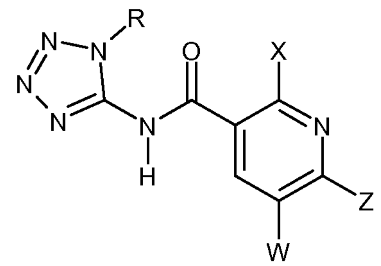

| Nr. | R | X | Z | W | Physikalische Daten ($^1$H-NMR, DMSO-$d_6$,400 MHz) |
|---|---|---|---|---|---|
| 3-1 | Me | Me | $CF_3$ | Me | |
| 3-2 | Me | Me | $CF_3$ | F | |
| 3-3 | Me | Me | $CF_3$ | Cl | |
| 3-4 | Me | Me | Cl | Me | |
| 3-5 | Me | Me | Cl | Cl | |
| 3-6 | Me | Me | SMe | Me | |
| 3-7 | Me | Me | $SO_2Me$ | Me | |
| 3-8 | Me | Me | SMe | Cl | |
| 3-9 | Me | Me | $SO_2Me$ | Cl | |
| 3-10 | Me | Cl | $CF_3$ | Me | |
| 3-11 | Me | Cl | $CF_3$ | F | |
| 3-12 | Me | Cl | $CF_3$ | Cl | |

(fortgesetzt)

| Nr. | R | X | Z | W | Physikalische Daten ($^1$H-NMR, DMSO-$d_6$,400 MHz) |
|---|---|---|---|---|---|
| 3-13 | Me | Cl | Cl | Me | |
| 3-14 | Me | Cl | Cl | F | 12.08 (brs,1H), 8.61 (d,1H), 4.01 (s,3H) |
| 3-15 | Et | Cl | Cl | F | 11.99 (brs,1H), 8.62 (d,1H), 4.37 (q,2H), 1.48 (t,3H) |
| 3-16 | Me | Cl | Cl | Cl | |
| 3-17 | Me | Cl | SMe | Me | |
| 3-18 | Me | Cl | $SO_2Me$ | Me | |
| 3-19 | Me | Cl | SMe | F | |
| 3-20 | Me | Cl | $SO_2Me$ | F | |
| 3-21 | Me | Cl | SMe | Cl | |
| 3-22 | Me | Cl | $SO_2Me$ | Cl | |

B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.

b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.

d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.

e) Ein in Wasser dispergierbares Granulat wird erhalten indem man

75 Gew.-Teile einer Verbindung der Formel (I) und/oder deren Salze,
10 Gew.-Teile ligninsulfonsaures Calcium,
5 Gew.-Teile Natriumlaurylsulfat,
3 Gew.-Teile Polyvinylalkohol und
7 Gew.-Teile Kaolin

mischt, auf einer Stiftmühle mahlt und das Pulver in einem Wirbelbett durch Aufsprühen von Wasser als Granulierflüssigkeit granuliert.

f) Ein in Wasser dispergierbares Granulat wird auch erhalten, indem man 25 Gew.-Teile einer Verbindung der Formel (I) und/oder deren Salze,

5 Gew.-Teile 2,2'-dinaphthylmethan-6,6'-disulfonsaures Natrium
2 Gew.-Teile oleoylmethyltaurinsaures Natrium,

1 Gew.-Teil Polyvinylalkohol,
17 Gew.-Teile Calciumcarbonat und
50 Gew.-Teile Wasser

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

C. Biologische Beispiele

1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 1-06, 2-01, 2-02, 2-03, 2-04, 2-05, 2-06, 2-16, 2-19, 2-21, 2-37, 2-43, 2-48, 2-49, 2-64 sowie 2-69 bei einer Aufwandmenge von 320 g/ha jeweils eine mindestens 80%-ige Wirkung gegen Alopecurus myosuroides und Amaranthus retroflexus auf. Die Verbindungen Nr. 2-17, 2-18, 2-20, 2-22, 2-23, 2-24, 2-27, 2-30, 2-33, 2-44, 2-45, 2-46, 2-47 sowie 2-62 zeigen bei einer Aufwandmenge von 320 g/ha jeweils eine mindestens 80%-ige Wirkung gegen Cyperus serotinus und Setaria viridis. Die Verbindungen Nr. 2-25, 2-26, 2-28, 2-29, 2-37, 2-42 sowie 2-34 zeigen bei einer Aufwandmenge von 320 g/ha jeweils eine mindestens 80%-ige Wirkung gegen Abutilon theophrasti und Avena fatua. Die Verbindungen Nr. 2-32, 2-37, 2-39, 2-50, 2-51, 2-61, 2-69 sowie 2-83 zeigen bei einer Aufwandmenge von 320 g/ha jeweils eine mindestens 80%-ige Wirkung gegen Echinochloa crus galli und Veronica persica.

2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 1-02, 1-03, 1-05, 1-06, 1-14, 1-15, 2-01, 2-02, 2-03, 2-04, 2-05, 2-06, 2-16, 2-17, 2-18, 2-19, 2-20, 2-21, 2-22, 2-23, 2-24, 2-25, 2-26, 2-27, 2-28, 2-29, 2-30, 2-31, 2-33, 2-34, 2-35, 2-39, 2-40, 2-41, 2-43, 2-44, 2-45, 2-46, 2-47, 2-48, 2-49, 2-50, 2-51, 2-52, 2-53, 2-60, 2-61, 2-62, 2-63, 2-64, 2-65, 2-69 sowie 2-83 bei einer Aufwandmenge von 80 g/ha jeweils eine mindestens 80%-ige Wirkung gegen Abutilon theophrasti und Veronica persica. Die Verbindungen Nr. 1-14, 2-32, 2-42, 2-66, 2-67, 2-69 sowie 2-83 zeigen bei einer Aufwandmenge von 80 g/ha jeweils eine mindestens 80%-ige Wirkung gegen Amaranthus retroflexus und Stellaria media.

3. Vergleichversuche

Zu Vergleichszwecken wurde die erfindungsgemäße Verbindung Nr. 1-15 und die aus EP 0 049 071 A1 bekannten Verbindung Nr. 118 und Nr. 119 im Nachauflauf bei verschiedenen niedrigen Dosierungen gegen eine Reihe von Schadpflanzen geprüft. Die Bonituren zeigen, dass die erfindungsgemäße Verbindung im Gegensatz zu den aus EP 0 049 071 A1 bekannten Verbindungen auch bei sehr niedrigen Dosierungen eine hohe herbizide Wirkung gegen die Schadpflanzen aufweisen.

| Verbindung | Dosierung [g/ha] | herbizide Wirkung gegen | | |
|---|---|---|---|---|
| | | ABUTH | PHBPU | VERPE |
| erfindungsgemäße Verbindung Nr. 1-15 | 80<br>20<br>5 | 90%<br>90%<br>90% | 80%<br>80%<br>80% | 80%<br>80%<br>40% |
| aus EP 0 049 071 A1 bekannte Verbindung Nr. 118 | 80<br>20<br>5 | 0%<br>0%<br>0% | 0%<br>0%<br>0% | 0%<br>0%<br>0% |
| aus EP 0 049 071 A1 bekannte Verbindung Nr. 119 | 80<br>20<br>5 | 0%<br>0%<br>0% | 0%<br>0%<br>0% | 0%<br>0%<br>0% |

Die verwendeten Abkürzungen bedeuten:

| | | | |
|---|---|---|---|
| ABUTH | Abutilon theophrasti | PHBPU | Pharbitis purpureum |
| VERPE | Veronica persica | | |

## Patentansprüche

**1.** 5-Phenylsubstituierte N-(Tetrazol-5-yl)- und N-(Triazol-5-yl)arylcarbonsäure-amide der Formel (I) oder deren Salze

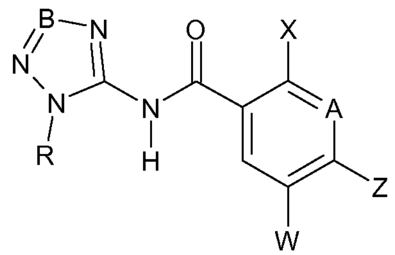

(I),

worin

A bedeutet N oder CY,
B bedeutet N oder CH,
X bedeutet Nitro, Halogen, Cyano, Formyl, Rhodano, $(C_1-C_6)$-Alkyl, Halogen-$(C_1-C_6)$-alkyl, $(C_2-C_6)$-Alkenyl, Halogen-$(C_2-C_6)$-alkenyl, $(C_2-C_6)$-Alkinyl, Halogen-$(C_3-C_6)$-alkinyl, $(C_3-C_6)$-Cycloalkyl, Halogen-$(C_3-C_6)$-cycloalkyl, $(C_3-C_6)$-Cycloalkyl-$(C_1-C_6)$-alkyl, Halogen-$(C_3-C_6)$-cycloalkyl-$(C_1-C_6)$-alkyl, $COR^1$, $COOR^1$, $OCOOR^1$, $NR^1COOR^1$, $C(O)N(R^1)_2$, $NR^1C(O)N(R^1)_2$, $OC(O)N(R^1)_2$, $C(O)NR^1OR^1$, $OR^1$, $OCOR^1$, $OSO_2R^2$, $S(O)_nR^2$, $SO_2OR^1$, $SO_2N(R^1)_2$, $NR^1SO_2R^2$, $NR^1COR^1$, $(C_1-C_6)$-Alkyl-$S(O)_nR^2$, $(C_1-C_6)$-Alkyl-$OR^1$, $(C_1-C_6)$-Alkyl-$OCOR^1$, $(C_1-C_6)$-Alkyl-$OSO_2R^2$, $(C_1-C_6)$-Alkyl-$CO_2R^1$, $(C_1-C_6)$-Alkyl-$SO_2OR^1$, $(C_1-C_6)$-Alkyl-$CON(R^1)_2$, $(C_1-C_6)$-Alkyl-$SO_2N(R^1)_2$, $(C_1-C_6)$-Alkyl-$NR^1COR^1$, $(C_1-C_6)$-Alkyl-$NR^1SO_2R^2$, $NR_1R_2$, $P(O)(OR^5)_2$, $CH_2P(O)(OR^5)_2$, $(C_1-C_6)$-Alkyl-Heteroaryl, $(C_1-C_6)$-Alkyl-Heterocyclyl, wobei die beiden letztgenannten Reste jeweils durch s Reste aus der Gruppe bestehend aus Halogen, $(C_1-C_6)$-Alkyl, Halogen-$(C_1-C_6)$-alkyl, $S(O)_n$-$(C_1-C_6)$-Alkyl, $(C_1-C_6)$-Alkoxy und Halogen-$(C_1-C_6)$-alkoxy substituiert sind, und wobei Heterocyclyl n Oxogruppen trägt,
Y bedeutet Wasserstoff, Nitro, Halogen, Cyano, Rhodano, $(C_1-C_6)$-Alkyl, Halogen-$(C_1-C_6)$-alkyl, $(C_2-C_6)$-Alkenyl, Halogen-$(C_2-C_6)$-alkenyl, $(C_2-C_6)$-Alkinyl, Halogen-$(C_2-C_6)$-alkinyl, $(C_3-C_6)$-Cycloalkyl, $(C_3-C_6)$-Cycloalkenyl, Halogen-$(C_3-C_6)$-cycloalkyl, $(C_3-C_6)$-Cycloalkyl-$(C_1-C_6)$-alkyl, Halogen-$(C_3-C_6)$-cycloalkyl-$(C_1-C_6)$-alkyl, $COR^1$, $COOR^1$, $OCOOR^1$, $NR^1COOR^1$, $C(O)N(R^1)_2$, $NR^1C(O)N(R^1)_2$, $OC(O)N(R^1)_2$, $CO(NOR^1)R^1$, $NR^1SO_2R^2$, $NR^1COR^1$, $OR^1$, $OSO_2R^2$, $S(O)_nR^2$, $SO_2OR^1$, $SO_2N(R^1)_2$ $(C_1-C_6)$-Alkyl-$S(O)_nR^2$, $(C_1-C_6)$-Alkyl-$OR^1$, $(C_1-C_6)$-Alkyl-$OCOR^1$, $(C_1-C_6)$-Alkyl-OSO2$R^2$, $(C_1-C_6)$-Alkyl-$CO_2R^1$, $(C_1-C_6)$-Alkyl-CN, $(C_1-C_6)$-Alkyl-$SO_2OR^1$, $(C_1-C_6)$-Alkyl-$CON(R^1)_2$, $(C_1-C_6)$-Alkyl-$SO_2N(R^1)_2$, $(C_1-C_6)$-Alkyl-$NR^1COR^1$, $(C_1-C_6)$-Alkyl-$NR^1SO_2R^2$, $N(R^1)_2$, $P(O)(OR^5)_2$, $CH_2P(O)(OR^5)_2$, $(C_1-C_6)$-Alkyl-Phenyl, $(C_1-C_6)$-Alkyl-Heteroaryl, $(C_1-C_6)$-Alkyl-Heterocyclyl, Phe-

nyl, Heteroaryl oder Heterocyclyl, wobei die sechs letztgenannten Reste jeweils durch s Reste aus der Gruppe bestehend aus Halogen, Nitro, Cyano, $(C_1-C_6)$-Alkyl, Halogen-$(C_1-C_6)$-alkyl, $(C_3-C_6)$-Cycloalkyl, $S(O)_n$-$(C_1-C_6)$-Alkyl, $(C_1-C_6)$-Alkoxy, Halogen-$(C_1-C_6)$-alkoxy, $(C_1-C_6)$-Alkoxy-$(C_1-C_4)$-alkyl und Cyanomethyl substituiert sind, und wobei Heterocyclyl n Oxogruppen trägt,

Z bedeutet Halogen, Cyano, Rhodano, Halogen-$(C_1-C_6)$-alkyl, $(C_2-C_6)$-Alkenyl, Halogen-$(C_2-C_6)$-alkenyl, $(C_2-C_6)$-Alkinyl, Halogen-$(C_2-C_6)$-alkinyl, $(C_3-C_6)$-Cycloalkyl, Halogen-$(C_3-C_6)$-cycloalkyl, $(C_3-C_6)$-Cycloalkyl-$(C_1-C_6)$-alkyl, Halogen-$(C_3-C_6)$-cycloalkyl-$(C_1-C_6)$-alkyl, $COR^1$, $COOR^1$, $OCOOR^1$, $NR^1COOR^1$, $C(O)N(R^1)_2$, $NR^1C(O)N(R^1)_2$, $OC(O)N(R^1)_2$, $C(O)NR^1OR^1$, $OSO_2R^2$, $S(O)_nR^2$, $SO_2OR^1$, $SO_2N(R^1)_2$, $NR^1SO_2R^2$, $NR^1COR^1$, $(C_1-C_6)$-Alkyl-$S(O)_nR^2$, $(C_1-C_6)$-Alkyl-$OR^1$, $(C_1-C_6)$-Alkyl-$OCOR^1$, $(C_1-C_6)$-Alkyl-$OSO_2R^2$, $(C_1-C_6)$-Alkyl-$CO_2R^1$, $(C_1-C_6)$-Alkyl-$SO_2OR^1$, $(C_1-C_6)$-Alkyl-$CON(R^1)_2$, $(C_1-C_6)$-Alkyl-$SO_2N(R^1)_2$, $(C_1-C_6)$-Alkyl-$NR^1COR^1$, $(C_1-C_6)$-Alkyl-$NR^1SO_2R^2$, $N(R^1)_2$, $P(O)(OR^5)_2$, Heteroaryl, Heterocyclyl oder Phenyl, wobei die drei letztgenannten Reste jeweils durch s Reste aus der Gruppe bestehend aus Halogen, Nitro, Cyano, $(C_1-C_6)$-Alkyl, Halogen-$(C_1-C_6)$-alkyl, $(C_3-C_6)$-Cycloalkyl, $S(O)_n$-$(C_1-C_6)$-Alkyl, $(C_1-C_6)$-Alkoxy oder Halogen-$(C_1-C_6)$-alkoxy substituiert sind, und wobei Heterocyclyl n Oxogruppen trägt, oder

Z kann auch Wasserstoff, $(C_1-C_6)$-Alkyl oder $(C_1-C_6)$-Alkoxy bedeuten, falls Y für den Rest $S(O)_nR^2$ steht,

W bedeutet $(C_1-C_6)$-Alkyl, Halogen-$(C_1-C_6)$-alkyl, $(C_2-C_6)$-Alkenyl, Halogen-$(C_2-C_6)$-alkenyl, $(C_2-C_6)$-Alkinyl, Halogen-$(C_2-C_6)$-alkinyl, $(C_3-C_7)$-Cycloalkyl, $(C_3-C_7)$-Halogencycloalkyl, $(C_1-C_6)$-Alkoxy, $(C_1-C_6)$-Halogenalkoxy, $S(O)_n$-$(C_1-C_6)$-Alkyl, $S(O)_n$-$(C_1-C_6)$-Halogenalkyl, $(C_1-C_6)$-Alkoxy-$(C_1-C_4)$-alkyl, $(C_1-C_6)$-Alkoxy-$(C_1-C_4)$-halogenalkyl, Halogen, Nitro, $NR^3COR^3$ oder Cyano,

R bedeutet $(C_1-C_8)$-Alkyl, Halogen-$(C_1-C_8)$-alkyl, $(C_2-C_8)$-Alkenyl, Halogen-$(C_2-C_8)$-alkenyl, $(C_2-C_8)$-Alkinyl, Halogen-$(C_2-C_8)$-alkinyl, wobei diese sechs vorstehend genannten Reste jeweils durch s Reste aus der Gruppe bestehend aus Hydroxy, Nitro, Cyano, $SiR^5_3$, $PO(OR^5)_2$, $S(O)_n$-$(C_1-C_6)$-Alkyl, $S(O)_n$-$(C_1-C_6)$-Halogenalkyl, $(C_1-C_6)$-Alkoxy, Halogen-$(C_1-C_6)$-alkoxy, $N(R^3)_2$, $COR^3$, $COOR^3$, $OCOR^3$, $NR^3COR^3$, $NR^3SO_2R^4$, $O(C_1-C_2)$-Alkyl-$(C_3-C_6)$-Cycloalkyl, $(C_3-C_6)$-Cycloalkyl, Heteroaryl, Heterocyclyl, Phenyl, Q-Heteroaryl, Q-Heterocyclyl, Q-Phenyl und Q-Benzyl substituiert sind, wobei die sieben letztgenannten Reste jeweils durch s Reste aus der Gruppe bestehend aus Methyl, Ethyl, Methoxy, Trifluormethyl, Cyano und Halogen substituiert sind, und wobei Heterocyclyl n Oxogruppen trägt, oder

R bedeutet jeweils durch s Reste aus der Gruppe bestehend aus Halogen, Nitro, Cyano, $(C_1-C_6)$-Alkyl, Halogen-$(C_1-C_6)$-alkyl, $(C_3-C_6)$-Cycloalkyl, $S(O)_n$-$(C_1-C_6)$-Alkyl, $(C_1-C_6)$-Alkoxy, Halogen-$(C_1-C_6)$-alkoxy und $(C_1-C_6)$-Alkoxy-$(C_1-C_4)$-alkyl substituiertes $(C_3-C_7)$-Cycloalkyl, Heteroaryl, Heterocyclyl oder Phenyl, wobei Heterocyclyl n Oxogruppen trägt,

Q bedeutet O, S, oder $NR^3$,

$R^1$ bedeutet Wasserstoff, $(C_1-C_6)$-Alkyl, $(C_1-C_6)$-Halogenalkyl, $(C_2-C_6)$-Alkenyl, $(C_2-C_6)$-Halogenalkenyl, $(C_2-C_6)$-Alkinyl, $(C_2-C_6)$-Halogenalkinyl, $(C_3-C_6)$-Cycloalkyl, $(C_3-C_6)$-Cycloalkenyl, $(C_3-C_6)$-Halogencycloalkyl, $(C_1-C_6)$-Alkyl-O-$(C_1-C_6)$-alkyl, $(C_3-C_6)$-Cycloalkyl-$(C_1-C_6)$-alkyl, Phenyl, Phenyl-$(C_1-C_6)$-alkyl, Heteroaryl, $(C_1-C_6)$-Alkyl-Heteroaryl, Heterocycl, $(C_1-C_6)$-Alkyl-Heterocyclyl, $(C_1-C_6)$-Alkyl-O-Heteroaryl, $(C_1-C_6)$-Alkyl-O-Heterocyclyl, $(C_1-C_6)$-Alkyl-$NR^3$-Heteroaryl oder $(C_1-C_6)$-Alkyl-$NR^3$-Heterocyclyl wobei die 21 letztgenannten Reste jeweils durch s Reste aus der Gruppe bestehend aus Cyano, Halogen, Nitro, Rhodano, $OR^3$, $S(O)_nR^4$, $N(R^3)_2$, $NR^3OR^3$, $COR^3$, $OCOR^3$, $SCOR^4$, $NR^3COR^3$, $NR^3SO_2R^4$, $CO_2R^3$, $COSR^4$, $CON(R^3)_2$ und $(C_1-C_4)$-Alkoxy-$(C_2-C_6)$-alkoxycarbonyl substituiert sind, und wobei Heterocyclyl n Oxogruppen trägt,

$R^2$ bedeutet $(C_1-C_6)$-Alkyl, $(C_1-C_6)$-Halogenalkyl, $(C_2-C_6)$-Alkenyl, $(C_2-C_6)$-Halogenalkenyl, $(C_2-C_6)$-Alkinyl, $(C_2-C_6)$-Halogenalkinyl, $(C_3-C_6)$-Cycloalkyl, $(C_3-C_6)$-Cycloalkenyl, $(C_3-C_6)$-Halogencycloalkyl, $(C_1-C_6)$-Alkyl-O-$(C_1-C_6)$-alkyl, $(C_3-C_6)$-Cycloalkyl-$(C_1-C_6)$-alkyl, Phenyl, Phenyl-$(C_1-C_6)$-alkyl, Heteroaryl, $(C_1-C_6)$-Alkyl-Heteroaryl, Heterocyclyl, $(C_1-C_6)$-Alkyl-Heterocyclyl, $(C_1-C_6)$-Alkyl-O-Heteroaryl, $(C_1-C_6)$-Alkyl-O-Heterocyclyl, $(C_1-C_6)$-Alkyl-$NR^3$-Heteroaryl oder $(C_1-C_6)$-Alkyl-$NR^3$-Heterocyclyl, wobei die 21 letztgenannten Reste jeweils durch s Reste aus der Gruppe bestehend aus Cyano, Halogen, Nitro, Rhodano, $OR^3$, $S(O)_nR^4$, $N(R^3)_2$, $NR^3OR^3$, $COR^3$, $OCOR^3$, $SCOR^4$, $NR^3COR^3$, $NR^3SO_2R^4$, $CO_2R^3$, $COSR^4$, $CON(R^3)_2$ und $(C_1-C_4)$-Alkoxy-$(C_2-C_6)$-alkoxycarbonyl substituiert sind, und wobei Heterocyclyl n Oxogruppen trägt,

$R^3$ bedeutet Wasserstoff, $(C_1-C_6)$-Alkyl, $(C_2-C_6)$-Alkenyl, $(C_2-C_6)$-Alkinyl, $(C_3-C_6)$-Cycloalkyl, $(C_3-C_6)$-Cycloalkyl-$(C_1-C_6)$-alkyl oder Phenyl,

$R^4$ bedeutet $(C_1-C_6)$-Alkyl, $(C_2-C_6)$-Alkenyl, $(C_2-C_6)$-Alkinyl, $(C_3-C_6)$-Cycloalkyl, $(C_3-C_6)$-Cycloalkyl-$(C_1-C_6)$-alkyl oder Phenyl,

$R^5$ bedeutet $(C_1-C_4)$-Alkyl,

n bedeutet 0, 1 oder 2,

s bedeutet 0, 1, 2 oder 3.

**2.** 5-Phenylsubstituierte N-(Tetrazol-5-yl)- und N-(Triazol-5-yl)arylcarbonsäure-amide nach Anspruch 1, worin

A bedeutet N oder CY,

B bedeutet N oder CH,

X bedeutet Nitro, Halogen, Cyano, Rhodano, $(C_1-C_6)$-Alkyl, Halogen-$(C_1-C_6)$-alkyl, $(C_2-C_6)$-Alkenyl, Halogen-$(C_2-C_6)$-alkenyl, $(C_2-C_6)$-Alkinyl, Halogen-$(C_3-C_6)$-alkinyl, $(C_3-C_6)$-Cycloalkyl, Halogen-$(C_3-C_6)$-cycloalkyl, $(C_1-C_6)$-Alkyl-O-$(C_1-C_6)$-alkyl, $(C_3-C_6)$-Cycloalkyl-$(C_1-C_6)$-alkyl, Halogen-$(C_3-C_6)$-cycloalkyl-$(C_1-C_6)$-alkyl, $COR^1$, $OR^1$, $OCOR^1$, $OSO_2R^2$, $S(O)_nR^2$, $SO_2OR^1$, $SO_2N(R^1)_2$, $NR^1SO_2R^2$, $NR^1COR^1$, $(C_1-C_6)$-Alkyl-$S(O)_nR^2$, $(C_1-C_6)$-Alkyl-$OR^1$, $(C_1-C_6)$-Alkyl-$OCOR^1$, $(C_1-C_6)$-Alkyl-$OSO_2R^2$, $(C_1-C_6)$-Alkyl-$CO_2R^1$, $(C_1-C_6)$-Alkyl-$SO_2OR^1$, $(C_1-C_6)$-Alkyl-$CON(R^1)_2$, $(C_1-C_6)$-Alkyl-$SO_2N(R^1)_2$, $(C_1-C_6)$-Alkyl-$NR^1COR^1$ oder $(C_1-C_6)$-Alkyl-$NR^1SO_2R^2$, $(C_1-C_6)$-Alkyl-Heteroaryl, $(C_1-C_6)$-Alkyl-Heterocyclyl, wobei die beiden letztgenannten Reste jeweils durch s Reste aus der Gruppe bestehend aus Halogen, $(C_1-C_6)$-Alkyl, Halogen-$(C_1-C_6)$-alkyl, $S(O)_n$-$(C_1-C_6)$-Alkyl, $(C_1-C_6)$-Alkoxy und Halogen-$(C_1-C_6)$-alkoxy substituiert sind, und wobei Heterocyclyl n Oxogruppen trägt,

Y bedeutet Wasserstoff, Nitro, Halogen, Cyano, Rhodano, $(C_1-C_6)$-Alkyl, Halogen-$(C_1-C_6)$-alkyl, $(C_2-C_6)$-Alkenyl, Halogen-$(C_2-C_6)$-alkenyl, $(C_2-C_6)$-Alkinyl, Halogen-$(C_3-C_6)$-alkinyl, $(C_3-C_6)$-Cycloalkyl, $(C_3-C_6)$-Cycloalkenyl, Halogen-$(C_3-C_6)$-cycloalkyl, $(C_3-C_6)$-Cycloalkyl-$(C_1-C_6)$-alkyl, Halogen-$(C_3-C_6)$-cycloalkyl-$(C_1-C_6)$-alkyl, $COR^1$, $OR^1$, $COOR^1$, $OSO_2R^2$, $S(O)_nR^2$, $SO_2OR^1$, $SO_2$ $N(R^1)_2$, $N(R^1)_2$, $NR^1SO_2R^2$, $NR^1COR^1$, $(C_1-C_6)$-Alkyl-$S(O)_nR^2$, $(C_1-C_6)$-Alkyl-$OR^1$, $(C_1-C_6)$-Alkyl-$OCOR^1$, $(C_1-C_6)$-Alkyl-$OSO_2R^2$, $(C_1-C_6)$-Alkyl-$CO_2R^1$, $(C_1-C_6)$-Alkyl-$SO_2OR^1$, $(C_1-C_6)$-Alkyl-$CON(R^1)_2$, $(C_1-C_6)$-Alkyl-$SO_2N(R^1)_2$, $(C_1-C_6)$-Alkyl-$NR^1COR^1$, $(C_1-C_6)$-Alkyl-$NR^1SO_2R^2$, $(C_1-C_6)$-Alkyl-Phenyl, $(C_1-C_6)$-Alkyl-Heteroaryl, $(C_1-C_6)$-Alkyl-Heterocyclyl, Phenyl, Heteroaryl oder Heterocyclyl, wobei die sechs letztgenannten Reste jeweils durch s Reste aus der Gruppe bestehend Halogen, Nitro, Cyano, $(C_1-C_6)$-Alkyl, Halogen-$(C_1-C_6)$-alkyl, $(C_3-C_6)$-Cycloalkyl, $S(O)_n$-$(C_1-C_6)$-Alkyl, $(C_1-C_6)$-Alkoxy, Halogen-$(C_1-C_6)$-alkoxy, $(C_1-C_6)$-Alkoxy-$(C_1-C_4)$-alkyl und Cyanomethyl substituiert sind, und wobei Heterocyclyl n Oxogruppen trägt,

Z bedeutet Halogen, Cyano, Rhodano, Halogen-$(C_1-C_6)$-alkyl, $(C_2-C_6)$-Alkenyl, Halogen-$(C_2-C_6)$-alkenyl, $(C_2-C_6)$-Alkinyl, Halogen-$(C_3-C_6)$-alkinyl, $(C_3-C_6)$-Cycloalkyl, Halogen-$(C_3-C_6)$-cycloalkyl, $(C_3-C_6)$-Cycloalkyl-$(C_1-C_6)$-alkyl, Halogen-$(C_3-C_6)$-cycloalkyl-$(C_1-C_6)$-alkyl, $COR^1$, $COOR^1$, $C(O)N(R^1)_2$, $C(O)NR^1OR^1$, $OSO_2R^2$, $S(O)_nR^2$, $SO_2OR^1$, $SO_2N(R^1)_2$, $NR^1SO_2R^2$, $NR^1COR^1$, $(C_1-C_6)$-Alkyl-$S(O)_nR^2$, $(C_1-C_6)$-Alkyl-$OR^1$, $(C_1-C_6)$-Alkyl-$OCOR^1$, $(C_1-C_6)$-Alkyl-$OSO_2R^2$, $(C_1-C_6)$-Alkyl-$CO_2R^1$, $(C_1-C_6)$-Alkyl-$SO_2OR^1$, $(C_1-C_6)$-Alkyl-$CON(R^1)_2$, $(C_1-C_6)$-Alkyl-$SO_2N(R^1)_2$, $(C_1-C_6)$-Alkyl-$NR^1COR^1$, $(C_1-C_6)$-Alkyl-$NR^1SO_2R^2$, 1,2,4-Triazol-1-yl, oder

Z kann auch Wasserstoff, $(C_1-C_6)$-Alkyl oder $(C_1-C_6)$-Alkoxy bedeuten, falls Y für den Rest $S(O)_nR^2$ steht,

W bedeutet $(C_1-C_6)$-Alkyl, Halogen-$(C_1-C_6)$-alkyl, $(C_1-C_6)$-Alkoxy, $(C_1-C_6)$-Halogenalkoxy, $S(O)_n$-$(C_1-C_6)$-alkyl, $S(O)_n$-$(C_1-C_6)$-halogenalkyl, $(C_1-C_6)$-Alkoxy-$(C_1-C_4)$-alkyl, Halogen, Nitro oder Cyano,

R bedeutet $(C_1-C_8)$-Alkyl, Halogen-$(C_1-C_8)$-alkyl, $(C_2-C_8)$-Alkenyl, Halogen-$(C_2-C_8)$-alkenyl, $(C_2-C_8)$-Alkinyl, Halogen-$(C_2-C_8)$-alkinyl, wobei diese sechs vorstehend genannten Reste jeweils durch s Reste aus der Gruppe bestehend aus Nitro, Cyano, $SiR^5_3$, $P(OR^5)_3$, $S(O)_n$-$(C_1-C_6)$-Alkyl, $(C_1-C_6)$-Alkoxy, Halogen-$(C_1-C_6)$-alkoxy, $N(R^3)_2$, $COR^3$, $COOR^3$, $OCOR^3$, $NR^3COR^3$, $NR^3SO_2R^4$, $(C_3-C_6)$-Cycloalkyl, Heteroaryl, Heterocyclyl, Phenyl, Q-Heteroaryl, Q-Heterocyclyl, Q-Phenyl und Q-Benzyl substituiert sind, wobei die sieben letztgenannten Reste jeweils durch s Reste aus der Gruppe bestehend aus Methyl, Ethyl, Methoxy, Trifluormethyl, Cyano und Halogen substituiert sind, und wobei Heterocyclyl n Oxogruppen trägt, oder

R bedeutet jeweils durch s Reste aus der Gruppe bestehend aus Halogen, Nitro, Cyano, $(C_1-C_6)$-Alkyl, Halogen-$(C_1-C_6)$-alkyl, $(C_3-C_6)$-Cycloalkyl, $S(O)_n$-$(C_1-C_6)$-Alkyl, $(C_1-C_6)$-Alkoxy, Halogen-$(C_1-C_6)$-alkoxy und $(C_1-C_6)$-Alkoxy-$(C_1-C_4)$-alkyl substituiertes $(C_3-C_7)$-Cycloalkyl, Heteroaryl, Heterocyclyl oder Phenyl,

Q bedeutet O, S, oder $NR^3$,

$R^1$ bedeutet Wasserstoff, $(C_1-C_6)$-Alkyl, $(C_2-C_6)$-Alkenyl, $(C_2-C_6)$-Alkinyl, $(C_3-C_6)$-Cycloalkyl, $(C_3-C_6)$-Cycloalkyl-$(C_1-C_6)$-alkyl, $(C_1-C_6)$-Alkyl-O-$(C_1-C_6)$-alkyl, Phenyl, Phenyl-$(C_1-C_6)$-alkyl, Heteroaryl, $(C_1-C_6)$-Alkyl-Heteroaryl, Heterocyclyl, $(C_1-C_6)$-Alkyl-Heterocyclyl, $(C_1-C_6)$-Alkyl-O-Heteroaryl, $(C_1-C_6)$-Alkyl-O-Heterocyclyl, $(C_1-C_6)$-Alkyl-$NR^3$-Heteroaryl oder $(C_1-C_6)$-Alkyl-$NR^3$-Heterocyclyl, wobei die sechzehn letztgenannten Reste jeweils durch s Reste aus der Gruppe bestehend aus Cyano, Halogen, Nitro, $OR^3$, $S(O)_nR^4$, $N(R^3)_2$, $NR^3OR^3$, $COR^3$, $OCOR^3$, $NR^3COR^3$, $NR^3SO_2R^4$, $CO_2R^3$, $CON(R^3)_2$ und $(C_1-C_4)$-Alkoxy-$(C_2-C_6)$-alkoxycarbonyl substituiert sind, und wobei Heterocyclyl n Oxogruppen trägt,

$R^2$ bedeutet $(C_1-C_6)$-Alkyl, $(C_2-C_6)$-Alkenyl, $(C_2-C_6)$-Alkinyl, $(C_3-C_6)$-Cycloalkyl, $(C_3-C_6)$-Cycloalkyl-$(C_1-C_6)$-alkyl, $(C_1-C_6)$-Alkyl-O-$(C_1-C_6)$-alkyl, Phenyl, Phenyl-$(C_1-C_6)$-alkyl, Heteroaryl, $(C_1-C_6)$-Alkyl-Heteroaryl, Heterocyclyl, $(C_1-C_6)$-Alkyl-Heterocyclyl, $(C_1-C_6)$-Alkyl-O-Heteroaryl, $(C_1-C_6)$-Alkyl-O-Heterocyclyl, $(C_1-C_6)$-Alkyl-$NR^3$-Heteroaryl oder $(C_1-C_6)$-Alkyl-$NR^3$-Heterocyclyl, wobei diese sechzehn letztgenannten Reste jeweils durch s Reste aus der Gruppe bestehend aus Cyano, Halogen, Nitro, $OR^3$, $S(O)_nR^4$, $N(R^3)_2$, $NR^3OR^3$, $NR^3SO_2R^4$, $COR^3$, $OCOR^3$, $NR^3COR^3$, $CO_2R^3$, $CON(R^3)_2$ und $(C_1-C_4)$-Alkoxy-$(C_2-C_6)$-alkoxycarbonyl substituiert sind, und wobei Heterocyclyl n Oxogruppen trägt,

$R^3$ bedeutet Wasserstoff, $(C_1-C_6)$-Alkyl, $(C_2-C_6)$-Alkenyl, $(C_2-C_6)$-Alkinyl, $(C_3-C_6)$-Cycloalkyl oder $(C_3-C_6)$-Cy-

cloalkyl-($C_1$-$C_6$)-alkyl,
$R^4$ bedeutet ($C_1$-$C_6$)-Alkyl, ($C_2$-$C_6$)-Alkenyl oder ($C_2$-$C_6$)-Alkinyl,
$R^5$ bedeutet Methyl oder Ethyl,
n bedeutet 0, 1 oder 2,
s bedeutet 0, 1, 2 oder 3.

**3.** 5-Phenylsubstituierte N-(Tetrazol-5-yl)- und N-(Triazol-5-yl)arylcarbonsäure-amide nach Anspruch 1 oder 2, worin

A bedeutet N oder CY,
B bedeutet N oder CH,
X bedeutet Nitro, Halogen, Cyano, ($C_1$-$C_6$)-Alkyl, Halogen-($C_1$-$C_6$)-alkyl, ($C_3$-$C_6$)-Cycloalkyl, $OR^1$, $S(O)_nR^2$, ($C_1$-$C_6$)-Alkyl-$S(O)_nR^2$, ($C_1$-$C_6$)-Alkyl-$OR^1$, ($C_1$-$C_6$)-Alkyl-$CON(R^1)_2$, ($C_1$-$C_6$)-Alkyl-$SO_2N(R^1)_2$, ($C_1$-$C_6$)-Alkyl-$NR^1COR^1$, ($C_1$-$C_6$)-Alkyl-$NR^1SO_2R^2$, ($C_1$-$C_6$)-Alkyl-Heteroaryl, ($C_1$-$C_6$)-Alkyl-Heterocyclyl, wobei die beiden letzt genannten Reste jeweils durch s Reste Halogen, ($C_1$-$C_6$)-Alkyl, Halogen-($C_1$-$C_6$)-alkyl, $S(O)_n$-($C_1$-$C_6$)-Alkyl, ($C_1$-$C_6$)-Alkoxy und Halogen-($C_1$-$C_6$)-alkoxy substituiert sind, und wobei Heterocyclyl n Oxogruppen trägt,
Y Wasserstoff, Nitro, Halogen, Cyano, ($C_1$-$C_6$)-Alkyl, ($C_1$-$C_6$)-Halogenalkyl, $OR^1$, $S(O)_nR^2$, $SO_2N(R^1)_2$, $N(R^1)_2$, $NR^1SO_2R^2$, $NR^1COR^1$, ($C_1$-$C_6$)-Alkyl-$S(O)_nR^2$, ($C_1$-$C_6$)-Alkyl-$OR^1$, ($C_1$-$C_6$)-Alkyl-$CON(R^1)_2$, ($C_1$-$C_6$)-Alkyl-$SO_2N(R^1)_2$, ($C_1$-$C_6$)-Alkyl-$NR^1COR^1$, ($C_1$-$C_6$)-Alkyl-$NR^1SO_2R^2$, ($C_1$-$C_6$)-Alkyl-Phenyl, ($C_1$-$C_6$)-Alkyl-Heteroaryl, ($C_1$-$C_6$)-Alkyl-Heterocyclyl, Phenyl, Heteroaryl oder Heterocyclyl, wobei die sechs letztgenannten Reste jeweils durch s Reste aus der Gruppe bestehend Halogen, Nitro, Cyano, ($C_1$-$C_6$)-Alkyl, Halogen-($C_1$-$C_6$)-alkyl, ($C_3$-$C_6$)-Cycloalkyl, $S(O)_n$-($C_1$-$C_6$)-Alkyl, ($C_1$-$C_6$)-Alkoxy, Halogen-($C_1$-$C_6$)-alkoxy, ($C_1$-$C_6$)-Alkoxy-($C_1$-$C_4$)-alkyl und Cyanomethyl substituiert sind, und wobei Heterocyclyl n Oxogruppen trägt,
Z bedeutet Halogen, Cyano, Halogen-($C_1$-$C_6$)-alkyl, ($C_3$-$C_6$)-Cycloalkyl, $S(O)_nR^2$, 1,2,4-Triazol-1-yl, oder
Z kann auch Wasserstoff, Methyl, Methoxy oder Ethoxy bedeuten, falls Y für den Rest $S(O)_nR^2$ steht,
W bedeutet Methyl, Ethyl, Methoxymethyl, Methoxy, Fluor, Chlor oder $S(O)_nCH_3$,
R bedeutet ($C_1$-$C_8$)-Alkyl, Halogen-($C_1$-$C_8$)-alkyl, ($C_2$-$C_8$)-Alkenyl, Halogen-($C_2$-$C_8$)-alkenyl, ($C_2$-$C_8$)-Alkinyl, Halogen-($C_2$-$C_8$)-alkinyl, wobei diese sechs vorstehend genannten Reste jeweils durch s Reste aus der Gruppe bestehend aus Cyano, $S(O)_n$-($C_1$-$C_6$)-Alkyl, ($C_1$-$C_6$)-Alkoxy, Halogen-($C_1$-$C_6$)-alkoxy, $COR^3$, $COOR^3$, $OCOR^3$, $NR^3COR^3$, $NR^3SO_2R^4$, ($C_3$-$C_6$)-Cycloalkyl, Heteroaryl, Heterocyclyl und Phenyl substituiert sind, wobei die drei letztgenannten Reste jeweils durch s Reste aus der Gruppe bestehend aus Methyl, Ethyl, Methoxy, Trifluormethyl, Cyano und Halogen substituiert sind, und wobei Heterocyclyl 0 bis 2 Oxogruppen trägt, oder
R bedeutet durch s Reste aus der Gruppe bestehend aus Halogen, Nitro, Cyano, ($C_1$-$C_6$)-Alkyl, Halogen-($C_1$-$C_6$)-alkyl, ($C_3$-$C_6$)-Cycloalkyl, $S(O)_n$-($C_1$-$C_6$)-Alkyl, ($C_1$-$C_6$)-Alkoxy, Halogen-($C_1$-$C_6$)-alkoxy, ($C_1$-$C_6$)-Alkoxy-($C_1$-$C_4$)-alkyl substituiertes Phenyl,
$R^1$ bedeutet Wasserstoff, ($C_1$-$C_6$)-Alkyl, ($C_2$-$C_6$)-Alkenyl, ($C_2$-$C_6$)-Alkinyl, ($C_3$-$C_6$)-Cycloalkyl, ($C_3$-$C_6$)-Cycloalkyl-($C_1$-$C_6$)-alkyl, ($C_1$-$C_6$)-Alkyl-O-($C_1$-$C_6$)-alkyl, Phenyl, Phenyl-($C_1$-$C_6$)-alkyl, Heteroaryl, ($C_1$-$C_6$)-Alkyl-Heteroaryl, Heterocyclyl, ($C_1$-$C_6$)-Alkyl-Heterocyclyl, ($C_1$-$C_6$)-Alkyl-O-Heteroaryl, ($C_1$-$C_6$)-Alkyl-O-Heterocyclyl, ($C_1$-$C_6$)-Alkyl-$NR^3$-Heteroaryl oder ($C_1$-$C_6$)-Alkyl-$NR^3$-Heterocyclyl, wobei die sechzehn letztgenannten Reste jeweils durch s Reste aus der Gruppe bestehend aus Cyano, Halogen, Nitro, $OR^3$, $S(O)_nR^4$, $N(R^3)_2$, $NR^3OR^3$, $COR^3$, $OCOR^3$, $NR^3COR^3$, $NR^3SO_2R^4$, $CO_2R^3$, $CON(R^3)_2$ und ($C_1$-$C_4$)-Alkoxy-($C_2$-$C_6$)-alkoxycarbonyl substituiert sind, und wobei Heterocyclyl n Oxogruppen trägt,
$R^2$ bedeutet jeweils durch s Reste aus der Gruppe bestehend aus Halogen und $OR^3$ substituiertes ($C_1$-$C_6$)-Alkyl, ($C_3$-$C_6$)-Cycloalkyl oder ($C_3$-$C_6$)-Cycloalkyl-($C_1$-$C_6$)-alkyl,
$R^3$ bedeutet Wasserstoff oder ($C_1$-$C_6$)-Alkyl,
$R^4$ bedeutet ($C_1$-$C_6$)-Alkyl,
$R^5$ bedeutet Methyl oder Ethyl,
n bedeutet 0, 1 oder 2,
s bedeutet 0, 1, 2 oder 3.

**4.** Herbizide Mittel, **gekennzeichnet durch** einen herbizid wirksamen Gehalt an mindestens einer Verbindung der Formel (I) gemäß einem der Ansprüche 1 bis 3.

**5.** Herbizide Mittel nach Anspruch 4 in Mischung mit Formulierungshilfsmitteln.

**6.** Herbizide Mittel nach Anspruch 4 oder 5 enthaltend mindestens einen weiteren pestizid wirksamen Stoff aus der Gruppe Insektizide, Akarizide, Herbizide, Fungizide, Safener und Wachstumsregulatoren.

**7.** Herbizide Mittel nach Anspruch 6 enthaltend einen Safener.

**8.** Herbizide Mittel nach Anspruch 7 enthaltend cyprosulfamid, cloquintocet-mexyl, mefenpyr-diethyl oder isoxadifen-ethyl.

**9.** Herbizide Mittel nach einem der Ansprüche 6 bis 8 enthaltend ein weiteres Herbizid.

**10.** Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 3 oder eines herbiziden Mittels nach einem der Ansprüche 4 bis 9 auf die Pflanzen oder auf den Ort des unerwünschten Pflanzenwachstums appliziert.

**11.** Verwendung von Verbindungen der Formel (I) gemäß einem der Ansprüche 1 bis 3 oder von herbiziden Mitteln nach einem der Ansprüche 4 bis 9 zur Bekämpfung unerwünschter Pflanzen.

**12.** Verwendung nach Anspruch 11, **dadurch gekennzeichnet, daß** die Verbindungen der Formel (I) zur Bekämpfung unerwünschter Pflanzen in Kulturen von Nutzpflanzen eingesetzt werden.

**13.** Verwendung nach Anspruch 12, **dadurch gekennzeichnet, daß** die Nutzpflanzen transgene Nutzpflanzen sind.

## Claims

**1.** 5-Phenyl-substituted N-(tetrazol-5-yl)- and N-(triazol-5-)arylcarboxamides of the formula (I) or salts thereof

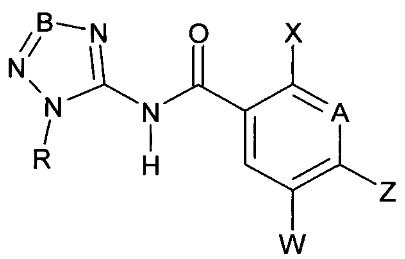

(I),

in which

A is N or CY,
B is N or CH,
X is nitro, halogen, cyano, formyl, thiocyanato, $(C_1-C_6)$-alkyl, halo-$(C_1-C_6)$-alkyl, $(C_2-C_6)$ -alkenyl, halo-$(C_2-C_6)$-alkenyl, $(C_2-C_6)$ -alkynyl, halo- $(C_3-C_6)$ -alkynyl, $(C_3-C_6)$-cycloalkyl, halo- $(C_3-C_6)$ -cycloalkyl, $(C_3-C_6)$-cycloalkyl-$(C_1-C_6)$-alkyl, halo-$(C_3-C_6)$-cycloalkyl-$(C_1-C_6)$-alkyl, $COR^1$, $COOR^1$, $OCOOR^1$, $NR^1COOR^1$, $C(O)N(R^1)_2$, $NR^1C(O)N(R^1)_2$, $OC(O)N(R^1)_2$, $C(O)NR^1OR^1$, $OR^1$, $OCOR^1$, $OSO_2R^2$, $S(O)_nR^2$, $SO_2OR^1$, $SO_2N(R^1)_2$, $NR^1SO_2R^2$, $NR^1COR^1$, $(C_1-C_6)$-alkyl-S (0) $_nR^2$, $(C_1-C_6)$ -alkyl-$OR^1$, $(C_1-C_6)$ -alkyl-$OCOR^1$, $(C_1-C_6)$-alkyl-$OSO_2R^2$, $(C_1-C_6)$ -alkyl-$CO_2R^1$, $(C_1-C_6)$ -alkyl-$SO_2OR^1$, $(C_1-C_6)$-alkyl-$CON(R^1)_2$, $(C_1-C_6)$-alkyl-$SO_2N(R^1)2$, $(C_1-C_6)$-alkyl-$NR^1COR^1$, $(C_1-C_6)$-alkyl-$NR^1SO_2R^2$, $NR_1R_2$, $P(O)(OR^5)_2$, $CH_2P(O)(OR^5)_2$, $(C_1-C_6)$-alkylheteroaryl, $(C_1-C_6)$-alkylheterocyclyl, where the two last-mentioned radicals are each substituted by s radicals from the group consisting of halogen, $(C_1-C_6)$ -alkyl, halo-$(C_1-C_6)$-alkyl, $S(O)_n$-$(C_1-C_6)$-alkyl, $(C_1-C_6)$ -alkoxy and halo-$(C_1-C_6)$-alkoxy, and where heterocyclyl carries n oxo groups,
Y is hydrogen, nitro, halogen, cyano, thiocyanato, $(C_1-C_6)$-alkyl, halo-$(C_1-C_6)$-alkyl, $(C_2-C_6)$ -alkenyl, halo-$(C_2-C_6)$-alkenyl, $(C_2-C_6)$-alkynyl, halo- $(C_2-C_6)$-alkynyl, $(C_3-C_6)$-cycloalkyl, $(C_3-C_6)$-cycloalkenyl, halo-$(C_3-C_6)$-cycloalkyl, $(C_3-C_6)$-cycloalkyl-$(C_1-C_6)$-alkyl, halo-$(C_3-C_6)$-cycloalkyl-$(C_1-C_6)$-alkyl, $COR^1$, $COOR^1$, $OCOOR^1$, $NR^1COOR^1$, $C(O)N(R^1)_2$, $NR^1C(O)N(R^1)_2$, $OC(O)N(R^1)_2$, $CO(NOR^1)R^1$, $NR^1SO_2R^2$, $NR^1COR^1$, $OR^1$, $OSO_2R^2$, $S(O)_nR^2$, $SO_2OR^1$, $SO_2N(R^1)_2$, $(C_1-C_6)$-alkyl-$S(O)_nR^2$, $(C_1-C_6)$-alkyl-$OR^1$, $(C_1-C_6)$ -alkyl-$OCOR^1$, $(C_1-C_6)$ -alkyl-OSO2$R^2$, $(C_1-C_6)$ -alkyl-$CO_2R^1$, $(C_1-C_6)$ -alkyl-CN, $(C_1-C_6)$ -alkyl-$SO_2OR^1$, $(C_1-C_6)$ -alkyl-$CON(R^1)_2$, $(C_1-C_6)$-alkyl-$SO_2N(R^1)_2$, $(C_1-C_6)$-alkyl-$NR^1COR^1$, $(C_1-C_6)$-alkyl-$NR^1SO_2R^2$, $N(R^1)_2$, $P(O)(OR^5)_2$, $CH_2P(O)(OR^5)_2$, $(C_1-C_6)$-alkylphenyl, $(C_1-C_6)$-alkylheteroaryl, $(C_1-C_6)$-alkylheterocyclyl, phenyl, heteroaryl or heterocyclyl, where the 6 last-mentioned radicals are each substituted by s radicals from the group consisting of halogen, nitro, cyano, $(C_1-C_6)$-alkyl, halo-$(C_1-C_6)$-alkyl, $(C_3-C_6)$-cycloalkyl, $S(O)_n$-$(C_1-C_6)$-alkyl, $(C_1-C_6)$-alkoxy, halo- $(C_1-C_6)$ -alkoxy, $(C_1-C_6)$-alkoxy-$(C_1-C_4)$-alkyl and cyanomethyl, and where heterocyclyl carries n oxo groups,

Z is halogen, cyano, thiocyanato, halo-($C_1$-$C_6$)-alkyl, ($C_2$-$C_6$)-alkenyl, halo- ($C_2$-$C_6$) -alkenyl, ($C_2$-$C_6$)-alkynyl, halo-($C_2$-$C_6$)-alkynyl, ($C_3$-$C_6$)-cycloalkyl, halo-($C_3$-$C_6$)-cycloalkyl, ($C_3$-$C_6$)-cycloalkyl-($C_1$-$C_6$)-alkyl, halo-($C_3$-$C_6$)-cycloalkyl-($C_1$-$C_6$)-alkyl, $COR^1$, $COOR^1$, $OCOOR^1$, $NR^1COOR^1$, $C(O)N(R^1)_2$, $NR^1C(O)N(R^1)_2$, $OC(O)N(R^1)_2$, $C(O)NR^1OR^1$, $OSO_2R^2$, $S(O)_nR^2$, $SO_2OR^1$, $SO_2N(R^1)_2$, $NR^1SO_2R^2$, $NR^1COR^1$, ($C_1$-$C_6$)-alkyl-$S(O)_nR^2$, ($C_1$-$C_6$)-alkyl-$OR^1$, ($C_1$-$C_6$)-alkyl-$OCOR^1$, ($C_1$-$C_6$) -alkyl-$OSO_2R^2$, ($C_1$-$C_6$) -alkyl-$CO_2R^1$, ($C_1$-$C_6$)-alkyl-$SO_2OR^1$, ($C_1$-$C_6$)-alkyl-$CON(R^1)_2$, ( $C_1$-$C_6$)-alkyl-$SO_2N(R^1)_2$, ($C_1$-$C_6$)-alkyl-$NR^1COR^1$, ($C_1$-$C_6$) -alkyl-$NR^1SO_2R^2$, $N(R^1)_2$, $P(O)(OR^5)_2$, heteroaryl, heterocyclyl or phenyl, where the three last-mentioned radicals are each substituted by s radicals from the group consisting of halogen, nitro, cyano, ($C_1$-$C_6$)-alkyl, halo- ($C_1$-$C_6$) -alkyl, ($C_3$-$C_6$) -cycloalkyl, S (0) $_n$-($C_1$-$C_6$)-alkyl, ($C_1$-$C_6$) -alkoxy and halo- ($C_1$-$C_6$) -alkoxy, and where heterocyclyl carries n oxo groups, or

Z may also be hydrogen, ($C_1$-$C_6$) -alkyl or ($C_1$-$C_6$) -alkoxy if Y is the radical $S(O)_nR^2$,

W is ($C_1$-$C_6$) -alkyl, halo- ($C_1$-$C_6$) -alkyl, ($C_2$-$C_6$)-alkenyl, halo- ($C_2$-$C_6$) -alkenyl, ($C_2$-$C_6$) -alkynyl, halo-($C_2$-$C_6$)-alkynyl, ($C_3$-$C_7$)-cycloalkyl, ($C_3$-$C_7$)-halocycloalkyl, ($C_1$-$C_6$) -alkoxy, ($C_1$-$C_6$)-haloalkoxy, $S(O)_n$-($C_1$-$C_6$)-alkyl, $S(O)_n$-($C_1$-$C_6$) -haloalkyl, ($C_1$-$C_6$)-alkoxy-($C_1$-$C_4$)-alkyl, ($C_1$-$C_6$)-alkoxy-($C_1$-$C_4$)-haloalkyl, halogen, nitro, $NR^3COR^3$ or cyano,

R is ($C_1$-$C_8$)-alkyl, halo-($C_1$-$C_8$)-alkyl, ($C_2$-$C_8$)-alkenyl, halo- ($C_2$-$C_8$) -alkenyl, ($C_2$-$C_8$)-alkynyl, halo-($C_2$-$C_8$)-alkynyl, where these six above-mentioned radicals are each substituted by s radicals from the group consisting of hydroxy, nitro, cyano, $SiR^5_3$, $PO(OR^5)_2$, $S(O)_n$-($C_1$-$C_6$)-alkyl, $S(O)_n$-($C_1$-$C_6$)-haloalkyl, ($C_1$-$C_6$)-alkoxy, halo-($C_1$-$C_6$)-alkoxy, $N(R^3)_2$, $COR^3$, $COOR^3$, $OCOR^3$, $NR^3COR^3$, $NR^3SO_2R^4$, O($C_1$-$C_2$)-alkyl-($C_3$-$C_6$)-cycloalkyl, ($C_3$-$C_6$)-cycloalkyl, heteroaryl, heterocyclyl, phenyl, Q-heteroaryl, Q-heterocyclyl, Q-phenyl and Q-benzyl, where the seven last-mentioned radicals are each substituted by s radicals from the group consisting of methyl, ethyl, methoxy, trifluoromethyl, cyano and halogen, and where heterocyclyl carries n oxo groups, or

R is ($C_3$-$C_7$)-cycloalkyl, heteroaryl, heterocyclyl or phenyl, each of which is substituted by s radicals from the group consisting of halogen, nitro, cyano, ($C_1$-$C_6$)-alkyl, halo-($C_1$-$C_6$)-alkyl, ($C_3$-$C_6$)-cycloalkyl, $S(O)_n$-($C_1$-$C_6$)-alkyl, ($C_1$-$C_6$)-alkoxy, halo-($C_1$-$C_6$)-alkoxy and ($C_1$-$C_6$) -alkoxy- ($C_1$-$C_4$) -alkyl, where heterocyclyl carries n oxo groups,

Q is O, S or $NR^3$,

$R^1$ is hydrogen, ($C_1$-$C_6$) -alkyl, ($C_1$-$C_6$) -haloalkyl, ($C_2$-$C_6$) -alkenyl, ($C_2$-$C_6$) -haloalkenyl, ($C_2$-$C_6$) -alkynyl, ($C_2$-$C_6$) -haloalkynyl, ($C_3$-$C_6$) -cycloalkyl, ($C_3$-$C_6$)-cycloalkenyl, ($C_3$-$C_6$)-halocycloalkyl, ($C_1$-$C_6$)-alkyl-O-($C_1$-$C_6$)-alkyl, ($C_3$-$C_6$)-cycloalkyl-($C_1$-$C_6$)-alkyl, phenyl, phenyl- ($C_1$-$C_6$) -alkyl, heteroaryl, ($C_1$-$C_6$)-alkylheteroaryl, heterocyclyl, ($C_1$-$C_6$)-alkylheterocyclyl, ($C_1$-$C_6$) -alkyl-O-heteroaryl, ($C_1$-$C_6$)-alkyl-O-heterocyclyl, ($C_1$-$C_6$)-alkyl-$NR^3$-heteroaryl or ($C_1$-$C_6$) -alkyl-$NR^3$-heterocyclyl, where the 21 last-mentioned radicals are each substituted by s radicals from the group consisting of cyano, halogen, nitro, thiocyanato, $OR^3$, $S(O)_nR^4$, $N(R^3)_2$, $NR^3OR^3$, $COR^3$, $OCOR^3$, $SCOR^4$, $NR^3COR^3$, $NR^3SO_2R^4$, $CO_2R^3$, $COSR^4$, $CON(R^3)$ and ($C_1$-$C_4$)-alkoxy-($C_2$-$C_6$)-alkoxycarbonyl, and where heterocyclyl carries n oxo groups,

$R^2$ is ($C_1$-$C_6$) -alkyl, ($C_1$-$C_6$) -haloalkyl, ($C_2$-$C_6$)-alkenyl, ($C_2$-$C_6$) -haloalkenyl, ($C_2$-$C_6$) -alkynyl, ($C_2$-$C_6$)-hal.oalkynyl, ($C_3$-$C_6$) -cycloalkyl, ($C_3$-$C_6$) -cycloalkenyl, ($C_3$-$C_6$) -halocycloalkyl, ($C_1$-$C_6$) -alkyl-O- ($C_1$-$C_6$) -alkyl, ($C_3$-$C_6$) -cycloalkyl- ($C_1$-$C_6$) -alkyl, phenyl, phenyl- ($C_1$-$C_6$)-alkyl, heteroaryl, ($C_1$-$C_6$)-alkylheteroaryl, heterocyclyl, ($C_1$-$C_6$) -alkylheterocyclyl, ($C_1$-$C_6$)-alkyl-O-heteroaryl, ($C_1$-$C_6$)-alkyl-O-heterocyclyl, ($C_1$-$C_6$)-alkyl-$NR^3$-heteroaryl or ($C_1$-$C_6$) -alkyl-$NR^3$-heterocyclyl, where the 21 last-mentioned radicals are each substituted by s radicals from the group consisting of cyano, halogen, nitro, thiocyanato, $OR^3$, $S(O)_nR^4$, $N(R^3)_2$, $NR^3OR^3$, $COR^3$, $OCOR^3$, $SCOR^4$, $NR^3COR^3$, $NR^3SO_2R^4$, $CO_2R^3$, $COSR^4$, $CON(R^3)_2$ and ($C_1$-$C_4$)-alkoxy-($C_2$-$C_6$)-alkoxycarbonyl, and where heterocyclyl carries n oxo groups,

$R^3$ is hydrogen, ($C_1$-$C_6$) -alkyl, ($C_2$-$C_6$)-alkenyl, ($C_2$-$C_6$)-alkynyl, ($C_3$-$C_6$)-cycloalkyl, ($C_3$-$C_6$)-cycloalkyl-($C_1$-$C_6$)-alkyl or phenyl,

$R^4$ is ($C_1$-$C_6$)-alkyl, ($C_2$-$C_6$)-alkenyl, ($C_2$-$C_6$)-alkynyl, ($C_3$-$C_6$)-cycloalkyl, ($C_3$-$C_6$)-cycloalkyl-($C_1$-$C_6$)-alkyl or phenyl,

$R^5$ is ($C_1$-$C_4$)-alkyl,

n is 0, 1 or 2;

s is 0, 1, 2 or 3.

**2.** 5-Phenyl-substituted N-(tetrazol-5-yl)-and N-(triazol-5-yl)arylcarboxamides according to Claim 1, in which

A is N or CY,

B is N or CH,

X is nitro, halogen, cyano, thiocyanato, ($C_1$-$C_6$)-alkyl, halo-($C_1$-$C_6$)-alkyl, ($C_2$-$C_6$)-alkenyl, halo-($C_2$-$C_6$)-alkenyl, ($C_2$-$C_6$)-alkynyl, halo-($C_3$-$C_6$)-alkynyl, ($C_3$-$C_6$)-cycloalkyl, halo-($C_3$-$C_6$)-cycloalkyl, ($C_1$-$C_6$)-alkyl-O-($C_1$-$C_6$)-alkyl, ($C_3$-$C_6$)-cycloalkyl-($C_1$-$C_6$)-alkyl, halo-($C_3$-$C_6$)-cycloalkyl-($C_1$-$C_6$)-alkyl, $COR^1$, $OR^1$, $OCOR^1$,

$OSO_2R^2$, $S(O)_nR^2$, $SO_2OR^1$, $SO_2N(R^1)_2$, $NR^1SO_2R^2$, $NR^1COR^1$, ($C_1$-$C_6$)-alkyl-$S(O)_nR^2$, ($C_1$-$C_6$)-alkyl-$OR^1$, ($C_1$-$C_6$)-alkyl-$OCOR^1$, ($C_1$-$C_6$)-alkyl-$OSO_2R^2$, ($C_1$-$C_6$)-alkyl-$CO_2R^1$, ($C_1$-$C_6$)-alkyl-$SO_2OR^1$, ($C_1$-$C_6$)-alkyl-$CON(R^1)_2$, ($C_1$-$C_6$)-alkyl-$SO_2N(R^1)_2$, ($C_1$-$C_6$)-alkyl-$NR^1COR^1$ or ($C_1$-$C_6$)-alkyl-$NR^1SO_2R^2$, ($C_1$-$C_6$)-alkyl-heteroaryl, ($C_1$-$C_6$)-alkyl-heterocyclyl, where the two last-mentioned radicals are each substituted by s radicals from the group consisting of halogen, ($C_1$-$C_6$)-alkyl, halo-($C_1$-$C_6$)-alkyl, $S(O)_n$-($C_1$-$C_6$)-alkyl, ($C_1$-$C_6$)-alkoxy and halo-($C_1$-$C_6$)-alkoxy radicals, and where heterocyclyl carries n oxo groups,

Y is hydrogen, nitro, halogen, cyano, thiocyanato, ($C_1$-$C_6$) -alkyl, halo- ($C_1$-$C_6$) -alkyl, ($C_2$-$C_6$) -alkenyl, halo-($C_2$-$C_6$) -alkenyl, ($C_2$-$C_6$) -alkynyl, halo- ($C_3$-$C_6$) -alkynyl, ($C_3$-$C_6$) -cycloalkyl, ($C_3$-$C_6$) -cycloalkenyl, halo-($C_3$-$C_6$)-cycloalkyl, ($C_3$-$C_6$)-cycloalkyl- ($C_1$-$C_6$) -alkyl, halo- ($C_3$-$C_6$) -cycloalkyl- ($C_1$-$C_6$) -alkyl, $COR^1$, $OR^1$, $COOR^1$, $OSO_2R^2$, $S(O)_nR^2$, $SO_2OR^1$, $SO_2$ $N(R^1)_2$, $N(R^1)_2$, $NR^1SO_2R^2$, $NR^1COR^1$, ($C_1$-$C_6$) -alkyl-S (O) $_nR^2$, ($C_1$-$C_6$) -alkyl-$OR^1$, ($C_1$-$C_6$) -alkyl-$OCOR^1$, ($C_1$-$C_6$) -alkyl-$OSO_2R^2$, ($C_1$-$C_6$) -alkyl-$CO_2R^1$, ($C_1$-$C_6$) -alkyl-$SO_2OR^1$, ($C_1$-$C_6$) -alkyl-$CON(R^1)_2$, ($C_1$-$C_6$) -alkyl-$SO_2N(R^1)_2$, ($C_1$-$C_6$) -alkyl-$NR^1COR^1$, ($C_1C_6$) -alkyl-$NR^1SO_2R^2$, ($C_1$-$C_6$)-alkyl-phenyl, ($C_1$-$C_6$) -alkyl-heteroaryl, ($C_1$-$C_6$) -alkyl-heterocyclyl, phenyl, heteroaryl or heterocyclyl, where the six last-mentioned radicals are each substituted by s radicals from the group consisting of halogen, nitro, cyano, ($C_1$-$C_6$) -alkyl, halo- ($C_1$-$C_6$) -alkyl, ($C_3$-$C_6$)-cycloalkyl, $S(O)_n$-($C_1$-$C_6$)-alkyl, ($C_1$-$C_6$)-alkoxy, halo-($C_1$-$C_6$)-alkoxy, ($C_1$-$C_6$)-alkoxy-($C_1$-$C_4$)-alkyl and cyanomethyl, and where heterocyclyl carries n oxo groups,

Z is halogen, cyano, thiocyanato, halo-($C_1$-$C_6$)-alkyl, ($C_2$-$C_6$)-alkenyl, halo- ($C_2$-$C_6$)-alkenyl, ($C_2$-$C_6$)-alkynyl, halo- ($C_3$-$C_6$)-alkynyl, ($C_3$-$C_6$)-cycloalkyl, halo-($C_3$-$C_6$)-cycloalkyl, ($C_3$-$C_6$)-cycloalkyl-($C_1$-$C_6$)-alkyl, halo-($C_3$-$C_6$)-cycloalkyl-($C_1$-$C_6$)-alkyl, $COR^1$, $COOR^1$, $C(O)N(R^1)_2$, $C(O)NR^1OR^1$, $OSO_2R^2$, $S(O)_nR^2$, $SO_2OR^1$, $SO_2N(R^1)_2$, $NR^1SO_2R^2$, $NR^1COR^1$, ($C_1$-$C_6$)-alkyl-$S(O)_nR^2$, ($C_1$-$C_6$)-alkyl-$OR^1$, ($C_1$-$C_6$)-alkyl-$OCOR^1$, ($C_1$-$C_6$)-alkyl-$OSO_2R^2$, ($C_1$-$C_6$)-alkyl-$CO_2R^1$, ($C_1$-$C_6$)-alkyl-$SO_2OR^1$, ($C_1$-$C_6$)-alkyl-$CON(R^1)_2$, ($C_1$-$C_6$)-alkyl-$SO_2N(R^1)_2$, ($C_1$-$C_6$)-alkyl-$NR^1COR^1$, ($C_1$-$C_6$)-alkyl-$NR^1SO_2R^2$ or 1,2,4-triazol-1-yl, or

Z may also be hydrogen, ($C_1$-$C_6$)-alkyl or ($C_1$-$C_6$)-alkoxy if Y is the radical $S(O)_nR^2$,

W is ($C_1$-$C_6$)-alkyl, halo- ($C_1$-$C_6$)-alkyl, ($C_1$-$C_6$)-alkoxy, ($C_1$-$C_6$)-haloalkoxy, $S(O)_n$-($C_1$-$C_6$)-alkyl, $S(O)_n$-($C_1$-$C_6$)-haloalkyl, ($C_1$-$C_6$)-alkoxy- ($C_1$-$C_4$)-alkyl, halogen, nitro or cyano,

R is ($C_1$-$C_8$)-alkyl, halo- ($C_1$-$C_8$)-alkyl, ($C_2$-$C_8$)-alkenyl, halo- ($C_2$-$C_8$)-alkenyl, ($C_2$-$C_8$)-alkynyl, halo-($C_2$-$C_8$)-alkynyl, where these six above-mentioned radicals are each substituted by s radicals from the group consisting of nitro, cyano, $SiR^5_3$, $P(OR^5)_3$, $S(O)_n$-($C_1$-$C_6$)-alkyl, ($C_1$-$C_8$)-alkoxy, halo-($C_1$-$C_6$)-alkoxy, $N(R^3)_2$, $COR^3$, $COOR^3$, $OCOR^3$, $NR^3COR^3$, $NR^3SO_2R^4$, ($C_3$-$C_6$)-cycloalkyl, heteroaryl, heterocyclyl, phenyl, Q-heteroaryl, Q-heterocyclyl, Q-phenyl and Q-benzyl, where the seven last-mentioned radicals are each substituted by s radicals from the group consisting of methyl, ethyl, methoxy, trifluoromethyl, cyano and halogen, and where heterocyclyl carries n oxo groups, or

R is ($C_3$-$C_7$)-cycloalkyl, heteroaryl, heterocyclyl or phenyl, each of which is substituted by s radicals from the group consisting of halogen, nitro, cyano, ($C_1$-$C_6$)-alkyl, halo-($C_1$-$C_6$)-alkyl, ($C_3$-$C_6$)-cycloalkyl, $S(O)_n$-($C_1$-$C_6$)-alkyl, ($C_1$-$C_6$)-alkoxy, halo- ($C_1$-$C_6$)-alkoxy and ($C_1$-$C_6$)-alkoxy-($C_1$-$C_4$)-alkyl,

Q is 0, S or $NR^3$,

$R^1$ is hydrogen, ($C_1$-$C_6$)-alkyl, ($C_2$-$C_6$)-alkenyl, ($C_2$-$C_6$)-alkynyl, ($C_3$-$C_6$)-cycloalkyl, ($C_3$-$C_6$)-cycloalkyl-($C_1$-$C_6$)-alkyl, ($C_1$-$C_6$)-alkyl-O-($C_1$-$C_6$)-alkyl, phenyl, phenyl-($C_1$-$C_6$)-alkyl, heteroaryl, ($C_1$-$C_6$)-alkylheteroaryl, heterocyclyl, ($C_1$-$C_6$)-alkylheterocyclyl, ($C_1$-$C_6$)-alkyl-O-heteroaryl, ($C_1$-$C_6$)-alkyl-O-heterocyclyl, ($C_1$-$C_6$)-alkyl-$NR^3$-heteroaryl or ($C_1$-$C_6$)-alkyl-$NR^3$-heterocyclyl, where the sixteen last-mentioned radicals are each substituted by s radicals from the group consisting of cyano, halogen, nitro, $OR^3$, $S(O)_nR^4$, $N(R^3)_2$, $NR^3OR^3$, $COR^3$, $OCOR^3$, $NR^3COR^3$, $NR^3SO_2R^4$, $CO_2R^3$, $CON(R^3)_2$ and ($C_1$-$C_4$)-alkoxy-($C_2$-$C_6$)-alkoxycarbonyl, and where heterocyclyl carries n oxo groups,

$R^2$ is ($C_1$-$C_6$)-alkyl, ($C_2$-$C_6$)-alkenyl, ($C_2$-$C_6$)-alkynyl, ($C_3$-$C_6$)-cycloalkyl, ($C_3$-$C_6$)-cycloalkyl- ($C_1$-$C_6$)-alkyl, ($C_1$-$C_6$)-alkyl-O-($C_1$-$C_6$)-alkyl, phenyl, phenyl-($C_1$-$C_6$)-alkyl, heteroaryl, ($C_1$-$C_6$)-alkylheteroaryl, heterocyclyl, ($C_1$-$C_6$)-alkylheterocyclyl, ($C_1$-$C_6$)-alkyl-O-heteroaryl, ($C_1$-$C_6$)-alkyl-O-heterocyclyl, ($C_1$-$C_6$)-alkyl-$NR^3$-heteroaryl or ($C_1$-$C_6$)-alkyl-$NR^3$-heterocyclyl, where these sixteen last-mentioned radicals are each substituted by s radicals from the group consisting of cyano, halogen, nitro, $OR^3$, $S(O)_nR^4$, $N(R^3)_2$, $NR^3OR^3$, $NR^3SO_2R^4$, $COR^3$, $OCOR^3$, $NR^3COR^3$, $CO_2R^3$, $CON(R^3)_2$ and ($C_1$-$C_4$)-alkoxy-($C_2$-$C_6$)-alkoxycarbonyl, and where heterocyclyl carries n oxo groups,

$R^3$ is hydrogen, ($C_1$-$C_6$)-alkyl, ($C_2$-$C_6$)-alkenyl, ($C_2$-$C_6$)-alkynyl, ($C_3$-$C_6$)-cycloalkyl or ($C_3$-$C_6$)-cycloalkyl-($C_1$-$C_6$)-alkyl,

$R^4$ is ($C_1$-$C_6$)-alkyl, ($C_2$-$C_6$)-alkenyl or ($C_2$-$C_6$)-alkynyl,

$R^5$ is methyl or ethyl,

n is 0, 1 or 2;

s is 0, 1, 2 or 3.

**3.** 5-Phenyl-substituted N-(tetrazol-5-yl)- and N-(triazol-5-yl)arylcarboxamides according to Claim 1 or 2, in which

A is N or CY,
B is N or CH,
X is nitro, halogen, cyano, $(C_1-C_6)$-alkyl, halo-$(C_1-C_6)$-alkyl, $(C_3-C_6)$-cycloalkyl, $OR^1$, $S(O)_nR^2$, $(C_1-C_6)$-alkyl-S$(O)_nR^2$, $(C_1-C_6)$-alkyl-$OR^1$, $(C_1-C_6)$-alkyl-$CON(R^1)_2$, $(C_1-C_6)$-alkyl-$SO_2N(R^1)_2$, $(C_1-C_6)$-alkyl-$NR^1COR^1$, $(C_1-C_6)$-alkyl-$NR^1SO_2R^2$, $(C_1-C_6)$-alkylheteroaryl, $(C_1-C_6)$-alkylheterocyclyl, where the two last-mentioned radicals are each substituted by s radicals halogen, $(C_1$-**$C_6$**$)$-alkyl, halo-$(C_1-C_6)$-alkyl, $S(O)_n$-$(C_1-C_6)$-alkyl, $(C_1-C_6)$-alkoxy and halo-$(C_1-C_6)$-alkoxy radicals, and where heterocyclyl carries n oxo groups,
Y is hydrogen, nitro, halogen, cyano, $(C_1-C_6)$-alkyl, $(C_1-C_6)$-haloalkyl, $OR^1$, $S(O)_nR^2$, $SO_2N(R^1)_2$, $N(R^1)_2$, $NR^1SO_2R^2$, $NR^1COR^1$, $(C_1-C_6)$-alkyl-$S(O)_nR^2$, $(C_1-C_6)$-alkyl-$OR^1$, $(C_1-C_6)$-alkyl-$CON(R^1)_2$, $(C_1-C_6)$-alkyl-$SO_2N(R^1)_2$, $(C_1-C_6)$ -alkyl-$NR^1COR^1$, $(C_1-C_6)$ -alkyl-$NR^1SO_2R^2$, $(C_1-C_6)$-alkylphenyl, $(C_1-C_6)$ -alkylheteroaryl, $(C_1-C_6)$-alkylheterocyclyl, phenyl, heteroaryl or heterocyclyl, where the 6 last-mentioned radicals are each substituted by s radicals from the group consisting of halogen, nitro, cyano, $(C_1-C_6)$-alkyl, halo- $(C_1-C_6)$-alkyl, $(C_3-C_6)$-cycloalkyl, $S(O)_n$-$(C_1-C_6)$-alkyl, $(C_1-C_6)$-alkoxy, halo- $(C_1-C_6)$ -alkoxy, $(C_1-C_6)$-alkoxy- $(C_1-C_4)$-alkyl and cyanomethyl, and where heterocyclyl carries n oxo groups,
Z is halogen, cyano, halo- $(C_1-C_6)$-alkyl, $(C_3-C_6)$-cycloalkyl, $S(O)_nR^2$, 1,2,4-triazol-1-yl, or
Z may also be hydrogen, methyl, methoxy or ethoxy if Y is the $S(O)_nR^2$ radical,
W is methyl, ethyl, methoxymethyl, methoxy, fluorine, chlorine or $S(O)_nCH_3$,
R is $(C_1-C_8)$-alkyl, halo- $(C_1-C_8)$-alkyl, $(C_2-C_8)$-alkenyl, halo-$(C_2-C_8)$-alkenyl, $(C_2-C_8)$-alkynyl, halo-$(C_2-C_8)$-alkynyl, where these six above-mentioned radicals are each substituted by s radicals from the group consisting of cyano, $S(O)_n$-$(C_1-C_6)$-alkyl, $(C_1-C_6)$-alkoxy, halo- $(C_1-C_6)$-alkoxy, $COR^3$, $COOR^3$, $OCOR^3$, $NR^3COR^3$, $NR^3SO_2R^4$, $(C_3-C_6)$-cycloalkyl, heteroaryl, heterocyclyl and phenyl, where the three last-mentioned radicals are each substituted by s radicals from the group consisting of methyl, ethyl, methoxy, trifluoromethyl, cyano and halogen, and where heterocyclyl carries 0 to 2 oxo groups, or
R is phenyl which is substituted by s radicals from the group consisting of halogen, nitro, cyano, $(C_1-C_6)$-alkyl, halo-$(C_1-C_6)$-alkyl, $(C_3-C_6)$-cycloalkyl, $S(O)_n$-$(C_1-C_6)$-alkyl, $(C_1-C_6)$-alkoxy, halo- $(C_1-C_6)$-alkoxy and $(C_1-C_6)$-alkoxy-$(C_1-C_4)$-alkyl,
$R^1$ is hydrogen, $(C_1-C_6)$-alkyl, $(C_2-C_6)$-alkenyl, ($C_2$-C6-alkynyl, $(C_3-C_6)$-cycloalkyl, $(C_3-C_6)$-cycloalkyl-$(C_1-C_6)$-alkyl, $(C_1-C_6)$-alkyl-O-$(C_1-C_6)$-alkyl, phenyl, phenyl-$(C_1-C_6)$-alkyl, heteroaryl, $(C_1-C_6)$ -alkylheteroaryl, heterocyclyl, $(C_1-C_6)$-alkylheterocyclyl, $(C_1-C_6)$-alkyl-O-heteroaryl, $(C_1-C_6)$-alkyl-O-heterocyclyl, $(C_1-C_6)$-alkyl-$NR^3$-heteroaryl or $(C_1-C_6)$-alkyl-$NR^3$-heterocyclyl, where the sixteen last-mentioned radicals are each substituted by s radicals from the group consisting of cyano, halogen, nitro, $OR^3$, $S(O)_nR^4$, $N(R^3)_2$, $NR^3OR^3$, $COR^3$, $OCOR^3$, $NR^3COR^3$, $NR^3SO_2R^4$, $CO_2R^3$, $CON(R^3)_2$ and $(C_1-C_4)$-alkoxy-$(C_2-C_6)$-alkoxycarbonyl, and where heterocyclyl carries n oxo groups,
$R^2$ is $(C_1-C_6)$-alkyl, $(C_3-C_6)$-cycloalkyl or $(C_3-C_6)$-cycloalkyl-$(C_1-C_6)$-alkyl, each of which is substituted by s radicals from the group consisting of halogen and $OR^3$,
$R^3$ is hydrogen or $(C_1-C_6)$-alkyl,
$R^4$ is $(C_1-C_6)$-alkyl,
$R^5$ is methyl or ethyl,
n is 0, 1 or 2;
s is 0, 1, 2 or 3.

**4.** Herbicidal compositions **characterized by** a herbicidally effective amount of at least one compound of the formula (I) according to any of Claims 1 to 3.

**5.** Herbicidal compositions according to Claim 4 in a mixture with formulation auxiliaries.

**6.** Herbicidal compositions according to Claim 4 or 5, comprising at least one further pesticidally active substance from the group of insecticides, acaricides, herbicides, fungicides, safeners and growth regulators.

**7.** Herbicidal compositions according to Claim 6, comprising a safener.

**8.** Herbicidal compositions according to Claim 7, comprising cyprosulfamide, cloquintocet-mexyl, mefenpyr-diethyl or isoxadifen-ethyl.

**9.** Herbicidal compositions according to any of Claims 6 to 8, comprising a further herbicide.

**10.** Method for controlling unwanted plants, **characterized in that** an effective amount of at least one compound of the formula (I) according to any of Claims 1 to 3 or of a herbicidal composition according to any of Claims 4 to 9 is applied to the plants or the site of the unwanted vegetation.

**11.** Use of compounds of the formula (I) according to any of Claims 1 to 3 or of herbicidal compositions according to any of Claims 4 to 9 for controlling unwanted plants.

**12.** Use according to Claim 11, **characterized in that** the compounds of the formula (I) are used for controlling unwanted plants in crops of useful plants.

**13.** Use according to Claim 12, **characterized in that** the useful plants are transgenic useful plants.

**Revendications**

**1.** Amides 5-phénylsubstitués de l'acide N-(tétrazol-5-yl)arylcarboxylique et de l'acide N-(triazol-5-yl)arylcarboxylique de formule (I) ou leurs sels

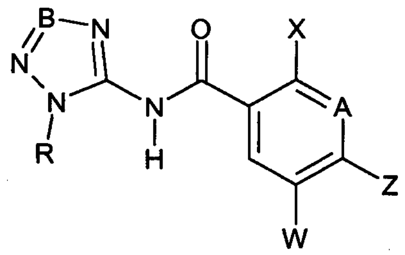

(I),

dans laquelle

A signifie N ou CY,
B signifie N ou CH,
X signifie nitro, halogéno, cyano, formyle, thiocyano, $(C_1-C_6)$-alkyle, halogéno- $(C_1-C_6)$-alkyle, $(C_2-C_6)$-alcényle, halogéno- $(C_2-C_6)$-alcényle, $(C_2-C_6)$-alcynyle, halogéno- $(C_3-C_6)$-alcynyle, $(C_3-C_6)$-cycloalkyle, halogéno-$(C_3-C_6)$-cycloalkyle, $(C_3-C_6)$-cycloalkyl-$(C_1-C_6)$-alkyle, halogéno-$(C_3-C_6)$-cycloalkyl-$(C_1-C_6)$-alkyle, $COR^1$, $COOR^1$, $OCOOR^1$, $NR^1COOR^1$, $C(O)N(R^1)_2$, $NR^1C(O)N(R^1)_2$, $OC(O)N(R^1)_2$, $C(O)NR^1OR^1$, $OR^1$, $OCOR^1$, $OSO_2R^2$, $S(O)_nR^2$, $SO_2OR^1$, $SO_2N(R^1)_2$, $NR^1SO_2R^2$, $NR^1COR^1$, $(C_1-C_6)$-alkyl-$S(O)_nR^2$, $(C_1-C_6)$-alkyl-$OR^1$, $(C_1-C_6)$-alkyl-$OCOR^1$, $(C_1-C_6)$-alkyl-$OSO_2R^2$, $(C_1-C_6)$-alkyl-$CO_2R^1$, $(C_1-C_6)$-alkyl-$SO_2OR^1$, $(C_1-C_6)$-alkyl-$CON(R^1)_2$, $(C_1-C_6)$-alkyl-$SO_2N(R^1)_2$, $(C_1-C_6)$-alkyl-$NR^1COR^1$, $(C_1-C_6)$-alkyl-$NR^1SO_2R^2$, $NR_1R_2$, $P(O)(OR^5)_2$, $CH_2P(O)(OR^5)_2$, $(C_1-C_6)$-alkyl-hétéroaryle, $(C_1-C_6)$-alkyl-hétérocyclyle, les deux derniers radicaux mentionnés étant à chaque fois substitués par s radicaux du groupe constitué par halogéno, $(C_1-C_6)$-alkyle, halogéno-$(C_1-C_6)$-alkyle, $S(O)_n$-$(C_1-_6)$-alkyle, $(C_1-_6)$-alcoxy et halogéno-$(C_1-C_6)$-alcoxy, et hétérocyclyle portant n groupes oxo,
Y signifie hydrogène, nitro, halogéno, cyano, thiocyano, $(C_1-C_6)$-alkyle, halogéno-$(C_1-C_6)$-alkyle, $(C_2-C_6)$-alcényle, halogéno- $(C_2-C_6)$-alcényle, $(C_2-C_6)$-alcynyle, halogéno- $(C_2-C_6)$-alcynyle, $(C_3-C_6)$-cycloalkyle, $(C_3-C_6)$-cycloalcényle, halogéno-$(C_3-C_6)$-cycloalkyle, $(C_3-C_6)$-cycloalkyl- $(C_1-C_6)$-alkyle, halogéno- $(C_3-C_6)$-cycloalkyl- $(C_1-C_6)$-alkyle, $COR^1$, $COOR^1$, $OCOOR^1$, $NR^1COOR^1$, $C(O)N(R^1)_2$, $NR^1C(O)N(R^1)_2$, $OC(O)N(R^1)_2$, $CO(NOR^1)R^1$, $NR^1SO_2R^2$, $NR^1COR^1$, $OR^1$, $OSO_2R^2$, $S(O)_nR^2$, $SO_2OR^1$, $SO_2N(R^1)_2$ $(C_1-C_6)$-alkyl-$S(O)_nR^2$, $(C_1-C_6)$-alkyl-$OR^1$, $(C_1-C_6)$-alkyl-$OCOR^1$, $(C_1-C_6)$-alkyl-$OSO_2R^2$, $(C_1-C_6)$-alkyl-$CO_2R^1$, $(C_1-C_6)$-alkyl-CN, $(C_1-C_6)$-alkyl-$SO_2OR^1$, $(C_1-C_6)$-alkyl-$CON(R^1)_2$, $(C_1-C_6)$-alkyl-$SO_2N(R^1)_2$, $(C_1-C_6)$-alkyl-$NR^1COR^1$, $(C_1-C_6)$-alkyl-$NR^1SO_2R^2$, $N(R^1)_2$, $P(O)(OR^5)_2$, $CH_2P(O)(OR^5)_2$, $(C_1-C_6)$-alkyl-phényle, $(C_1-C_6)$-alkyl-hétéroaryle, $(C_1-C_6)$-alkyl-hétérocyclyle, phényle, hétéroaryle ou hétérocyclyle, les six derniers radicaux mentionnés étant à chaque fois substitués par s radicaux du groupe constitué par halogéno, nitro, cyano, $(C_1-C_6)$-alkyle, halogéno- $(C_1-C_6)$-alkyle, $(C_3-C_6)$-cycloalkyle, $S(O)_n$-$(C_1-C_6)$-alkyle, $(C_1-C_6)$-alcoxy, halogéno-$(C_1-C_6)$-alcoxy, $(C_1-C_6)$-alcoxy-$(C_1-C_4)$-alkyle et cyanométhyle, et hétérocyclyle portant n groupes oxo,
Z signifie halogéno, cyano, thiocyano, halogéno-$(C_1-C_6)$-alkyle, $(C_2-C_6)$-alcényle, halogéno-$(C_2-C_6)$-alcényle, $(C_2-C_6)$-alcynyle, halogéno-$(C_2-C_6)$-alcynyle, $(C_3-C_6)$-cycloalkyle, halogéno-$(C_3-C_6)$-cycloalkyle, $(C_3-C_6)$-cycloalkyl- $(C_1-C_6)$-alkyle, halogéno-$(C_3-C_6)$-cycloalkyl-$(C_1-C_6)$-alkyle, $COR^1$, $COOR^1$, $OCOOR^1$, $NR^1COOR^1$,

$C(O)N(R^1)_2$, $NR^1C(O)N(R^1)_2$, $OC(O)N(R^1)_2$, $C(O)NR^1OR^1$, $OSO_2R^2$, $S(O)_nR^2$, $SO_2OR^1$, $SO_2N(R^1)_2$, $NR^1SO_2R^2$, $NR^1COR^1$, $(C_1\text{-}C_6)$-alkyl-$S(O)_nR^2$, $(C_1\text{-}C_6)$-alkyl-$OR^1$, $(C_1$-C6-alkyl-$OCOR^1$, $(C_1\text{-}C_6)$-alkyl-$OSO_2R^2$, $(C_1\text{-}C_6)$-alkyl-$CO_2R^1$, $(C_1\text{-}C_6)$-alkyl-$SO_2OR^1$, $(C_1\text{-}C_6)$-alkyl-$CON(R^1)_2$, $(C_1\text{-}C_6)$-alkyl-$SO_2N(R^1)_2$, $(C_1\text{-}C_6)$-alkyl-$NR^1COR^1$, $(C_1\text{-}C_6)$-alkyl-$NR^1SO_2R^2$, $N(R^1)_2$, $P(O)(OR^5)_2$, hétéroaryle, hétérocyclyle ou phényle, les trois derniers radicaux mentionnés étant à chaque fois substitués par s radicaux du groupe constitué par halogéno, nitro, cyano, $(C_1\text{-}C_6)$-alkyle, halogéno-$(C_1\text{-}C_6)$-alkyle, $(C_3\text{-}C_6)$-cycloalkyle, $S(O)_n$-$(C_1\text{-}C_6)$-alkyle, $(C_1\text{-}C_6)$-alcoxy ou halogéno-$(C_1\text{-}C_6)$-alcoxy, et hétérocyclyle portant n groupes oxo, ou

Z peut également signifier hydrogène, $(C_1\text{-}C_6)$-alkyle ou $(C_1\text{-}C_6)$-alcoxy si Y représente le radical $S(O)_nR^2$,

W signifie $(C_1\text{-}C_6)$-alkyle, halogéno-$(C_1\text{-}C_6)$-alkyle, $(C_2\text{-}C_6)$-alcényle, halogéno-$(C_2\text{-}C_6)$-alcényle, $(C_2\text{-}C_6)$-alcynyle, halogéno-$(C_2\text{-}C_6)$-alcynyle, $(C_3\text{-}C_7)$-cycloalkyle, $(C_3\text{-}C_7)$-halogénocycloalkyle, $(C_1\text{-}C_6)$-alcoxy, $(C_1\text{-}C_6)$-halogénoalcoxy, $S(O)_n$-$(C_1\text{-}C_6)$-alkyle, $S(O)_n$-$(C_1\text{-}C_6)$-halogénoalkyle, $(C_1\text{-}C_6)$-alcoxy- $(C_1\text{-}C_4)$-alkyle, $(C_1\text{-}C_6)$-alcoxy- $(C_1\text{-}C_4)$-halogénoalkyle, halogéno, nitro, $NR^3COR^3$ ou cyano,

R signifie $(C_1\text{-}C_8)$-alkyle, halogéno-$(C_1\text{-}C_8)$-alkyle, $(C_2\text{-}C_8)$-alcényle, halogéno-$(C_2\text{-}C_8)$-alcényle, $(C_2\text{-}C_8)$-alcynyle, halogéno-$(C_2\text{-}C_8)$-alcynyle, ces six radicaux mentionnés ci-dessus étant à chaque fois substitués par s radicaux du groupe constitué par hydroxy, nitro, cyano, $SiR^5_3$, $PO(OR^5)_2$, $S(O)_n$-$(C_1\text{-}C_6)$-alkyle, $S(O)_n$-$(C_1C_6)$-halogénoalkyle, $(C_1\text{-}C_6)$-alcoxy, halogéno-$(C_1\text{-}C_6)$-alcoxy, $N(R^3)_2$, $COR^3$, $COOR^3$, $OCOR^3$, $NR^3COR^3$, $NR^3SO_2R^4$ , $O(C_1\text{-}C_2)$-alkyl-$(C_3\text{-}C_6)$-cycloalkyle, $(C_3\text{-}C_6)$-cycloalkyle, hétéroaryle, hétérocyclyle, phényle, Q-hétéroaryle, Q-hétérocyclyle, Q-phényle et Q-benzyle, les sept derniers radicaux mentionnés étant à chaque fois substitués par s radicaux du groupe constitué par méthyle, éthyle, méthoxy, trifluorométhyle, cyano et halogéno, et hétérocyclyle portant n groupes oxo, ou

R signifie $(C_3\text{-}C_7)$-cycloalkyle, hétéroaryle, hétérocyclyle ou phényle à chaque fois substitué par s radicaux du groupe constitué par halogéno, nitro, cyano, $(C_1\text{-}C_6)$-alkyle, halogéno-$(C_1\text{-}C_6)$-alkyle, $(C_3\text{-}C_6)$-cycloalkyle, $S(O)_n$-$(C_1\text{-}C_6)$-alkyle, $(C_1\text{-}C_6)$-alcoxy, halogéno-$(C_1\text{-}C_6)$-alcoxy et $(C_1\text{-}C_6)$-alcoxy-$(C_1\text{-}C_4)$-alkyle, hétérocyclyle portant n groupes oxo,

Q signifie 0, S ou $NR^3$,

$R^1$ signifie hydrogène, $(C_1\text{-}C_6)$-alkyle, $(C_1\text{-}C_6)$-halogénoalkyle, $(C_2\text{-}C_6)$-alcényle, $(C_2\text{-}C_6)$-halogénoalcényle, $(C_2\text{-}C_6)$-alcynyle, $(C_2\text{-}C_6)$-halogénoalcynyle, $(C_3\text{-}C_6)$-cycloalkyle, $(C_3\text{-}C_6)$-cycloalcényle, $(C_3\text{-}C_6)$-halogénocycloalkyle, $(C_1\text{-}C_6)$-alkyl-O-$(C_1\text{-}C_6)$-alkyle, $(C_3\text{-}C_6)$ -cycloalkyl- $(C_1\text{-}C_6)$-alkyle, phényle, phényl-$(C_1\text{-}C_6)$-alkyle, hétéroaryle, $(C_1\text{-}C_6)$-alkyl-hétéroaryle, hétérocyclyle, $(C_1\text{-}C_6)$-alkyl-hétérocyclyle, $(C_1\text{-}C_6)$-alkyl-0-hétéroaryle, $(C_1\text{-}C_6)$-alkyl-O-hétérocyclyle, $(C_1\text{-}C_6)$-alkyl-$NR^3$-hétéroaryle ou $(C_1\text{-}C_6)$-alkyl-$NR^3$-hétérocyclyle, les 21 derniers radicaux mentionnés étant à chaque fois substitués par s radicaux du groupe constitué par cyano, halogéno, nitro, thiocyano, $OR^3$, $S(O)_nR^4$, $N(R^3)_2$, $NR^3OR^3$, $COR^3$, $OCOR^3$, $SCOR^4$, $NR^3COR^3$, $NR^3SO_2R^4$, $CO_2R^3$, $COSR^4$, $CON(R^3)_2$ et $(C_1\text{-}C_4)$-alcoxy-$(C_2\text{-}C_6)$-alcoxycarbonyle, et hétérocyclyle portant n groupes oxo,

$R^2$ signifie $(C_1\text{-}C_6)$-alkyle, $(C_1\text{-}C_6)$-halogénoalkyle, $(C_2\text{-}C_6)$-alcényle, $(C_2\text{-}C_6)$-halogénoalcényle, $(C_2\text{-}C_6)$-alcynyle, $(C_2\text{-}C_6)$-halogénoalcynyle, $(C_3\text{-}C_6)$-cycloalkyle, $(C_3\text{-}C_6)$-cycloalcényle, $(C_3\text{-}C_6)$-halogénocycloalkyle, $(C_1\text{-}C_6)$-alkyl-O-$(C_1\text{-}C_6)$-alkyle, $(C_3\text{-}C_6)$-cycloalkyl-$(C_1\text{-}C_6)$-alkyle, phényle, phényl-$(C_1\text{-}C_6)$-alkyle, hétéroaryle, $(C_1\text{-}C_6)$-alkyl-hétéroaryle, hétérocyclyle, $(C_1\text{-}C_6)$-alkyl-hétérocyclyle, $(C_1\text{-}C_6)$-alkyl-O-hétéroaryle, $(C_1\text{-}C_6)$-alkyl-0-hétérocyclyle, $(C_1\text{-}C_6)$-alkyl-$NR^3$-hétéroaryle ou $(C_1\text{-}C_6)$-alkyl-$NR^3$-hétérocyclyle, les 21 derniers radicaux mentionnés étant à chaque fois substitués par s radicaux du groupe cyano, halogéno, nitro, thiocyano, $OR^3$, $S(O)_nR^4$, $N(R^3)_2$, $NR^3OR^3$, $COR^3$, $OCOR^3$, $SCOR^4$, $NR^3COR^3$, $NR^3SO_2R^4$, $CO_2R^3$, $COSR^4$, $CON(R^3)_2$ et $(C_1\text{-}C_4)$-alcoxy-$(C_2\text{-}C_6)$-alcoxycarbonyle, et hétérocyclyle portant n groupes oxo,

$R^3$ signifie hydrogène, $(C_1\text{-}C_6)$-alkyle, $(C_2\text{-}C_6)$-alcényle, $(C_2\text{-}C_6)$-alcynyle, $(C_3\text{-}C_6)$-cycloalkyle, $(C_3\text{-}C_6)$-cycloalkyl-$(C_1\text{-}C_6)$-alkyle ou phényle,

$R^4$ signifie $(C_1\text{-}C_6)$-alkyle, $(C_2\text{-}C_6)$-alcényle, $(C_2\text{-}C_6)$-alcynyle, $(C_3\text{-}C_6)$-cycloalkyle, $(C_3\text{-}C_6)$-cycloalkyl-$(C_1\text{-}C_6)$-alkyle ou phényle,

$R^5$ signifie $(C_1\text{-}C_4)$-alkyle,

n signifie 0, 1 ou 2,

s signifie 0, 1, 2 ou 3.

**2.** Amides 5-phénylsubstitués de l'acide N-(tétrazol-5-yl)arylcarboxylique et de l'acide N-(triazol-5-yl)arylcarboxylique selon la revendication 1, dans lesquels

A signifie N ou CY,

B signifie N ou CH,

X signifie nitro, halogéno, cyano, thiocyano, $(C_1\text{-}C_6)$ -alkyle, halogéno-$(C_1\text{-}C_6)$-alkyle, $(C_2\text{-}C_6)$-alcényle, halogéno- $(C_2\text{-}C_6)$-alcényle, $(C_2\text{-}C_6)$-alcynyle, halogéno- $(C_3\text{-}C_6)$-alcynyle, $(C_3\text{-}C_6)$-cycloalkyle, halogéno- $(C_3\text{-}C_6)$-cycloalkyle, $(C_1\text{-}C_6)$-alkyl-O-$(C_1\text{-}C_6)$-alkyle, $(C_3\text{-}C_6)$-cycloalkyl- $(C_1\text{-}C_6)$-alkyle, halogéno-$(C_3\text{-}C_6)$-cycloalkyl-$(C_1\text{-}C_6)$-alkyle, $COR^1$, $OR^1$, $OCOR^1$, $OSO_2R^2$, $S(O)_nR^2$, $SO_2OR^1$, $SO_2N(R^1)_2$, $NR^1SO_2R^2$, $NR^1COR^1$,

$(C_1-C_6)$-alkyl-$S(O)_nR^2$, $(C_1-C_6)$-alkyl-$OR^1$, $(C_1-C_6)$-alkyl-$OCOR^1$, $(C_1-C_6)$-alkyl-$OSO_2R^2$, $(C_1-C_6)$-alkyl-$CO_2R^1$, $(C_1-C_6)$-alkyl-$SO_2OR^1$, $(C_1-C_6)$-alkyl-CON $(R^1)_2$, $(C_1-C_6)$-alkyl-$SO_2N(R^1)_2$, $(C_1-C_6)$-alkyl-$NR^1COR^1$ ou $(C_1-C_6)$-alkyl-$NR^1SO_2R^2$, $(C_1-C_6)$-alkyl-hétéroaryle, $(C_1-C_6)$-alkyl-hétérocyclyle, les deux derniers radicaux mentionnés étant à chaque fois substitués par s radicaux du groupe constitué par halogéno, $(C_1-C_6)$-alkyle, halogéno-$(C_1-C_6)$-alkyle, S $(O)_n$-$(C_1-C_6)$-alkyle, $(C_1-C_6)$-alcoxy et halogéno-$(C_1-C_6)$-alcoxy, et hétérocyclyle portant n groupes oxo,

Y signifie hydrogène, nitro, halogéno, cyano, thiocyano, $(C_1-C_6)$-alkyle, halogéno-$(C_1-C_6)$-alkyle, $(C_2-C_6)$-alcényle, halogéno- $(C_2-C_6)$-alcényle, $(C_2-C_6)$-alcynyle, halogéno- $(C_3-C_6)$-alcynyle, $(C_3-C_6)$-cycloalkyle, $(C_3-C_6)$-cycloalcényle, halogéno-$(C_3-C_6)$-cycloalkyle, $(C_3-C_6)$-cycloalkyl- $(C_1-C_6)$-alkyle, halogéno-$(C_3-C_6)$-cycloalkyl-$(C_1-C_6)$-alkyle, $COR^1$, $OR^1$, $COOR^1$, $OSO_2R^2$, $S(O)_nR^2$, $SO_2OR^1$, $SO_2N(R^1)_2$, $N(R^1)_2$, $NR^1SO_2R^2$, $NR^1COR^1$, $(C_1-C_6)$-alkyl-$S(O)_nR^2$, $(C_1-C_6)$-alkyl-$OR^1$, $(C_1-C_6)$-alkyl-$OCOR^1$, $(C_1-C_6)$ -alkyl-$OSO_2R^2$, $(C_1-C_6)$-alkyl-$CO_2R^1$, $(C_1-C_6)$-alkyl-$SO_2OR^1$, $(C_1-C_6)$-alkyl-$CON(R^1)_2$, $(C_1-C_6)$-alkyl-$SO_2N$ $(R^1)_2$, $(C_1-C_6)$-alkyl-$NR^1COR^1$, $(C_1-C_6)$-alkyl-$NR^1SO_2R^2$, $(C_1-C_6)$-alkyl-phényle, $(C_1-C_6)$-alkyl-hétéroaryle, $(C_1-C_6)$-alkyl-hétérocyclyle, phényle, hétéroaryle ou hétérocyclyle, les six derniers radicaux mentionnés étant à chaque fois substitués par s radicaux du groupe constitué par halogéno, nitro, cyano, $(C_1-C_6)$-alkyle, halogéno- $(C_1-C_6)$-alkyle, $(C_3-C_6)$-cycloalkyle, S $(O)_n$-$(C_1-C_6)$-alkyle, $(C_1-C_6)$-alcoxy, halogéno-$(C_1-C_6)$-alcoxy, $(C_1-C_6)$-alcoxy-$(C_1-C_4)$-alkyle et cyanométhyle, et hétérocyclyle portant n groupes oxo,

Z signifie halogéno, cyano, thiocyano, halogéno-$(C_1-C_6)$-alkyle, $(C_2-C_6)$-alcényle, halogéno- $(C_2-C_6)$-alcényle, $(C_2-C_6)$-alcynyle, halogéno-$(C_3-C_6)$-alcynyle, $(C_3-C_6)$-cycloalkyle, halogéno- $(C_3-C_6)$-cycloalkyle, $(C_3-C_6)$-cycloalkyl-$(C_1-C_6)$-alkyle, halogéne-$(C_3-C_6)$-cycloalkyl-$(C_1-C_6)$-alkyle, $COR^1$, $COOR^1$, $C(O)N(R^1)_2$, $C(O)NR^1OR^1$, $OSO_2R^2$, $S(O)_nR^2$, $SO_2OR^1$, $SO_2N(R^1)_2$, $NR^1SO_2R^2$, $NR^1COR^1$, $(C_1-C_6)$-alkyl-$S(O)_nR^2$, $(C_1-C_6)$-alkyl-$OR^1$, $(C_1-C_6)$-alkyl-$OCOR^1$, $(C_1-C_6)$-alkyl-$OSO_2R^2$, $(C_1-C_6)$-alkyl-$CO_2R^1$, $(C_1-C_6)$-alkyl-$SO_2OR^1$, $(C_1-C_6)$-alkyl-$CON(R^1)_2$, $(C_1-C_6)$-alkyl-$SO_2N(R^1)_2$, $(C_1-C_6)$-alkyl-$NR^1COR^1$, $(C_1-C_6)$-alkyl-$NR^1SO_2R^2$, 1,2,4-triazol-1-yle, ou

Z peut également signifier hydrogène, $(C_1-C_6)$-alkyle ou $(C_1-C_6)$-alcoxy si Y représente le radical $S(O)_nR^2$,

W signifie $(C_1-C_6)$-alkyle, halogéno-$(C_1-C_6)$-alkyle, $(C_1-C_6)$-alcoxy, $(C_1-C_6)$-halogénoalcoxy, $S(O)_n$-$(C_1-C_6)$-alkyle, $S(O)_n$-$(C_1-C_6)$-halogénoalkyle, $(C_1-C_6)$-alcoxy-$(C_1-C_4)$-alkyle, halogéno, nitro ou cyano,

R signifie $(C_1-C_8)$-alkyle, halogéno-$(C_1-C_6)$-alkyle, $(C_2-C_8)$-alcényle, halogéno-$(C_2-C_8)$-alcényle, $(C_2-C_8)$-alcynyle, halogéno-$(C_2-C_8)$-alcynyle, ces six radicaux mentionnés ci-dessus étant à chaque fois substitués par s radicaux du groupe constitué par nitro, cyano, $SiR^5_3$, $P(OR^5)_3$, $S(O)_n$-$(C_1-C_6)$-alkyle, $(C_1-C_6)$-alcoxy, halogéno-$(C_1-C_6)$-alcoxy, $N(R^3)_2$, $COR^3$, $COOR^3$, $OCOR^3$, $NR^3COR^3$, $NR^3SO_2R^4$, $(C_3-C_6)$-cycloalkyle, hétéroaryle, hétérocyclyle, phényle, Q-hétéroaryle, Q-hétérocyclyle, Q-phényle et Q-benzyle, les sept derniers radicaux mentionnés étant à chaque fois substitués par s radicaux du groupe constitué par méthyle, éthyle, méthoxy, trifluorométhyle, cyano et halogéno, et hétérocyclyle portant n groupes oxo, ou

R signifie $(C_3-C_7)$-cycloalkyle, hétéroaryle, hétérocyclyle ou phényle à chaque fois substitué par s radicaux du groupe constitué par halogéno, nitro, cyano, $(C_1-C_6)$-alkyle, halogéno-$(C_1-C_6)$-alkyle, $(C_3-C_6)$-cycloalkyle, $S(O)_n$-$(C_1-C_6)$-alkyle, $(C_1-C_6)$-alcoxy, halogéno-$(C_1-C_6)$-alcoxy et $(C_1-C_6)$-alcoxy-$(C_1-C_4)$-alkyle,

Q signifie 0, S ou $NR^3$,

$R^1$ signifie hydrogène, $(C_1-C_6)$-alkyle, $(C_2-C_6)$-alcényle, $(C_2-C_6)$-alcynyle, $(C_3-C_6)$-cycloalkyle, $(C_3-C_6)$ -cycloalkyl- $(C_1-C_6)$-alkyle, $(C_1-C_6)$-alkyl-O-$(C_1-C_6)$-alkyle, phényle, phényl-$(C_1-C_6)$-alkyle, hétéroaryle, $(C_1-C_6)$-alkyl-hétéroaryle, hétérocyclyle, $(C_1-C_6)$-alkyl-hétérocyclyle, $(C_1-C_6)$-alkyl-0-hétéroaryle, $(C_1-C_6)$-alkyl-O-hétérocyclyle, $(C_1-C_6)$-alkyl-$NR^3$-hétéroaryle ou $(C_1-C_6)$-alkyl-$NR^3$-hétérocyclyle, les seize derniers groupes mentionnés étant à chaque fois substitués par s radicaux du groupe constitué par cyano, halogéno, nitro, $OR^3$, $S(O)R^4$, $N(R^3)_2$, $NR^3OR^3$, $COR^3$, $OCOR^3$, $NR^3COR^3$, $NR^3SO_2R^4$, $CO_2R^3$, $CON(R^3)_2$ et $(C_1-C_4)$-alcoxy-$(C_2-C_6)$-alcoxycarbonyle, et hétérocyclyle portant n groupes oxo,

$R^2$ signifie $(C_1-C_6)$-alkyle, $(C_2-C_6)$-alcényle, $(C_2-C_6)$-alcynyle, $(C_3-C_6)$-cycloalkyle, $(C_3-C_6)$-cycloalkyl-$(C_1-C_6)$-alkyle, $(C_1-C_6)$-alkyl-O-$(C_1-C_6)$-alkyle, phényle, phényl-$(C_1-C_6)$-alkyle, hétéroaryle, $(C_1-C_6)$-alkyl-hétéroaryle, hétérocyclyle, $(C_1-C_6)$-alkyl-hétérocyclyle, $(C_1-C_6)$-alkyl-O-hétéroaryle, $(C_1-C_6)$-alkyl-O-hétérocyclyle, $(C_1-C_6)$-alkyl-$NR^3$-hétéroaryle ou $(C_1-C_6)$-alkyl-$NR^3$-hétérocyclyle, ces seize derniers radicaux mentionnés étant à chaque fois substitués par s radicaux du groupe constitué par cyano, halogéno, nitro, $OR^3$, $S(O)_nR^4$, $N(R^3)_2$, $NR^3OR^3$, $NR^3SO_2R^4$, $COR^3$, $OCOR^3$, $NR^3COR^3$, $CO_2R^3$, $CON(R^3)_2$ et $(C_1-C_4)$-alcoxy-$(C_2-C_6)$-alcoxycarbonyle, et hétérocyclyle portant n groupes oxo,

$R^3$ signifie hydrogène, $(C_1-C_6)$-alkyle, $(C_2-C_6)$-alcényle, $(C_2-C_6)$-alcynyle, $(C_3-C_6)$-cycloalkyle ou $(C_3-C_6)$-cycloalkyl-$(C_1-C_6)$-alkyle,

$R^4$ signifie $(C_1-C_6)$-alkyle, $(C_2-C_6)$-alcényle ou $(C_2-C_6)$-alcynyle,

$R^5$ signifie méthyle ou éthyle,

n signifie 0, 1 ou 2,

s signifie 0, 1, 2 ou 3.

3. Amides 5-phénylsubstitués de l'acide N-(tétrazol-5-yl) arylcarboxylique et de l'acide N-(triazol-5-yl)arylcarboxylique selon la revendication 1 ou 2, dans lesquels

A signifie N ou CY,
B signifie N ou CH,
X signifie nitro, halogéno, cyano, ($C_1$-$C_6$)-alkyle, halogéno-($C_1$-$C_6$)-alkyle, ($C_3$-$C_6$)-cycloalkyle, $OR^1$, $S(O)_nR^2$, ($C_1$-$C_6$)-alkyl-$S(O)_nR^2$, ($C_1$-$C_6$)-alkyl-$OR^1$, ($C_1$-$C_6$)-alkyl-$CON(R^1)_2$, ($C_1$-$C_6$)-alkyl-$SO_2N(R^1)_2$, ($C_1$-$C_6$)-alkyl-$NR^1COR^1$, ($C_1$-$C_6$)-alkyl-$NR^1SO_2R^2$, ($C_1$-$C_6$)-alkyl-hétéroaryle, ($C_1$-$C_6$)-alkyl-hétérocyclyle, les deux derniers radicaux mentionnés étant à chaque fois substitués par s radicaux halogéno, ($C_1$-$C_6$)-alkyle, halogéno-($C_1$-$C_6$)-alkyle, $S(O)_n$-($C_1$-$C_6$)-alkyle, ($C_1$-$C_6$)-alcoxy et halogéno-($C_1$-$C_6$)-alcoxy, et hétérocyclyle portant n groupes oxo,
Y signifie hydrogène, nitro, halogéno, cyano, ($C_1$-$C_6$)-alkyle, ($C_1$-$C_6$)-halogénoalkyle, $OR^1$, $S(O)_nR^2$, $SO_2N(R^1)_2$, $N(R^1)_2$, $NR^1SO_2R^2$, $NR^1COR^1$, ($C_1$-$C_6$)-alkyl-$S(O)_nR^2$, ($C_1$-$C_6$)-alkyl-$OR^1$, ($C_1$-$C_6$)-alkyl-$CON(R^1)_2$, ($C_1$-$C_6$)-alkyl-$SO_2N(R^1)_2$, ($C_1$-$C_6$)-alkyl-$NR^1COR^1$, ($C_1$-$C_6$)-alkyl-$NR^1SO_2R^2$, ($C_1$-$C_6$)-alkyl-phényle, ($C_1$-$C_6$)-alkyl-hétéroaryle, ($C_1$-$C_6$)-alkyl-hétérocyclyle, phényle, hétéroaryle ou hétérocyclyle, les six derniers radicaux mentionnés étant à chaque fois substitués par s radicaux du groupe constitué par halogéno, nitro, cyano, ($C_1$-$C_6$)-alkyle, halogéno-($C_1$-$C_6$)-alkyle, ($C_3$-$C_6$)-cycloalkyle, $S(O)_n$-($C_1$-$C_6$)-alkyle, ($C_1$-$C_6$)-alcoxy, halogéno- ($C_1$-$C_6$)-alcoxy, ($C_1$-$C_6$)-alcoxy-($C_1$-$C_4$)-alkyle et cyanométhyle, et hétérocyclyle portant n groupes oxo,
Z signifie halogéno, cyano, halogéno-($C_1$-$C_6$)-alkyle, ($C_3$-$C_6$)-cycloalkyle, $S(O)_nR^2$, 1,2,4-triazol-1-yle, ou
Z peut également signifier hydrogène, méthyle, méthoxy ou éthoxy si Y représente le radical $S(O)_nR^2$,
W signifie méthyle, éthyle, méthoxyméthyle, méthoxy, fluor, chlore ou $S(O)_nCH_3$,
R signifie ($C_1$-$C_8$)-alkyle, halogéno-($C_1$-$C_8$)-alkyle, ($C_2$-$C_8$)-alcényle, halogéno-($C_2$-$C_8$)-alcényle, ($C_2$-$C_8$)-alcynyle, halogéno-($C_2$-$C_8$)-alcynyle, ces six radicaux mentionnés ci-dessus étant à chaque fois substitués par s radicaux du groupe constitué par cyano, $S(O)_n$-($C_1$-$C_6$)-alkyle, ($C_1$-$C_6$)-alcoxy, halogéno-($C_1$-$C_6$)-alcoxy, $COR^3$, $COOR^3$, $OCOR^3$, $NR^3COR^3$, $NR^3SO_2R^4$, ($C_3$-$C_6$) -cycloalkyle, hétéroaryle, hétérocyclyle et phényle, les trois derniers radicaux mentionnés étant à chaque fois substitués par s radicaux du groupe constitué par méthyle, éthyle, méthoxy, trifluorométhyle, cyano et halogéno, et hétérocyclyle portant 0 à 2 groupes oxo, ou
R signifie phényle substitué par s radicaux du groupe constitué par halogéno, nitro, cyano, ($C_1$-$C_6$)-alkyle, halogéno-($C_1$-$C_6$)-alkyle, ($C_3$-$C_6$)-cycloalkyle, $S(O_n$-($C_1$-$C_6$)-alkyle, ($C_1$-$C_6$)-alcoxy, halogéno-($C_1$-$C_6$)-alcoxy, ($C_1$-$C_6$)-alcoxy-($C_1$-$C_4$)-alkyle,
$R^1$ signifie hydrogène, ($C_1$-$C_6$)-alkyle, ($C_2$-$C_6$)-alcényle, ($C_2$-$C_6$)-alcynyle, ($C_3$-$C_6$)-cycloalkyle, ($C_3$-$C_6$) -cycloalkyl- ($C_1$-$C_6$) -alkyle, ($C_1$-$C_6$) -alkyl-O-($C_1$-$C_6$)-alkyle, phényle, phényl-($C_1$-$C_6$)-alkyle, hétéroaryle, ($C_1$-$C_6$)-alkyl-hétéroaryle, hétérocyclyle, ($C_1$-$C_6$) -alkyl-hétérocyclyle, ($C_1$-$C_6$)-alkyl-0-hétéroaryle, ($C_1$-$C_6$)-alkyl-O-hétérocyclyle, ($C_1C_6$)-alkyl-$NR^3$-hétéroaryle ou ($C_1$-$C_6$)-alkyl-$NR^3$-hétérocyclyle, les seize derniers groupes mentionnés étant à chaque fois substitués par s radicaux du groupe constitué par cyano, halogéno, nitro, $OR^3$, $S(O)_nR^4$, $N(R^3)_2$, $NR^3OR^3$, $COR^3$, $OCOR^3$, $NR^3COR^3$, $NR^3SO_2R^4$, $CO_2R^3$, $CON(R^3)_2$ et ($C_1$-$C_4$)-alcoxy-($C_2$-$C_6$)-alcoxycarbonyle, et hétérocyclyle portant n groupes oxo,
$R^2$ signifie ($C_1$-$C_6$)-alkyle, ($C_3$-$C_6$)-cycloalkyle ou ($C_3$-$C_6$)-cycloalkyl-($C_1$-$C_6$)-alkyle à chaque fois substitué par s radicaux du groupe constitué par halogéno et $OR^3$,
$R^3$ signifie hydrogène ou ($C_1$-$C_6$)-alkyle,
$R^4$ signifie ($C_1$-$C_6$)-alkyle,
$R^5$ signifie méthyle ou éthyle,
n signifie 0, 1 ou 2,
s signifie 0, 1, 2 ou 3.

4. Agents herbicides, **caractérisés par** une teneur active en tant qu'herbicide en au moins un composé de formule (I) selon l'une quelconque des revendications 1 à 3.

5. Agents herbicides selon la revendication 4 en mélange avec des adjuvants de formulation.

6. Agents herbicides selon la revendication 4 ou 5 contenant au moins une autre substance active en tant que pesticide du groupe formé par les insecticides, les acaricides, les herbicides, les fongicides, les antidotes et les régulateurs de croissance.

7. Agents herbicides selon la revendication 6 contenant un antidote.

8. Agents herbicides selon la revendication 7, contenant du cyprosulfamide, du cloquintocet-mexyl, du méfenpyr-diéthyl ou de l'isoxadifen-éthyl.

**9.** Agents herbicides selon l'une quelconque des revendications 6 à 8 contenant un autre herbicide.

**10.** Procédé pour lutter contre des plantes non souhaitées, **caractérisé en ce qu'**on applique une quantité active d'au moins un composé de formule (I) selon l'une quelconque des revendications 1 à 3 ou d'un agent herbicide selon l'une quelconque des revendications 4 à 9 sur les plantes ou sur le lieu de la croissance non souhaitée des plantes.

**11.** Utilisation de composés de formule (I) selon l'une quelconque des revendications 1 à 3 ou d'agents herbicides selon l'une quelconque des revendications 4 à 9 pour lutter contre des plantes non souhaitées.

**12.** Utilisation selon la revendication 11, **caractérisée en ce que** les composés de formule (I) sont utilisés pour lutter contre des plantes non souhaitées dans les cultures de plantes utiles.

**13.** Utilisation selon la revendication 12, **caractérisée en ce que** les plantes utiles sont des plantes utiles transgéniques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2003010143 A
- WO 2003010153 A
- EP 0049071 A1
- WO 2012028579 A1
- JP 63122673 B
- WO 9954328 A1
- WO 9746530 A1
- EP 0221044 A
- EP 0131624 A
- WO 9211376 A
- WO 9214827 A
- WO 9119806 A
- EP 0242236 A
- EP 242246 A
- WO 9200377 A
- EP 0257993 A
- US 5013659 A
- EP 0142924 A
- EP 0193259 A
- WO 9113972 A


### In der Beschreibung aufgeführte Nicht-Patentliteratur


- *Journal of the American Chemical Society,* 1954, vol. 76, 923-924
- *Journal of the American Chemical Society,* 1954, vol. 76, 88-89
- *Chemie,* 1990, vol. 30 (12), 436-437
- *Chemische Berichte,* 1964, vol. 97 (2), 396-404
- *Angewandte Chemie,* 1963, vol. 75, 918
- **D. TIEBES.** Combinatorial Chemistry - Synthesis, Analysis, Screening (Herausgeber Günther Jung). Verlag Wiley, 1999, 1-34
- Polymer-Supported Scavengers and Reagents for Solution-Phase Synthesis (Sigma-Aldrich). *Chem-Files,* vol. 4 (1
- **BARRY A. BUNIN.** The Combinatorial Index. Verlag Academic Press, 1998
- Combinatorial Chemistry - Synthesis, Analysis, Screening (Herausgeber Günther Jung). Verlag Wiley, 1999
- Organic and Medicinal Chemistry (Herausgeber C. O. Kappe und a. Stadler). Verlag Wiley, 2005
- Gene Transfer to Plants, Springer Lab Manual. Springer Verlag, 1995
- **CHRISTOU.** *Trends in Plant Science,* 1996, vol. 1, 423-431
- **SAMBROOK et al.** Molecular Cloning, A Laboratory Manual. Cold Spring Harbor Laboratory Press, 1989
- **WINNACKER.** Gene und Klone. VCH Weinheim, 1996
- **BRAUN et al.** *EMBO J.,* 1992, vol. 11, 3219-3227
- **WOLTER et al.** *Proc. Natl. Acad. Sci. USA,* 1988, vol. 85, 846-850
- **SONNEWALD et al.** *Plant J.,* 1991, vol. 1, 95-106
- **WINNACKER-KÜCHLER.** Chemische Technologie. C. Hanser Verlag, 1986, vol. 7
- **WADE VAN VALKENBURG.** Pesticide Formulations. Marcel Dekker, 1973
- **K. MARTENS.** Spray Drying" Handbook. G. Goodwin Ltd. London, 1979
- **WATKINS.** Handbook of Insecticide Dust Diluents and Carriers. Darland Books
- **H.V. OLPHEN.** Introduction to Clay Colloid Chemistry. J. Wiley & Sons
- **C. MARSDEN.** Solvents Guide. Interscience, 1963
- **MCCUTCHEON'S.** Detergents and Emulsifiers Annual. MC Publ. Corp,
- **SISLEY ; WOOD.** Encyclopedia of Surface Active Agents. Chem. Publ. Co. Inc, 1964
- **SCHÖNFELDT.** Grenzflächenaktive Äthylenoxidaddukte. Wiss. Verlagsgesell, 1976
- **VERFAHREN.** Spray-Drying Handbook. G. Goodwin Ltd, 1979
- Agglomeration. **J.E. BROWNING.** Chemical and Engineering. 1967, 147 ff
- Perry's Chemical Engineer's Handbook. McGraw-Hill, 1973, 8-57
- **G.C. KLINGMAN.** Weed Control as a Science. John Wiley and Sons, Inc, 1961, 81-96
- **J.D. FREYER ; S.A. EVANS.** Weed Control Handbook. Blackwell Scientific Publications, 1968, 101-103
- *Weed Research,* 1986, vol. 26, 441-445
- The Pesticide Manual. The British Crop Protection Council and the Royal Soc. of Chemistry. 2009